# EUROPEAN PATENT SPECIFICATION

(11) **EP 2 582 682 B1**
(45) Date of publication and mention of the grant of the patent: **14.01.2015**
(21) Application number: 11807233.9
(22) Date of filing: 16.06.2011
(51) Int. Cl.: A61K 45/06, A61P 9/12, A61P 11/00, A61K 31/00, A61K 31/137, A61K 31/437, A61K 31/353, A61K 31/517, A61K 38/05

(54) **MITOTIC KINESIN INHIBITORS FOR USE IN THE TREATMETNOF PULMONARY ARTERIAL HYPERTENSION**
MITOTISCHE KINESIN-HEMMER ZUR BEHANDLUNG DER PULMONALEN ARTERIELLEN HYPERTONIE
INHIBITEURS DE KINESINE MITOTIQUE POUR LE TRAITEMENT DE L'HYPERTENSION ARTERIELLE PULMONAIRE

(30) Priority: 02.12.2010 US 419202 P; 17.06.2010 US 355900 P
(43) Date of publication of application: 24.04.2013
(73) Proprietor: Cytokinetics, Inc., South San Francisco, CA 94080 (US)
(72) Inventor: MORGANS, David J., Jr., Los Altos California 94024 (US); MALIK, Fady, Burlingame California 94010 (US); WOOD, Kenneth, Foster City California 94404 (US); PANNIRSELVAM, Malar, Foster City California 94404 (US)
(74) Representative: Marshall, Cameron John
(86) International application number: PCT/US2011/040749
(87) International publication number: WO 2012/009097

(56) References cited:
- US-A1- 2008 255 182
- US-A1- 2010 099 697
- US-A1- 2010 104 574
- US-A1- 2010 144 782
- KYLE D HOLEN ET AL: "A first in human study of SB-743921, a kinesin spindle protein inhibitor, to determine pharmacokinetics, biologic effects and establish a recommended phase II dose", CANCER CHEMOTHERAPY AND PHARMACOLOGY, SPRINGER, BERLIN, DE, vol. 67, no. 2, 12 May 2010 (2010-05-12), pages 447-454, XP019877057, ISSN: 1432-0843, DOI: 10.1007/S00280-010-1346-5
- COLEMAN P J ET AL: "Inhibitors of the mitotic kinesin spindle protein", EXPERT OPINION ON THERAPEUTIC PATENTS, INFORMA HEALTHCARE, GB, vol. 14, no. 12, 1 January 2004 (2004-01-01), pages 1659-1668, XP002350201, ISSN: 1354-3776, DOI: 10.1517/13543776.14.12.1659
- KAMPOLIS C ET AL: "The presence of anti-centromere antibodies may predict progression of estimated pulmonary arterial systolic pressure in systemic sclerosis", SCANDINAVIAN JOURNAL OF RHEUMATOLOGY, vol. 37, no. 4, 2008, pages 278-283, XP009173836, ISSN: 0300-9742
- VOSWINCKEL R ET AL: "Therapie der pulmonalarteriellen Hypertonie", DER INTERNIST ; ORGAN DES BERUFSVERBANDES DEUTSCHER INTERNISTEN ORGAN DER DEUTSCHEN GESELLSCHAFT FÜR INNERE MEDIZIN, SPRINGER, BERLIN, DE, vol. 50, no. 9, 20 August 2009 (2009-08-20), pages 1101-1110, XP019730222, ISSN: 1432-1289, DOI: 10.1007/S00108-009-2336-8
- M. DELCROIX ET AL: "Long-term outcome in pulmonary arterial hypertension: a plea for earlier parenteral prostacyclin therapy", EUROPEAN RESPIRATORY REVIEW, vol. 18, no. 114, 30 November 2009 (2009-11-30), pages 253-259, XP055085608, ISSN: 0905-9180, DOI: 10.1183/09059180.00003109

## Description

Pulmonary arterial hypertension (PAH) is a syndrome characterized by a progressive increase in pulmonary vascular resistance leading to right ventricular overload and eventually cardiac failure. Approximately 2000 - 5000 patients are newly diagnosed with PAH each year in the United States and, despite recent advances in the understanding and treatment of this disorder, PAH is still a progressive and fatal illness.

PAH is a chronic disorder that involves all layers of the pulmonary vessels. Vasoconstriction, structural changes in the pulmonary vessel wall (vascular remodeling) and thrombosis contribute to the increased pulmonary vascular resistance in PAH. Structural and functional changes of the endothelium lead to endothelial dysfunction. Increased vasoconstrictive factors (e.g., endothelin) and decreased vasodilation capacity (e.g., less prostacyclin) result in vasoconstriction and increased pulmonary vascular resistance. Current treatments that seek to address vasoconstriction may slow the progression of PAH or ameliorate the clinical symptoms for a limited time, but they have not proven to substantially reduce overall PAH morbidity and mortality rates. Underlying structural changes to the pulmonary vessels - vascular remodeling - are not affected by these treatments.

Vascular remodeling that occurs in PAH is characterized by proliferative and obstructive changes involving many cell types, including endothelial cells, smooth muscle cells and fibroblasts. Vascular remodeling can manifest itself, for example, as medial thickening of pulmonary vessels due to smooth muscle cell hyperplasia and hypertrophy, formation of a neointima made of smooth muscle cells and/or myofibroblasts, and/or formation of plexiform lesions, which consist of localized proliferations of endothelial cells, smooth muscle cells, lymphocytes and mast cells. Vascular remodeling results in obstruction of the vessel lumen leading to pulmonary hypertension. There is a need for therapies that address the proliferative aspect of PAH.

Idiopathic pulmonary fibrosis (IPF) is a type of idiopathic interstitial pneumonia (IIP), which in turn is a type of interstitial lung disease (also known as diffuse parenchymal lung disease (DPLD)). Interstitial lung disease concerns alveolar epithelium, pulmonary capillary endothelium, basement membrane, perivascular and perilymphatic tissues. Other forms of idiopathic interstitial pneumonias include non-specific interstitial pneumonia (NSIP), desquamative interstitial pneumonia (DIP) and acute interstitial pneumonia (AIP). Examples of known causes of interstitial lung disease include sarcoidosis, hypersensitivity pneumonitis, pulmonary Langerhans cell histiocytosis, asbestosis and collagen vascular diseases such as scleroderma and rheumatoid arthritis.

Pulmonary fibrosis is the formation or development of excess fibrous connective tissue in the lungs. IPF (or cryptogenic fibrosing alveolitis (CFA)) is a type of pulmonary fibrosis of unknown cause that results in scarring, or fibrosis, of the lungs. In time, this fibrosis can build up to the point where the lungs are unable to provide oxygen to the tissues of the body. While the exact cause of IPF is unknown, IPF may result from an autoimmune disorder, the effects of an infection (e.g., a viral infection), or other injuries to the lung. The early stages of IPF are marked by alveolitis, an inflammation of the alveoli in the lungs. As IPF progresses, the alveoli become damaged and scarred, thus stiffening the lungs. The stiffening makes breathing difficult and brings on a feeling of breathlessness (dyspnea), especially during activities that require extra effort. In addition, scarring of the alveoli reduces the ability of the lungs to transfer oxygen. The resulting lack of oxygen in the blood (hypoxemia) may cause increases in the pressure inside the blood vessels of the lungs, a situation known as pulmonary hypertension. The high blood pressure in the lungs then puts a strain on the right ventricle, the lower right side of the heart, which pumps the oxygen-poor blood into the lungs.

IPF is commonly complicated by the development of pulmonary hypertension. The prevalence of pulmonary hypertension in IPF has been variably reported to occur in anywhere from 35 to 85% in patients, and the presence of pulmonary hypertension in IPF is generally associated with higher mortality. (See, e.g., Corte, T.J., et al., Pulmonary Hypertension in Idiopathic Pulmonary Fibrosis: A Review, Sarcoidosis Vasculitis and Diffuse Lung Diseases, 26, (2009), 7-19; Nathan, Steven, et al., Idiopathic Pulmonary Fibrosis and Pulmonary Hypertension: Connecting the Dots, Amer. J. Respiratory and Critical Care Medicine, 175, (2007), 875-880).

Lymphangioleiomyomatosis (LAM) is a rare lung disease that results in a proliferation of disorderly smooth muscle growth (leiomyoma) throughout the lungs, in the bronchioles, alveolar septa, perivascular spaces, and lymphatics, resulting in the obstruction of small airways (leading to pulmonary cyst formation and pneumothorax) and lymphatics (leading to chylous pleural effusion). LAM occurs in a sporadic form, which mainly affects females usually of childbearing age. LAM also occurs in patients who have tuberous sclerosis (TSC), often referred to as TSC-LAM. TSC is a genetic disease caused by a defect in one or more of two genes, TSC1 and TSC2. Patients who have LAM may also have abnormal TSC1 and TSC2 genes.

Mitotic kinesins are enzymes essential for assembly and function of a cell's mitotic spindle and other events that are key to mitosis, but are not generally part of other microtubule structures in non-proliferating cells, such as in nerve processes. During mitosis, certain kinesins organize microtubules into the bipolar structure of the mitotic spindle. Kinesins also mediate movement of chromosomes along spindle microtubules, as well as structural changes in the mitotic spindle associated with specific phases of mitosis. Perturbation of mitotic kinesin function can cause malformation and/or dysfunction of the mitotic spindle, frequently resulting in cell cycle arrest and cell death. Mitotic kinesin inhibitors have been shown to retard the growth and proliferation of certain cells, such as cancer cells.

### Brief Description of the Figures

Figure 1A is a graph depicting the cell number per mL for cultured pulmonary arterial smooth muscle cells derived from distal blood vessels taken from lungs harvested from patients with idiopathic pulmonary arterial hypertension (IPAH) treated with various concentrations of iloprost.
Figure 1B is a graph depicting percent cell growth normalized to to FBS-induced growth for the experiment depicted in Figure 1A.
Figure 2A is a graph depicting the cell number per mL for cultured pulmonary arterial smooth muscle cells derived from distal blood vessels taken from lungs harvested from patients with IPAH treated with various concentrations of treprostinil sodium.
Figure 2B is a graph depicting percent cell growth normalized to to FBS-induced growth for the experiment depicted in Figure 2A.
Figure 3A is a graph depicting the cell number per mL for cultured pulmonary arterial smooth muscle cells derived from distal blood vessels taken from lungs harvested from patients with IPAH treated with various concentrations of bosentan.
Figure 3B is a graph depicting percent cell growth normalized to to FBS-induced growth for the experiment depicted in Figure 3A.
Figure 4A is a graph depicting the cell number per mL for cultured pulmonary arterial smooth muscle cells derived from distal blood vessels taken from lungs harvested from patients with IPAH treated with various concentrations of ambrisentan.
Figure 4B is a graph depicting percent cell growth normalized to to FBS-induced growth for the experiment depicted in Figure 4A.
Figure 5A is a graph depicting the cell number per mL for cultured pulmonary arterial smooth muscle cells derived from distal blood vessels taken from lungs harvested from patients with IPAH treated with various concentrations of BQ788.
Figure 5B is a graph depicting percent cell growth normalized to to FBS-induced growth for the experiment depicted in Figure 5A.
Figure 6A is a graph depicting the cell number per mL for cultured pulmonary arterial smooth muscle cells derived from distal blood vessels taken from lungs harvested from patients with IPAH treated with various concentrations of Compound B.
Figure 6B is a graph depicting percent cell growth normalized to to FBS-induced growth for the experiment depicted in Figure 6A.
Figure 7A is a graph depicting the cell number per mL for cultured pulmonary arterial smooth muscle cells derived from distal blood vessels taken from lungs harvested from patients with IPAH treated with various concentrations of Compound A.
Figure 7B is a graph depicting percent cell growth normalized to to FBS-induced growth for the experiment depicted in Figure 7A.
Figure 8A is a graph depicting the cell number per mL for cultured pulmonary arterial smooth muscle cells derived from distal blood vessels taken from lungs harvested from patients with IPAH treated with various concentrations of ispinesib mesylate.
Figure 8B is a graph depicting percent cell growth normalized to to FBS-induced growth for the experiment depicted in Figure 8A.
Figure 9A is a graph depicting the cell number per mL for cultured pulmonary arterial smooth muscle cells derived from distal blood vessels taken from lungs harvested from patients with IPAH treated with 10 nM treprostinil sodium in combination with various concentrations of ispinesib mesylate.
Figure 9B is a graph depicting percent cell growth normalized to to FBS-induced growth for the experiment depicted in Figure 9A.
Figure 10A is a graph depicting the cell number per mL for cultured pulmonary arterial smooth muscle cells derived from distal blood vessels taken from lungs harvested from patients with IPAH treated with 100 nM bosentan in combination with various concentrations of ispinesib mesylate.
Figure 10B is a graph depicting percent cell growth normalized to to FBS-induced growth for the experiment depicted in Figure 10A.
Figure 11A is a graph depicting the cell number per mL for cultured pulmonary arterial smooth muscle cells derived from distal blood vessels taken from lungs harvested from patients with IPAH treated with 100 nM ambrisentan in combination with various concentrations of ispinesib mesylate.
Figure 11B is a graph depicting percent cell growth normalized to to FBS-induced growth for the experiment depicted in Figure 11A.
Figure 12A is a graph depicting the cell number per mL for cultured pulmonary arterial smooth muscle cells derived from distal blood vessels taken from lungs harvested from patients with IPAH treated with 100 nM BQ788 in combination with various concentrations of ispinesib mesylate.
Figure 12B is a graph depicting percent cell growth normalized to to FBS-induced growth for the experiment depicted in Figure 12A.
Figure 13A is a graph depicting the cell number per mL for cultured pulmonary arterial smooth muscle cells derived from distal blood vessels taken from lungs harvested from patients with IPAH treated with 100 nM ambrisentan and 100 nM BQ788 in combination with various concentrations of ispinesib mesylate.
Figure 13B is a graph depicting percent cell growth normalized to to FBS-induced growth for the experiment depicted in Figure 13A.
Figure 14A is a graph depicting the cell number per mL for cultured lung fibroblasts derived from lungs harvested from patients with ideopathic pulmonary fibrosis (IPF) with secondary pulmonary arterial hypertension (PAH) treated with various concentrations of treprostinil sodium.
Figure 14B is a graph depicting percent cell growth normalized to to FBS-induced growth for the experiment depicted in Figure 14A.
Figure 15A is a graph depicting the cell number per mL for cultured lung fibroblasts derived from lungs harvested from patients with ideopathic pulmonary fibrosis (IPF) with secondary pulmonary arterial hypertension (PAH) treated with various concentrations of ispinesib mesylate.
Figure 15B is a graph depicting percent cell growth normalized to to FBS-induced growth for the experiment depicted in Figure 15A.
Figure 16A is a graph depicting the cell number per mL for cultured pulmonary arterial smooth muscle cells derived from distal blood vessels taken from lungs harvested from healthy patients treated with various concentrations of treprostinil sodium.
Figure 16B is a graph depicting percent cell growth normalized to to FBS-induced growth for the experiment depicted in Figure 16A.
Figure 17A is a graph depicting the cell number per mL for cultured pulmonary arterial smooth muscle cells derived from distal blood vessels taken from lungs harvested from healthy patients treated with various concentrations of ispinesib mesylate.
Figure 17B is a graph depicting percent cell growth normalized to to FBS-induced growth for the experiment depicted in Figure 17A.

Provided herein are mitotic kinesin inhibitors for use in methods of treating pulmonary arterial hypertension in a subject by administering to the subject a therapeutically effective amount of the mitotic kinesin inhibitor, optionally in combination with another therapeutic, wherein the mitotic kinesin inhibitor is an inhibitor of kinesin spindle protein (KSP) or wherein the mitotic kinesin inhibitor is an inhibitor of centromere-associated protein E (CENP-E). In some embodiments, the described treatment reduces pulmonary vascular resistance in a subject suffering from PAH. In some embodiments, the described treatment reduces the size and/or number of plexiform lesions and/or neointima in the pulmonary vessels of a subject suffering from PAH. In some embodiments, the described treatment prevents formation of new plexiform lesions and/or neointima in the pulmonary vessels of a subject suffering from PAH. In some embodiments, the described treatment halts and/or reduces thickening of a pulmonary vessel wall in a subject suffering from PAH. In some embodiments, the described treatment prevents additional thickening of a pulmonary vessel wall in a subject suffering from PAH. In some embodiments, the described treatment increases the size of a pulmonary vessel lumen and/or reduces or removes obstructions in a pulmonary vessel lumen of a subject suffering from PAH. In some embodiments, the described treatment reduces vascular remodeling of a pulmonary vessel due to PAH. In some embodiments, the described treatment prevents or reduces the incidence of cardiac failure in a subject suffering from PAH.

Also described herein are methods of treating pulmonary fibrosis, for example, idiopathic pulmonary fibrosis (IPF), in a subject by administering to the subject a therapeutically effective amount of a mitotic kinesin inhibitor, optionally in combination with another therapeutic. Also described herein are methods of treating IPF with secondary pulmonary arterial hypertension (PAH) in a subject by administering to the subject a therapeutically effective amount of a mitotic kinesin inhibitor, optionally in combination with another therapeutic.

Also described herein are methods of treating lymphangioleiomyomatosis (LAM) in a subject by administering to the subject a therapeutically effective amount of a mitotic kinesin inhibitor, optionally in combination with another therapeutic.

Mitotic kinesin inhibitors are compounds that act to inhibit the activity of one or more mitotic kinesins. The term "'inhibition" refers to a decrease in mitotic kinesin activity as a direct or indirect response to the presence of a chemical entity (i.e., a mitotic kinesin inhibitor), relative to the activity of the mitotic kinesin in the absence of the chemical entity. In some embodiments, the decrease in activity is due to the direct interaction of the chemical entity with mitotic kinesin. In other embodiments, the decrease in activity is due to the interaction of the chemical entity with one or more other factors that affect mitotic kinesin activity. For example, the presence of the chemical entity may decrease mitotic kinesin activity by directly binding to the mitotic kinesin, by causing (directly or indirectly) another factor to decrease mitotic kinesin activity, or by (directly or indirectly) decreasing the amount of mitotic kinesin present in the cell or organism. In this context, mitotic kinesin inhibitors may act by increasing or decreasing spindle pole separation, causing malformation (e.g., splaying) of mitotic spindle poles, or otherwise causing morphological perturbation of the mitotic spindle or by interfering with the normal function of the mitotic spindle (e.g. misalignment and inappropriate segregation of chromosomes). Meiotic spindles may also be disrupted. Mitotic kinesin inhibitors may be reversible or irreversible, may bind to the kinesin's active site or an allosteric site, and may act as competitive, uncompetitive or non-competitive inhibitors. In all instances, mitotic kinesin inhibitors halt or slow the normal process of cell division and proliferation.

A mitotic kinesin inhibitor may be an inhibitor of one or more of the mitotic kinesins selected from the kinesin superfamily proteins (KIFs). A mitotic kinesin inhibitor may be an inhibitor of one or more of the human mitotic kinesins selected from kinesin spindle protein (KSP, also known as KIF11, Eg5, and/or KNSL1; see, e.g., U.S. Pat. No. 6,617,115 ), centromere-associated protein-E (CENP-E, also known as KIF10), HSET (also known as KIFC1 and/or KNSL2; see, e.g., U.S. Pat. No. 6,361,993), MCAK (also known as KIF2C; see, e.g., U.S. Pat. No. 6,331,424), RabK-6 (also known as KIF20A; see, e.g., U.S. Pat. No. 6,544,766 ), Kif4 (also known as KIF4A; see, e.g., U.S. Pat. No. 6,440,684 ), MKLP1 (also known as KIF23 and/or KNSL5; see, e.g., U.S. Pat. No. 6,448,025), Kifl5 (also known as Hklp2; see, e.g., U.S. Pat. No. 6,355,466), Kid (also known as KIF22 and/or KNSL4; see, e.g., U.S. Pat. No. 6,387,644 ), Mppl, CMKrp, KinI-3 (see, e.g., U.S. Pat. No. 6,461,855 ), Mppl, CMKrp, KinI-3 (see, e.g., U.S. Pat. No. 6,461,855 ), Kip3a (see, e.g., U.S. Pat. No. 6,680,369 ), Kip3d (see, e.g., U.S. Pat. No. 6,492,151 ).

In some embodiments, the mitotic kinesin inhibitor is an inhibitor of kinesin spindle protein (KSP), for example, human KSP. Examples of KSP inhibitors include, for example, compounds and compositions described and/or disclosed in U.S. Patent Nos. 6,545,004, 6,630,479, 6,831,085, 7,105,668, 7,294,634, 7,671,200 and 7,763,628 . Examples of suitable KSP inhibitors include compounds of Formulae I-IV: 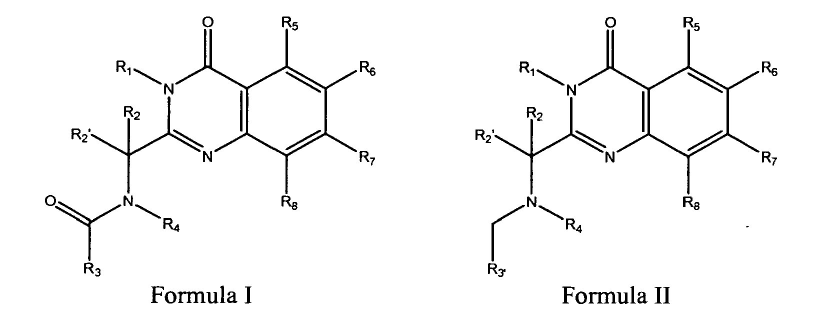 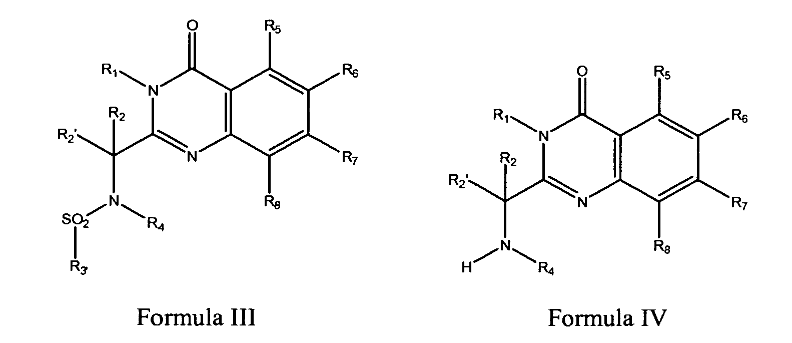 or a pharmaceutically acceptable salt of a compound of Formula I-IV; or a pharmaceutically acceptable solvate of a compound of Formula I-IV; or a pharmaceutically acceptable solvate of a pharmaceutically acceptable salt of a compound of Formula I-IV; and including single stereoisomers and mixtures of stereoisomers, wherein:
R₁ is chosen from hydrogen, alkyl, aryl, alkylaryl, heteroaryl, alkylheteroaryl, substituted alkyl, substituted aryl, substituted alkylaryl, substituted heteroaryl, and substituted alkylheteroaryl;
R₂ and R₂' are independently chosen from hydrogen, alkyl, oxaalkyl, aryl, alkylaryl, heteroaryl, alkylheteroaryl, substituted alkyl, substituted aryl, substituted alkylaryl, substituted heteroaryl, and substituted alkylheteroaryl; or R₂ and R₂' taken together form a 3- to 7-membered ring;
R₃ is chosen from hydrogen, alkyl, aryl, alkylaryl, heteroaryl, alkylheteroaryl, substituted alkyl, substituted aryl, substituted alkylaryl, substituted heteroaryl, substituted alkylheteroaryl, oxaalkyl, oxaalkylaryl, substituted oxaalkylaryl, R₁₅O- and R₁₅-NH-;
R_{3'} is chosen from hydrogen, alkyl, aryl, alkylaryl, heteroaryl, alkylheteroaryl, substituted alkyl, substituted aryl, substituted alkylaryl, substituted heteroaryl, substituted alkylheteroaryl and R₁₅-NH-;
R_{3"} is chosen from alkyl, aryl, alkylaryl, heteroaryl, alkylheteroaryl, substituted alkyl, substituted aryl, substituted alkylaryl, substituted heteroaryl, and substituted alkylheteroaryl;
R₄ is chosen from hydrogen, alkyl, aryl, alkylaryl, heteroaryl, alkylheteroaryl, substituted alkyl, substituted aryl, substituted alkylaryl, substituted heteroaryl, substituted alkylheteroaryl, and R₁₆-alkylene-;
R₅, R₆, R₇ and R₈ are independently chosen from hydrogen, alkyl, alkoxy, halogen, fluoroalkyl, nitro, dialkylamino, alkylsulfonyl, alkylsulfonamido, sulfonamidoalkyl, sulfonamidoaryl, alkylthio, carboxyalkyl, carboxamido, aminocarbonyl, aryl and heretoaryl;
R₁₅ is chosen from alkyl, aryl, alkylaryl, heteroaryl, alkylheteroaryl, substituted alkyl, substituted aryl, substituted alkylaryl, substituted heteroaryl, and substituted alkylheteroaryl; and
R₁₆ is chosen from alkoxy, amino, alkylamino, dialkylamino, N-heterocyclyl and substituted N-heterocyclyl.

An example of a suitable mitotic kinesin inhibitor is *N*-(3-aminopropyl)-*N*-[(1*R*)-1-(3-benzyl-7-chloro-4-oxo-3,4-dihydroquinazolin-2-yl)-2-methylpropyl]-4-methylbenzamide monomethanesulfonate (ispinesib mesylate).

Further examples of KSP inhibitors are compounds and compositions described and/or disclosed in U.S. Patent Nos. 6,924,376 and 7,629,477 . Examples of suitable KSP inhibitors include compounds of Formula V: 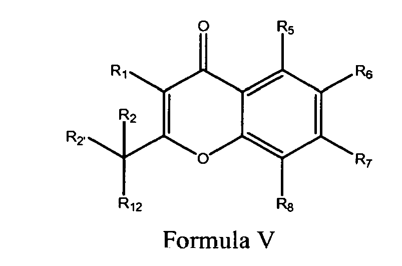 or a pharmaceutically acceptable salt of a compound of Formula I; or a pharmaceutically acceptable solvate of a compound of Formula I; or a pharmaceutically acceptable solvate of a pharmaceutically acceptable salt of a compound of Formula I; and including single stereoisomers and mixtures of stereoisomers, wherein:
R₁ is chosen from hydrogen, optionally substituted alkyl-, optionally substituted aryl-, optionally substituted aralkyl-, optionally substituted heteroaryl-, and optionally substituted heteroaralkyl-;
R₂ and R_{2'} are independently chosen from hydrogen, optionally substituted alkyl-, optionally substituted alkoxy, optionally substituted aryl-, optionally substituted aralkyl-, optionally substituted heteroaryl-, and optionally substituted heteroaralkyl-; or R₂ and R_{2'} taken together form an optionally substituted 3- to 7-membered ring;
R₁₂ is selected from the group consisting of optionally substituted imidazolyl, optionally substituted imidazolinyl, -NHR₄; -N(R₄)(COR₃); -N(R₄)(SO₂R₃ₐ); and-N(R₄)(CH₂R_{3b});
R₃ is chosen from hydrogen, optionally substituted alkyl-, optionally substituted aryl-, optionally substituted aralkyl-, optionally substituted heteroaryl-, optionally substituted heteroaralkyl-, R₁₅O- and R₁₇-NH-;
R₃ₐ is chosen from optionally substituted alkyl-, optionally substituted aryl-, optionally substituted aralkyl-, optionally substituted heteroaryl-, optionally substituted heteroaralkyl-, and R₁₇-NH-;
R_{3b} is chosen from hydrogen, optionally substituted alkyl-, optionally substituted aryl-, optionally substituted aralkyl-, optionally substituted heteroaryl-, and optionally substituted heteroaralkyl-;
R₄ is chosen from hydrogen, optionally substituted alkyl-, optionally substituted aryl-, optionally substituted aralkyl-, optionally substituted hetercyclyl-, and optionally substituted heteroaralkyl-;
R₅, R₆, R₇ and R₈ are independently chosen from hydrogen, acyl, optionally substituted alkyl-, optionally substituted alkoxy, halogen, hydroxyl, nitro, cyano, dialkylamino, alkylsulfonyl-, alkylsulfonamido-, alkylthio-, carboxyalkyl-, carboxamido-, aminocarbonyl-, optionally substituted aryl and optionally substituted heteroaryl-;
R₁₅ is chosen from optionally substituted alkyl-, optionally substituted aryl-, optionally substituted aralkyl-, optionally substituted heteroaryl-, and optionally substituted heteroaralkyl-; and
R₁₇ is hydrogen, optionally substituted alkyl-, optionally substituted aryl-, optionally substituted aralkyl-, optionally substituted heteroaryl-, or optionally substituted heteroaralkyl-.

An example of a suitable mitotic kinesin inhibitor is *N*-(3-aminopropyl)-*N*-[(*R*)-(1-3-benzyl-7-chloro-4-oxo-4H-chromen-2-yl)-2-methyl-propyl]-4-methyl-benzamide, or a pharmaceutically acceptable salt thereof. In cetain embodiments, the mitotic kinesin inhibitor is *N*-(3-aminopropyl)-*N*-[(*R*)-(1-3-benzyl-7-chloro-4-oxo-4H-chromen-2-yl)-2-methylpropyl]-4-methyl-benzamide hydrochloride hydrate.

Additional examples of KSP inhibitors that may be used in the present methods are compounds and compositions described and/or disclosed in U.S. Patent Nos. 6,992,082, 7,041,676, 7,038,048, 7,557,115, 7,211,580, 7,214,800, 7,166,595, 6,949,538, 7,271,167, 7,476,743, 7,439,254 and 7,208,487 .

In some embodiments, the mitotic kinesin inhibitor is a compound or composition described and/or disclosed in U.S. Patent No. 7,449,486 . Examples of such compounds include 2-(3-aminopropyl)-5-(3-fluorophenyl)-N-(2-methoxyethyl)-N-methyl-2-phenyl-1,3,4-thiadiazole-3(2H)-carboxamide, 2-(3-aminopropyl)-5-(3-fluorophenyl)-N-methoxy-N-methyl-2-phenyl-1,3,4-thiadiazole-3(2H)-carboxamide, 2-(3-aminopropyl)-5-(2,5-difluorophenyl)-N-methoxy-N-methyl-2-phenyl-1,3,4-thiadiazole-3(2H)-carboxamide, (S)-2-(3-aminopropyl)-5-(2,5-difluorophenyl)-N-methoxy-N-methyl-2-phenyl-1,3,4-thiadiazole-3(2H)-carboxamide, (R)-2-(3-aminopropyl)-5-(2,5-difluorophenyl)-N-methoxy-N-methyl-2-phenyl-1,3,4-thiadiazole-3(2H)-carboxamide, and 2-(3-aminopropyl)-5-(2,5-difluorophenyl)-N-hydroxy-N-methyl-2-phenyl-1,3,4-thiadiazole-3(2H)-carboxamide, or a pharmaceutically acceptable salt thereof. In a particular embodiment, the mitotic kinesin inhibitor is 2-(3-aminopropyl)-5-(2,5-difluorophenyl)-N-methoxy-N-methyl-2-phenyl-1,3,4-thiadiazole-3(2H)-carboxamide, or a pharmaceutically acceptable salt thereof. In another embodiment, the KSP inhibitor is (S)-2-(3-aminopropyl)-5-(2,5-difluorophenyl)-N-methoxy-N-methyl-2-phenyl-1,3,4-thiadiazole-3(2H)-carboxamide, or a pharmaceutically acceptable salt thereof. In other embodiments, the mitotic kinesin inhibitor is a compound or composition described and/or disclosed in U.S. Patent No. 7,795,282. One example of such compounds is 3-(5-(2,5-difluorophenyl)-3-(5-methyl-1,3,4-thiadiazol-2-yl)-2-phenyl-2,3-dihydro-1,3,4-thiadiazol-2-yl)propan-1-amine, or a pharmaceutically acceptable salt thereof. In other embodiments, the mitotic kinesin inhibitor is a compound or composition described and/or disclosed in any one of U.S. Patent Publication Nos. 2008/0182992, 2010/0041719 and 2010/045624. In a particular embodiment, the KSP inhibitor is ARRY-520 (Array BioPharma, Inc.), or a pharmaceutically acceptable salt thereof.

In other embodiments, the mitotic kinesin inhibitor is a compound or composition described and/or disclosed in U.S. Patent No. 7,465,746 . Examples of such compounds include (2S)-4-(2,5-difluorophenyl)-N-[(3S,4S)-3-fluoro-1-methylpiperidin-4-yl]-2-(hydroxymethyl)-N-methyl-2-phenyl-2,5-dihydro-1H-pyrrole-1-carboxamide, (2S)-4-(2,5-difluorophenyl)-N-[(3R,4R)-3-fluoro-1-methylpiperidin-4-yl]-2-(hydroxymethyl)-N-methyl-2-phenyl-2,5-dihydro-1H-pyrrole-1-carboxamide, (2S)-4-(2,5-difluorophenyl)-N-[(3S,4R)-3-fluoro-1-methylpiperidin-4-yl]-2-(hydroxymethyl)-N-methyl-2-phenyl-2,5-dihydro-1H-pyrrole-1-carboxamide, and (2S)-4-(2,5-difluorophenyl)-N-[(3R,4S)-3-fluoro-1-methylpiperidin-4-yl]-2-(hydroxymethyl)-N-methyl-2-phenyl-2,5-dihydro-1H-pyrrole-1-carboxamide, or a pharmaceutically acceptable salt thereof. In certain embodiments, the KSP inhibitor is (2S)-4-(2,5-difluorophenyl)-N-[(3R,4S)-3-fluoro-1-methylpiperidin-4-yl]-2-(hydroxymethyl)-N-methyl-2-phenyl-2,5-dihydro-1H-pyrrole-1-carboxamide, or a pharmaceutically acceptable salt thereof. In a particular embodiment, the mitotic kinesin inhibitor is MK-0731 (Merck). In other embodiments, the mitotic kinesin inhibitor is a compound or composition described and/or disclosed in any one of U.S. Patent Nos. 7,235,580 and 7,348,440 .

In other embodiments, the mitotic kinesin inhibitor is a compound or composition described and/or disclosed in any one of U.S. Patent Publication Nos. 2007/0203167, 2009/0176820 and 2010/0210681. In certain embodiments, the mitotic kinesin inhibitor is 1-[2-(dimethylamino)ethyl]-3-{[(2R,4aS,5R,10bS)-5-phenyl-9-(trifluoromethyl)-3,4,4a,5,6,10b-hexahydro-2H-pyrano[3,2-c]quinolin-2-yl]methyl}urea, or a pharmaceutically acceptable salt thereof. In a particular embodiment, the mitotic kinesin inhibitor is EMD 534085 (Merck-KGaA), or a pharmaceutically acceptable salt thereof.

In other embodiments, the mitotic kinesin inhibitor is a compound or composition described and/or disclosed in any one of U.S. Patent No. 7,498,333 and U.S. Patent Publication Nos. 2006/0041128, 2007/0249636 and 2009/0099210 . In certain embodiments, the mitotic kinesin inhibitor is (R)-N-(3-aminopropyl)-N-[1-(5-benzyl-3-methyl-4-oxo-4,5-dihydroisothiazolo[5,4-d]pyrimidin-6-yl)-2-methylpropyl]-4-methylbenzamide, or a pharmaceutically acceptable salt thereof. In a particular embodiment, the mitotic kinesin inhibitor is AZD4877 (AstraZeneca), or a pharmaceutically acceptable salt thereof.

In some embodiments, the mitotic kinesin inhibitor is a compound or composition described and/or disclosed in any one of U.S. Patent No. 7,425,636, U.S. Patent Publication Nos. 2007/0155804 and 2008/0194653, and International Patent Publication No. WO 2009/009470. In certain embodiments, the mitotic kinesin inhibitor is N-[4-(2,2-dimethyl-propionyl)-5-[(2-ethylamino-ethanesulfonylamino)-methyl]-5-phenyl-4,5-dihydro-[1,3,4]thiadiazol-2-yl]-2,2-dimethyl-propionamide, or a pharmaceutically acceptable salt thereof. In certain embodiments, the mitotic kinesin inhibitor is (R)-N-[4-(2,2-dimethyl-propionyl)-5-[(2-ethylamino-ethanesulfonylamino)-methyl]-5-phenyl-4,5-dihydro-[1,3,4]thiadiazol-2-yl]-2,2-dimethyl-propionamide, or a pharmaceutically acceptable salt thereof, (S)-N-[4-(2,2-dimethyl-propionyl)-5-[(2-ethylamino-ethanesulfonylamino)-methyl]-5-phenyl-4,5-dihydro-[1,3,4]thiadiazol-2-yl]-2,2-dimethyl-propionamide, or a pharmaceutically acceptable salt thereof, or a mixture of the two enantiomers. In a particular embodiment, the mitotic kinesin inhibitor is litronesib (also known as LY2523355; Eli Lilly/Kyowa Hakko), or a pharmaceutically acceptable salt thereof.

In some embodiments, the mitotic kinesin inhibitor is a compound or composition described and/or disclosed in U.S. Patent Publication No. 2009/0130097 . Examples of such compounds include N-(3-amino-propyl)-3-chloro-N-[(R)-1-(7-chloro-4-oxo-3-phenylamino-3,4-dihydro-quinazolin-2-yl)-but-3-ynyl]-2-fluoro-benzamide, N-(3-aminopropyl)-N-[1-(3-anilino-6-chloro-4-oxo-3,4-dihydroquinazolin-2-yl)propyl]-4-methylbenzamide, 2-{(R)-1-[(3-amino-propyl)-benzyl-amino]-propyl}-7-chloro-3-phenylamino-3H-quinazolin-4-one, 2-{(R)-1-[(3-amino-propyl)-(4-methyl-benzyl)-amino]-but-3-ynyl}-7-chloro-3-phenylamino-3H-quinazolin-4-one, N-(2-aminoethyl)-N-[1-(3-anilino-7-chloro-4-oxo-3,4-dihydroquinazolin-2-y-l)propyl]-3-chloro-2-fluorobenzamide, N-(3-amino-propyl)-N-[1-(7-chloro-4-oxo-3-phenylamino-3,4-dihydro-quinazo-lin-2-yl)-3-methylsulfanyl-propyl]-4-pyrazol-1-yl-benzamide, N-(3-amino-propyl)-N-[(R)-1-(7-chloro-4-oxo-3-phenylamino-3,4-dihydro-quinazolin-2-yl)-but-3-ynyl]-4-methyl-benzenesulfonamide, N-(3-amino-propyl)-N-[(R)-1-(7-chloro-4-oxo-3-phenylamino-3,4-dihydro-quinazolin-2-yl)-propyl]-3-fluoro-benzenesulfonamide, N-(3-aminopropyl)-N-[1-(3-anilino-7-chloro-4-oxo-3,4-dihydroquinazolin-2-yl)pentyl]-4-methylbenzamide, N-(3-aminopropyl)-N-[1-(3-anilino-7-chloro-4-oxo-3,4-dihydroquinazolin-2-yl)-3,3-dimethylbutyl]-4-bromobenzamide, N-(3-aminopropyl)-N-[1-(3-anilino-6-methyl-4-oxo-3,4-dihydroquinazolin-2-yl)propyl]-4-methylbenzamide, N-(3-aminopropyl)-N-[1-(7-chloro-4-oxo-3-phenoxy-3,4-dihydroquinazolin-2-yl)propyl]-4-methylbenzamide, N-(3-aminopropyl)-N-[(1R)-1-(3-anilino-7-chloro-4-oxo-3,4-dihydroquinazolin-2-yl)propyl]-1,3,5-trimethyl-1H-pyrazole-4-sulfonamide, N-(3-amino-propyl)-N-[(R)-1-(7-chloro-4-oxo-3-phenylamino-3,4-dihydroquinazolin-2-yl-but-3-ynyl]-2,3,5,6-tetrafluoro-benzamide, (R)-N-(3-aminopropyl)-N-(1-(7-chloro-4-oxo-3-(phenylamino)-3,4-dihydroquinazolin-2-yl)but-3-ynyl)-2,3,4,5-tetrafluorobenzamide, N-(3-aminopropyl)-3-chloro-N-(1-(7-chloro-4-oxo-3-(phenylamino)-3,4-dihydroquinazolin-2-yl)pent-3-ynyl)-2-fluorobenzamide, N-(3-amino-propyl)-N-[(R)-1-(7-chloro-4-oxo-3-phenylamino-3,4-dihydro-quinazolin-2-yl)-but-3-ynyl]-2,3-difluoro-4-methyl-benzamide, (R)-N-(3-aminopropyl)-N-(1-(7-chloro-4-oxo-3-phenylamino)-3,4-dihydroquinazolin-2-yl)but-3-ynyl)-2,3-difluoro-6-methoxybenzamide, (R)-N-(3-aminopropyl)-N-(1-(7-chloro-4-oxo-3-phenylamino)-3,4-dihydroquinazolin-2-yl)but-3-ynyl)-2,3-difluoro-4-methoxybenzamide, (R)-N-(3-aminopropyl)-4-chloro-N-(1-(7-chloro-4-oxo-3-phenylamino)-3,4-dihydroquinazolin-2-yl)but-3-ynyl)-2,6-difluoro-benzamide, (R)-N-(3-aminopropyl)-N-(1-(7-chloro-4-oxo-3-(phenylamino)-3,4-dihydroquinazolin-2-yl)but-3-ynyl)-3,5-difluorobenzamide, (R)-N-(3-aminopropyl)-N-(1-(7-chloro-4-oxo-3-(phenylamino)-3,4-dihydroquinazolin-2-yl)but-3-ynyl)-2,3,5-trifluorobenzamide, and (R)-N-(3-aminopropyl)-N-(1-(7-chloro-4-oxo-3-(phenylamino)-3,4-dihydroquinazolin-2-yl)but-3-ynyl)-2,3-difluorobenzamide, or a pharmaceutically acceptable salt thereof. In certain embodiments, the mitotic kinesin inhibitor is N-(3-amino-propyl)-3-chloro-N-[(R)-1-(7-chloro-4-oxo-3-phenylamino-3,4-dihydro-quinazolin-2-yl)-but-3-ynyl]-2-fluoro-benzamide, or a pharmaceutically acceptable salt thereof. In a particular embodiment, the mitotic kinesin inhibitor is ARQ 621 (ArQule), or a pharmaceutically acceptable salt thereof.

In some embodiments, the mitotic kinesin inhibitor is a compound or composition described and/or disclosed in any one of U.S. Patent No. 7,718,665, and U.S. Patent Publication Nos. 2006/0148838, 2008/0114017, 2008/0125452, 2009/0233902, 2009/0246169, 2010/0104659 and 2010/0183550 . In certain embodiments, the mitotic kinesin inhibitor is (3aS,10R)-2-(2-dimethylaminoethyl)-10-(3-hydroxyphenyl)-6-methoxy-3a-methyl-3a,4,9,10-tetrahydro-2,9,10a-triaza-cyclopenta-[b]fluorene-1,3-dione, or a pharmaceutically acceptable salt thereof. In other embodiments, the mitotic kinesin inhibitor is selected from (3aS,10R)-10-(3-hydroxy-phenyl)-2,3a-dimethyl-3a,4,9,10-tetrahydro-2,9,10a-triaza-cyclopenty[b]fluorene-1,3-dione, (3aS,10R)-10-(3-hydroxy-phenyl)-2-methyl-4,10-dihydro-3aH-9-thia-2,10-diaza-cyclopenta[b]fluorene-1,3-dione, (3aS,10R)-2-butyl-10-(3-hydroxy-phenyl)-4,10-dihydro-3aH-9-thia-2,10a-diaza-cyclopenta[b]fluorene-1,3-dione, (3aS,10R)-10-(3-hydroxy-phenyl)-2,3a-dimethyl-4,10-dihydro-3aH-9-thia-2,10a-diaza-cyclopenta[b]fluorene-1,3-dione, (3aS,10R)-2-(2-dimethylamino-ethyl)-10-(3-hydroxy-phenyl)-3a,4,9,10-tetrahydro-2,9,10a-triaza-cyclopenty[b]fluorene-1,3-dione, (3aS,10R)-2-(2-dimethylamino-ethyl)-10-(3-hydroxy-phenyl)-4,10-dihydro-3aH-9-thia-2,10-diaza-cyclopenta[b]fluorene-1,3-dione, (3aS,10R)-2-(2-dimethylamino-ethyl)-10-(3-hydroxyphenyl)-3a-methyl-4,10-dihydro-3aH-9-thia-2,10a-diaza-cyclopenta[b]fluorene-1,3-dione, (+/-)-(3aSR,10RS)-10-(3-hydroxy-phenyl)-2,3a-dimethyl-3a,4,9,10-tetrahydro-2,9,10a-triaza-cyclopenty[b]fluorene-1,3-dione, (3aSR,10RS)-10-(3-hydroxy-phenyl)-2,3a-dimethyl-4,10-dihydro-3aH-9-thia-2,10a-diaza-cyclopenta[b]fluorene-1,3-dione, (3aSR,10RS)-2-(2-dimethylamino-ethyl)-10-(3-hydroxy-phenyl)-3a-methyl-3a,4,9,10-tetrahydro-2,9,10a-triaza-cyclopenty[b]fluorene-1,3-dione, and (3aSR,10RS)-2-(2-dimethylamino-ethyl)-10-(3-hydroxy-phenyl)-3a-methyl-4,10-dihydro-3aH-9-thia-2,10a-diaza-cyclopenta[b]fluorene-1,3-dione, or a pharmaceutically acceptable salt thereof. In a particular embodiment, the mitotic kinesin inhibitor is 4SC-205 (4SC AG), or a pharmaceutically acceptable salt thereof.

In a particular embodiment, the mitotic kinesine inhibitor is CRi026 (combinatoRX) or a pharmaceutically acceptable salt thereof.

In some embodiments, the mitotic kinesin inhibitor is a compound or composition described and/or disclosed in International Patent Publication No. WO 2009/052288 In certain embodiments, the mitotic kinesin inhibitor is selected from compounds having any of the following structures, or a pharmaceutically acceptable salt thereof:

In particular embodiments, the mitotic kinesin inhibitor is a compound having the following structure, or a pharmaceutically acceptable salt thereof:

In certain embodiments, the mitotic kinesin inhibitor is SCH 2047069 (Schering Corporation), or a pharmaceutically acceptable salt thereof.

Also disclosed herein is use of an RNAi therapeutic. RNAi therapeutics may contain small interfering RNAs (siRNAs), micro RNAs (miRNAs), short hairpin RNAs (shRNAs), or combinations thereof. The RNAi therapeutic may target kinesin spindle protein (KSP), vascular endothelial growth factor (VEGF) or both. Examples of RNAi therapeutics are described and/or disclosed in U.S. Patent Nos. 7,718,629 and 7,786,290, and U.S. Patent Publication No. 2010/0280102. In some cases, the mitotic kinesin inhibitor contains siRNAs directed against VEGF and KSP. In certain cases, the mitotic kinesin inhibitor is a liposomal formulation containing siRNAs directed against VEGF and KSP. In particular cases, the RNAi therapeutic is ALN-VSP (Alnylam Pharmaceuticals).

In other embodiments, the mitotic kinesin inhibitor is an inhibitor of centromere-associated protein-E (CENP-E), for example, human CENP-E. Examples of CENP-E inhibitors include compounds disclosed and described in U.S. Patent Nos. 7,618,981, 7,504,413, and 7,582,668, and U.S. Patent Application Nos. 11/124,608, 12/350,114, 12/350,094 and 12/396,345). Examples of suitable CENP-E inhibitors include compounds of Formula VI: 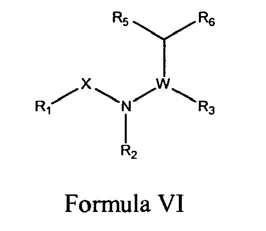 and pharmaceutically acceptable salts, solvates, chelates, non-covalent complexes, prodrugs, and mixtures thereof, wherein
R₁ is optionally substituted aryl or optionally substituted heteroaryl;
X is -CO or -SO₂-;
R₂ is hydrogen or optionally substituted lower alkyl;
W is --CR₄-, -CH₂CR₄-, or N;
R₃ is -CO-R₇, hydrogen, optionally substituted alkyl, optionally substituted heterocyclyl, cyano, optionally substituted sulfonyl, or optionally substituted aryl;
R₄ is hydrogen or optionally substituted alkyl;
R₅ is hydrogen, hydroxyl, optionally substituted amino, optionally substituted heterocyclyl; or optionally substituted lower alkyl;
R₆ is hydrogen, optionally substituted alkyl, optionally substituted alkoxy, optionally substituted aryloxy, optionally substituted heteraryloxy, optionally substituted alkoxycarbonyl-, optionally substituted aminocarbonyl-, optionally substituted aryl, optionally substituted heteroaryl, optionally substituted heterocyclyl, or optionally substituted aralkyl; and
R₇ is optionally substituted lower alkyl, optionally substituted aryl, hydroxyl, optionally substituted amino, optionally substituted aralkoxy, or optionally substituted alkoxy;
provided that if W is N, then R₅ is not hydroxyl or optionally substituted amino, and R₆ is not optionally substituted alkoxy, optionally substituted aralkoxy, optionally substituted heteroaralkoxy, or optionally substituted amino.

Additional examples of suitable CENP-E inhibitors include compounds of Formula VII: 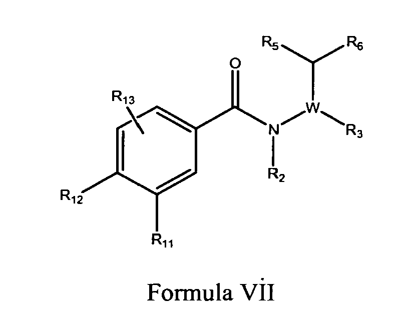 and pharmaceutically acceptable salts, solvates, chelates, non-covalent complexes, prodrugs, and mixtures thereof, wherein R₂, R₃, R₅, R₆, and W are as described for compounds of Formula VI and wherein
R₁₁ is optionally substituted heterocyclyl, optionally substituted lower alkyl, nitro, cyano, hydrogen, sulfonyl, or halo;
R₁₂ is hydrogen, halo, optionally substituted alkyl, optionally substituted amino, optionally substituted sulfanyl, optionally substituted alkoxy, optionally substituted aryloxy, optionally substituted heterocyclyl, or optionally substituted heteroaryloxy; and
R₁₃ is hydrogen, acyl, optionally substituted alkyl-, optionally substituted alkoxy, halo, hydroxyl, nitro, cyano, optionally substituted amino, alkylsulfonyl-, alkylsulfonamido-, alkylsulfonyl-, carboxyalkyl-, aminocarbonyl-, optionally substituted aryl or optionally substituted heteroaryl-.

Further examples of suitable CENP-E inhibitors include compounds of Formulae VIII and IX: 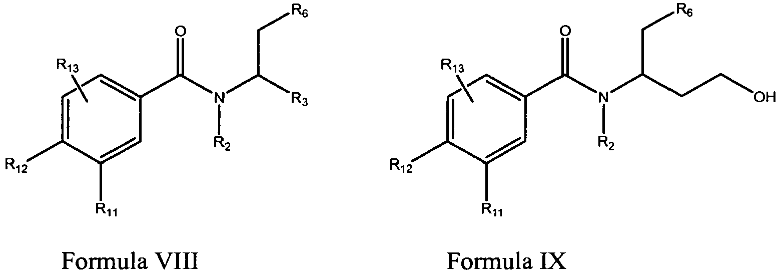 and pharmaceutically acceptable salts, solvates, chelates, non-covalent complexes, prodrugs, and mixtures thereof, wherein R₂, R₃, R₆, R₁₁, R₁₂, and R₁₃ are as described for compounds of Formula VII.

Other examples of suitable CENP-E inhibitors include compounds of Formula X: 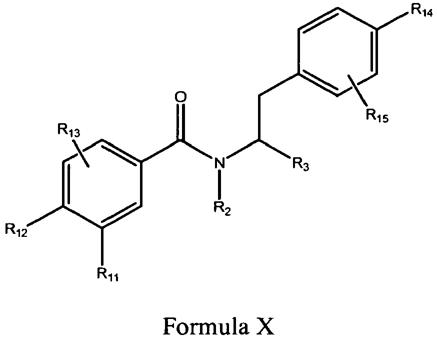 and pharmaceutically acceptable salts, solvates, chelates, non-covalent complexes, prodrugs, and mixtures thereof, wherein R₂, R₃, R₁₁, R₁₂, and R₁₃ are as described for compounds of Formula VII and wherein
R₁₄ is optionally substituted heteroaryl; and
R₁₅ is chosen from hydrogen, halo, hydroxyl, and lower alkyl.

Additional examples of suitable CENP-E inhibitors include compounds of Formula XI: 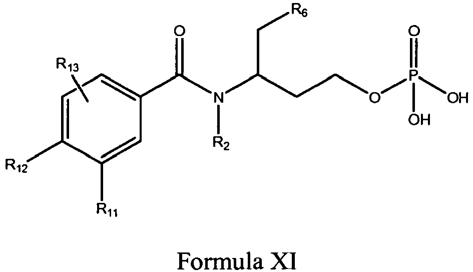 and pharmaceutically acceptable salts, solvates, chelates, non-covalent complexes, prodrugs, and mixtures thereof, wherein R₂, R₆, R₁₁, R₁₂, and R₁₃ are as described for compounds of Formula VI.

Further examples of suitable CENP-E inhibitors include compounds of Formula XII: 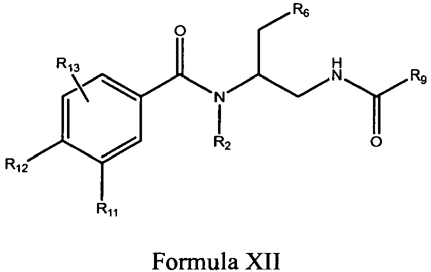 and pharmaceutically acceptable salts, solvates, chelates, non-covalent complexes, prodrugs, and mixtures thereof, wherein R₂, R₆, R₁₁, R₁₂, and R₁₃ are as described for compounds of Formula VII and wherein R₉ is chosen from optionally substituted amino and optionally substituted lower alkyl.

Other examples of suitable CENP-E inhibitors include compounds of Formula XII: 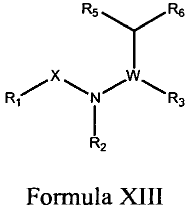 and pharmaceutically acceptable salts, solvates, chelates, non-covalent complexes, prodrugs, and mixtures thereof, wherein R₁, X, W, R₃, R₄, R₆, and R₇ are as defined for compounds of Formula VI and
R₂ and R₅, together with the atoms to which they are bound, form an optionally substituted 5-7 membered heterocycle which optionally may include one or two additional heteroatoms.

Additional examples of suitable CENP-E inhibitors include compounds of Formula XIV: 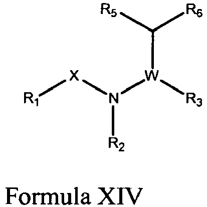 and pharmaceutically acceptable salts, solvates, chelates, non-covalent complexes, prodrugs, and mixtures thereof, wherein R₁, X, W, R₂, R₃, R₄, and R₇ are as defined for compounds of Formula VI and wherein
R₅ and R₆, together with the atoms to which they are bound, form an optionally substituted 5-7 membered heterocycle which optionally may include one or two heteroatoms.

Further examples of suitable CENP-E inhibitors include compounds of Formula XV: 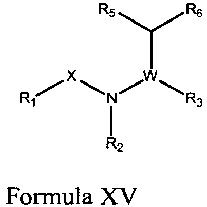 and pharmaceutically acceptable salts, solvates, chelates, non-covalent complexes, prodrugs, and mixtures thereof, wherein R₁, X ,W, R₄, R₅, R₆ and R₇ are as defined for compounds of Formula VI and wherein
R₂ and R₃, taken together with the atoms to which they are attached, form an optionally substituted 3- to 7-membered heterocyclic ring.

Other examples of suitable CENP-E inhibitors include compounds of Formula XVI: 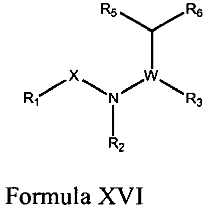 and pharmaceutically acceptable salts, solvates, chelates, non-covalent complexes, prodrugs, and mixtures thereof, wherein W, R₃, R₄, R₅, R₆ and R₇ are as defined for compounds of Formula VI and wherein
R₁, X, N, and R₂, taken together, form a substituted 2,4-dioxo-1,4-dihydro-2H-quinazolin-3-yl, 4-oxo-4H-quinazolin-3-yl, or 4-oxo-4H-pyridopyrimidin-3-yl ring.

Additional examples of suitable CENP-E inhibitors include compounds of Formula XVII: 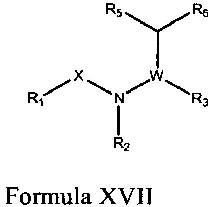 and pharmaceutically acceptable salts, solvates, chelates, non-covalent complexes, prodrugs, and mixtures thereof, wherein R₁, W, R₄, R₅, and R₆ are as defined for compounds of Formula VI and wherein -X-N(R₂)- is -C=N-; and
X taken together with R₃ forms an optionally substituted heterocyclic ring; in each case, provided that if W is N, then R₅ is not hydroxyl or optionally substituted amino, and R₆ is not optionally substituted alkoxy, optionally substituted aralkoxy, optionally substituted heteroaralkoxy, or optionally substituted amino.

Further examples of suitable CENP-E inhibitors include compounds of Formula XVIII: 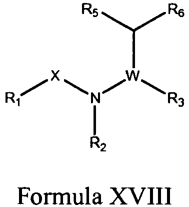 and pharmaceutically acceptable salts, solvates, chelates, non-covalent complexes, prodrugs, and mixtures thereof, wherein R₁, X, W, R₂, R₄, R₅, and R₇ are as defined for compounds of Formula VI and wherein
R₃ and R₆, together with the atoms to which they are bound, form an optionally substituted 5-7 membered heterocycle which optionally may include one or two additional heteroatoms.

In some embodiments, the mitotic kinesin inhibitor is a CENP-E inhibitor selected from N-(1-{4-[2-(1-acetylamino-ethyl)-1-ethyl-1H-imidazol-4-yl]-benzyl}-3-hydroxy-propyl)-3-chloro-4-(2,2,2-trifluoro-1-methyl-ethoxy)-benzamide, N-(2-(2-dimethylamino-acetylamino)-1-{4-[8-(1-hydroxy-ethyl)-imidazo[1,2-a]pyridin-2-yl]-benzyl}-ethyl)-3-chloro-4-isopropoxy-benzamide, N-(1-{4-[2-(1-methyl-1-hydroxy-ethyl)-1-ethyl-1H-imidazol-4-yl]-benzyl}-3-hydroxy-propyl)-3-chloro-4-(2,2,2-trifluoro-1-methyl-ethoxy)-benzamide, N-(2-(2-dimethylamino-acetylamino)-1-{4-[8-methyl-imidazo[1,2-a]pyridin-2-yl]-benzyl}-ethyl)-3-chloro-4-isopropoxy-benzamide, N-(1-{4-[2-(1-hydroxy-1-methyl-ethyl)-1-methyl-1H-imidazol-4-yl]-benzyl}-3-hydroxy-propyl)-3-chloro-4-(2,2,2-trifluoro-1-methyl-ethoxy)-benzamide, N-(2-(2-amino-2-methyl-propionylamino)-1-{4-[8-methyl-imidazo[1,2-a]pyridin-2-yl]-benzyl}-ethyl)-3-chloro-4-isopropoxy-benzamide, and N-{1-[4-(8-(1-hydroxy-ethyl)-imidazo[1,2-a]pyridin-2-yl)-benzyl]-3-hydroxy-propyl}-3-chloro-4-isopropoxy-benzamide, or a pharmaceutically acceptable salt thereof. In some embodiments, the mitotic kinesin inhibitor is the CENP-E inhibitor N-(2-(2-dimethylamino-acetylamino)-1-{4-[8-(1-hydroxy-ethyl)-imidazo[1,2-a]pyridin-2-yl]-benzyl}-ethyl)-3-chloro-4-isopropoxy-benzamide (also known as 3-Chloro-*N*-{(1S)-2-[(*N*,*N*-dimethylglycyl)amino]-1-[(4-{8-[(1S)-1-hydroxyethyl] imidazo[1,2-*a*]pyridin-2-yl}phenyl)methyl]ethyl}-4-[(1-methylethyl)oxy]benzamide), or a pharmaceutically acceptable salt thereof.

As used herein, alkyl refers to linear, branched, or cyclic aliphatic C₁-C₂₀ hydrocarbon structures and combinations thereof, which structures may be saturated or unsaturated. Lower-alkyl refers to alkyl groups of from 1 to 6 carbon atoms, or from 1 to 5 carbon atoms, or from 1 to 4 carbon atoms. Examples of lower-alkyl groups include methyl, ethyl, propyl, isopropyl, butyl, s-and t-butyl and the like. Cycloalkyl is a subset of alkyl and includes cyclic aliphatic hydrocarbon groups of from 3 to 13 carbon atoms. Examples of cycloalkyl groups include c-propyl, c-butyl, c-pentyl, norbornyl, adamantyl and the like. Cycloalkyl-alkyl- is another subset of alkyl and refers to cycloalkyl attached to the parent structure through a noncyclic alkyl. Examples of cycloalkyl-alkyl- include cyclohexylmethyl, cyclopropylmethyl, cyclohexylpropyl, and the like. Alkyl includes alkanyl, alkenyl and alkynyl residues; it is intended to include vinyl, allyl, isoprenyl and the like. Alkylene-, alkenylene-, and alkynylene- are other subsets of alkyl, including the same residues as alkyl, but having two points of attachment within a chemical structure. Examples of alkylene include ethylene (-CH₂CH₂-), propylene (-CH₂CH₂CH₂-), dimethylpropylene (-CH₂C(CH₃)₂CH₂-) and cyclohexylpropylene (-CH₂CH₂CH(C₆H₁₃)-). Likewise, examples of alkenylene include ethenylene (-CH=CH-), propenylene (-CH=CH-CH₂-), and cyclohexylpropenylene (-CH=CHCH(C₆H₁₃)- ). Examples of alkynylene include ethynylene (-C=C-) and propynylene (-CH=CH-CHz-₎. When an alkyl residue having a specific number of carbons is named, all geometric isomers having that number of carbons are intended to be encompassed; thus, for example, "butyl" is meant to include n-butyl, sec-butyl, isobutyl and t-butyl; "propyl" includes n-propyl, isopropyl, and c-propyl.

As used herein, alkoxy or alkoxyl refers to an alkyl group, for example, including from 1 to 8 carbon atoms, of a straight, branched, or cyclic configuration, or a combination thereof, attached to the parent structure through an oxygen (i.e., the group alkyl-O-). Examples include methoxy-, ethoxy-, propoxy-, isopropoxy-, cyclopropyloxy-, cyclohexyloxy- and the like. Lower-alkoxy refers to alkoxy groups containing 1 to 6 carbon atoms, or 1 to 4 carbon atoms.

As used herein, acyl refers to groups of from 1 to 8 carbon atoms of a straight, branched, or cyclic configuration or a combination thereof, attached to the parent structure through a carbonyl functionality. Such groups may be saturated or unsaturated, and aliphatic or aromatic. One or more carbons in the acyl residue may be replaced by nitrogen, oxygen or sulfur as long as the point of attachment to the parent remains at the carbonyl. Examples include acetyl, benzoyl, propionyl, isobutyryl, t-butoxycarbonyl, benzyloxycarbonyl and the like. Lower-acyl refers to acyl groups containing 1 to 6 carbon atoms, or 1 to 4 carbon atoms.

As used herein, amino refers to the group -NH₂. The term "substituted amino" refers to the group -NHR or -NRR where each R is independently selected from optionally substituted alkyl, optionally substituted alkoxy, optionally substituted amino carbonyl, optionally substituted aryl, optionally substituted heteroaryl, optionally substituted heterocyclyl, acyl, alkoxycarbonyl, sulfanyl, sulfinyl and sulfonyl, e.g., diethylamino, methylsulfonylamino, furanyl-oxy-sulfonamino.

As used herein, aminocarbonyl refers to the group -NR^{c}COR^{b}, -NR^{c}CO₂R^{b} ,or - NR^{c}CONR^{b}R^{c}, where
R^{b} is H or optionally substituted C₁-C₆ alkyl, aryl, heteroaryl, aryl-C₁-C₄ alkyl-, or heteroaryl-C₁-C₄ alkyl- group; and
R^{c} is hydrogen or C₁-C₄ alkyl; and
where each optionally substituted R^{b} group is independently unsubstituted or substituted with one or more substituents independently selected from C₁-C₄ alkyl, aryl, heteroaryl, aryl-C₁-C₄ alkyl-, heteroaryl-C₁-C₄ alkyl-, C₁-C₄ haloalkyl, -OC₁-C₄ alkyl, -OC₁-C₄ alkylphenyl, -C₁-C₄ alkyl-OH, -OC₁-C₄ haloalkyl, halogen, -OH, -NH₂, -C₁-C₄ alkyl-NH₂, -N(C₁-C₄ alkyl)(C₁-C₄ alkyl), -NH(C₁-C₄ alkyl), -N(C₁-C₄ alkyl)(C₁-C₄ alkylphenyl), -NH(C₁-C₄ alkylphenyl), cyano, nitro, oxo (as a substitutent for heteroaryl), -CO₂H, -C(O)OC₁-C₄ alkyl, -CON(C₁-C₄ alkyl)(C₁-C₄ alkyl), -CONH(C₁-C₄ alkyl), -CONH₂, -NHC(O)(C₁-C₄ alkyl), -NHC(O)(phenyl), -N(C₁-C₄ alkyl)C(O)(C₁-C₄ alkyl), -N(C₁-C₄ alkyl)C(O)(phenyl), -C(O)C₁-C₄ alkyl, -C(O)C₁-C₄ phenyl, -C(O)C₁-C₄ haloalkyl, -OC(O)C₁-C₄ alkyl, -SO₂(C₁-C₄ alkyl), - SO₂(phenyl), -SO₂(C₁-C₄ haloalkyl), -SO₂NH₂, -SO₂NH(C₁-C₄ alkyl), -SO₂NH(phenyl), - NHSO₂(C₁-C₄ alkyl), -NHSO₂(phenyl), and -NHSO₂(C₁-C₄ haloalkyl).

As used herein, aryl and heteroaryl mean a 5- or 6-membered aromatic or heteroaromatic ring containing 0 or 1-4 heteroatoms, respectively, selected from O, N, or S; a bicyclic 9- or 10-membered aromatic or heteroaromatic ring system containing 0 or 1-4 (or 1-6) heteroatoms, respectively, selected from O, N, or S; or a tricyclic 12- to 14-membered aromatic or heteroaromatic ring system containing 0 or 1-4 (or 1-6) heteroatoms, respectively, selected from O, N, or S. The aromatic 6- to 14-membered carbocyclic rings include, e.g., phenyl, naphthyl, indanyl, tetralinyl, and fluorenyl and the 5- to 10-membered aromatic heterocyclic rings include, e.g., imidazolyl, pyridinyl, indolyl, thienyl, benzopyranonyl, thiazolyl, furanyl, benzimidazolyl, quinolinyl, isoquinolinyl, quinoxalinyl, pyrimidinyl, pyrazinyl, tetrazolyl and pyrazolyl.

As used herein, aralkyl refers to a residue in which an aryl moiety (e.g., a C₆ or a C₁₀ aryl) is attached to the parent structure via an alkyl residue. The alkyl portion may include, for example, 1 to 6, or 1 to 4, carbon atoms. Examples include benzyl, phenethyl, phenylvinyl, phenylallyl and the like. Heteroaralkyl- refers to a residue in which a heteroaryl moiety (e.g., a 5-, 6-, 9- or 10-membered heteroaryl) is attached to the parent structure via an alkyl residue. The alkyl portion may include, for example, 1 to 6, or 1 to 4, carbon atoms. Examples include furanylmethyl, pyridinylmethyl, pyrimidinylethyl and the like.

As used herein, aralkoxy refers to the group -O-aralkyl. Similarly, heteroaralkoxy-refers to the group -O-heteroaralkyl; aryloxy- refers to the group -O-aryl; acyloxy- refers to the group -O-acyl; heteroaryloxy- refers to the group -0-heteroaryl; and heterocyclyloxy-refers to the group -O-heterocyclyl (i.e., aralkyl, heteroaralkyl, aryl, acyl, heterocyclyl, or heteroaryl is attached to the parent structure through an oxygen).

As used herein, carboxyalkyl- refers to the group -alkyl-COOH.

As used herein, carboxamido refers to the group -CONR^{b}R^{c}, where
R^{b} is H or optionally substituted C₁-C₆ alkyl, aryl, heteroaryl, aryl-C₁-C₄ alkyl-, or heteroaryl-C₁-C₄ alkyl- group; and
R^{c} is hydrogen or C₁-C₄ alkyl; and
where each optionally substituted R^{b} group is independently unsubstituted or substituted with one or more substituents independently selected from C₁-C₄ alkyl, aryl, heteroaryl, aryl-C₁-C₄ alkyl-, heteroaryl-C₁-C₄ alkyl-, C₁-C₄ haloalkyl, -OC₁-C₄ alkyl, -OC₁-C₄ alkylphenyl, -C₁-C₄ alkyl-OH, -OC₁-C₄ haloalkyl, halogen, -OH, -NH₂, -C₁-C₄ alkyl-NH₂, -N(C₁-C₄ alkyl)(C₁-C₄ alkyl), -NH(C₁-C₄ alkyl), -N(C₁-C₄ alkyl)(C₁-C₄ alkylphenyl), -NH(C₁-C₄ alkylphenyl), cyano, nitro, oxo (as a substitutent for heteroaryl), -CO₂H, -C(O)OC₁-C₄ alkyl, -CON(C₁-C₄ alkyl)(C₁-C₄ alkyl), -CONH(C₁-C₄ alkyl), -CONH₂, -NHC(O)(C₁-C₄ alkyl), -NHC(O)(phenyl), -N(C₁-C₄ alkyl)C(O)(C₁-C₄ alkyl), -N(C₁-C₄ alkyl)C(O)(phenyl), -C(O)C₁-C₄ alkyl, -C(O)C₁-C₄ phenyl, -C(O)C₁-C₄ haloalkyl, -OC(O)C₁-C₄ alkyl, -SO₂(C₁-C₄ alkyl), - SO₂(phenyl), -SO₂(C₁-C₄ haloalkyl), -SO₂NH₂, -SO₂NH(C₁-C₄ alkyl), -SO₂NH(phenyl), - NHSO₂(C₁-C₄ alkyl), -NHSO₂(phenyl), and -NHSO₂(C₁-C₄ haloalkyl).

As used herein, halogen or halo refers to fluorine, chlorine, bromine or iodine. In some embodiments, halogens are selected from fluorine, chlorine and bromine. Dihaloaryl, dihaloalkyl, trihaloaryl etc. refer to aryl and alkyl substituted with the designated plurality of halogens (here, 2, 2 and 3, respectively), but not necessarily a plurality of the same halogen; thus 4-chloro-3-fluorophenyl is within the scope of dihaloaryl.

As used herein, heterocyclyl means a cycloalkyl or aryl residue in which one to four of the carbons is replaced by a heteroatom such as oxygen, nitrogen or sulfur. Examples of heterocycles that fall within the scope of the invention include azetidinyl, imidazolinyl, pyrrolidinyl, pyrazolyl, pyrrolyl, indolyl, quinolinyl, isoquinolinyl, tetrahydroisoquinolinyl, benzofuranyl, benzodioxanyl, benzodioxyl (commonly referred to as methylenedioxyphenyl, when occurring as a substituent), tetrazolyl, morpholinyl, thiazolyl, pyridinyl, pyridazinyl, piperidinyl, pyrimidinyl, thienyl, furanyl, oxazolyl, oxazolinyl, isoxazolyl, dioxanyl, tetrahydrofuranyl and the like. "N-heterocyclyl" refers to a nitrogen-containing heterocycle. The term heterocyclyl encompasses heteroaryl, which is a subset of heterocyclyl. Examples of N-heterocyclyl residues include azetidinyl, 4-morpholinyl, 4-thiomorpholinyl, 1-piperidinyl, 1-pyrrolidinyl, 3-thiazolidinyl, piperazinyl and 4-(3,4-dihydrobenzoxazinyl). Examples of substituted heterocyclyl include 4-methyl-1-piperazinyl and 4-benzyl-1-piperidinyl.

As used herein, substituted alkoxy refers to alkoxy wherein the alkyl constituent is substituted (i.e., -O-(substituted alkyl)). One substituted alkoxy group is "polyalkoxy" or -O-(optionally substituted alkylene)-(optionally substituted alkoxy), and includes groups such as -OCH₂CH₂OCH₃, and residues of glycol ethers such as polyethyleneglycol, and -O(CH₂CH₂O)ₓCH₃, where x is an integer of about 2-20, or about 2-10, and or about 2-5. Another substituted alkoxy group is hydroxyalkoxy or -OCH₂(CH₂)_{y}OH, where y is an integer of about 1-10, or about 1-4.

As used herein, substituted alkyl, aryl, and heteroaryl, which includes the substituted alkyl, aryl and heteroaryl moieties of any group containing an optionally substituted alkyl, aryl and heteroaryl moiety (e.g., alkoxy, aralkyl and heteroaralkyl), refer respectively to alkyl, aryl, and heteroaryl wherein one or more (up to about 5, or up to about 3) hydrogen atoms are replaced by a substituent independently selected from the group:
-R^{a}, -OR^{b}, -O(C₁-C₂ alkyl)O- (as an aryl substituent), -SR^{b}, -NR^{b}R^{c}, halogen, cyano, nitro, -COR^{b}, -CO₂R^{b}, -CONR^{b}R^{c}, -OCOR^{b}, -OCO₂R^{b}, -OCONR^{b}R^{c}, -NR^{c}COR^{b}, -NR^{c}CO₂R^{b}, -NR^{c}CONR^{b}R^{c}, -CO₂R^{b}, -CONR^{b}R^{c}, -NR^{c}COR^{b}, -SOR^{a}, -SO₂R^{a}, -SO₂NR^{b}R^{c}, and -NR^{c}SO₂R^{a},
where R^{a} is an optionally substituted C₁-C₆ alkyl, aryl, heteroaryl, aryl-C₁-C₄ alkyl-, or heteroaryl-C₁-C₄ alkyl- group,
R^{b} is H or optionally substituted C₁-C₆ alkyl, aryl, heteroaryl, aryl-C₁-C₄ alkyl-, or heteroaryl-C₁-C₄ alkyl- group;
R^{c} is hydrogen or C₁-C₄ alkyl;
where each optionally substituted R^{a} group and R^{b} group is independently unsubstituted or substituted with one or more substituents independently selected from C₁-C₄ alkyl, aryl, heteroaryl, aryl-C₁-C₄ alkyl-, heteroaryl-C₁-C₄ alkyl-, C₁-C₄ haloalkyl, -OC₁-C₄ alkyl, -OC₁-C₄ alkylphenyl, -C₁-C₄ alkyl-OH, -OC₁-C₄ haloalkyl, halogen, -OH, -NH₂, -C₁-C₄ alkyl-NH₂, -N(C₁-C₄ alkyl)(C₁-C₄ alkyl), -NH(C₁-C₄ alkyl), -N(C₁-C₄ alkyl)(C₁-C₄ alkylphenyl), -NH(C₁-C₄ alkylphenyl), cyano, nitro, oxo (as a substitutent for heteroaryl), -CO₂H, -C(O)OC₁-C₄ alkyl, -CON(C₁-C₄ alkyl)(C₁-C₄ alkyl), -CONH(C₁-C₄ alkyl), -CONH₂, -NHC(O)(C₁-C₄ alkyl), -NHC(O)(phenyl), -N(C₁-C₄ alkyl)C(O)(C₁-C₄ alkyl), -N(C₁-C₄ alkyl)C(O)(phenyl), -C(O)C₁-C₄ alkyl, -C(O)C₁-C₄ phenyl, -C(O)C₁-C₄ haloalkyl, -OC(O)C₁-C₄ alkyl, -SO₂(C₁-C₄ alkyl),-SO₂(phenyl), -SO₂(C₁-C₄ haloalkyl), -SO₂NH₂, -SO₂NH(C₁-C₄ alkyl), -SO₂NH(phenyl), - NHSO₂(C₁-C₄ alkyl), -NHSO₂(phenyl), and -NHSO₂(C₁-C₄ haloalkyl).

As used herein, sulfanyl refers to the groups: -S-(optionally substituted alkyl), -S-(optionally substituted aryl), -S-(optionally substituted heteroaryl), and -S-(optionally substituted heterocyclyl).

As used herein, sulfinyl refers to the groups: -S(O)-H, -S(O)-(optionally substituted alkyl), -S(O)-optionally substituted aryl), -S(O)-(optionally substituted heteroaryl), -S(O)-(optionally substituted heterocyclyl); and -S(O)-(optionally substituted amino).

As used herein, sulfonyl refers to the groups: -S(O₂)-H, -S(O₂)-(optionall substituted alkyl), -S(O₂)-optionally substituted aryl), -S(O₂)-(optionally substituted heteroaryl), -S(O₂)-(optionally substituted heterocyclyl),-S(O₂)-(optionally substituted alkoxy), -S(O₂)-optionally substituted aryloxy), -S(O₂)-(optionally substituted heteroaryloxy), -S(O₂)-(optionally substituted heterocyclyloxy); and -S(O₂)-(optionally substituted amino).

As used herein, optional or optionally means that the subsequently described event or circumstance may or may not occur, and that the description includes instances where said event or circumstances occurs and instances in which it does not. For example, "optionally substituted alkyl" includes "alkyl" and "substituted alkyl" as defined herein. It will be understood by those skilled in the art with respect to any group containing one or more substituents that such groups are not intended to introduce any substitution or substitution patterns that are sterically impractical and/or synthetically non-feasible and/or inherently unstable.

As used herein, pharmaceutically acceptable salts refers to those salts that retain the biological effectiveness of the free compound and that are not biologically or otherwise undesirable, formed with a suitable acid or base, and includes pharmaceutically acceptable acid addition salts and base addition salts. Pharmaceutically acceptable acid addition salts include those derived from inorganic acids such as hydrochloric acid, hydrobromic acid, sulfuric acid, nitric acid, phosphoric acid and the like, and those derived from organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid and the like. Pharmaceutically acceptable base addition salts include those derived from inorganic bases such as sodium, potassium, lithium, ammonium, calcium, magnesium, iron, zinc, copper, manganese, aluminum salts and the like. In some embodiments, the pharmaceutically acceptable base addition salt is chosen from ammonium, potassium, sodium, calcium, and magnesium salts. Base addition salts also include those derived from pharmaceutically acceptable organic non-toxic bases, including salts of primary, secondary, and tertiary amines, substituted amines including naturally occurring substituted amines, cyclic amines and basic ion exchange resins, such as isopropylamine, trimethylamine, diethylamine, triethylamine, tripropylamine, and ethanolamine. Examples of pharmaceutically acceptable salts and methods of making them can be found, for example, in Berge et al., Pharmaceutical Salts, J. Pharmaceutical Sciences, January 1977, 66(1), 1-19.

As used herein, solvate refers to the compound formed by the interaction of a solvent and a compound. Suitable solvates are pharmaceutically acceptable solvates, such as hydrates, including monohydrates and hemi-hydrates.

Many of the compounds described herein contain one or more asymmetric centers and may thus give rise to enantiomers, diastereomers, and other stereoisomeric forms that may be defined, in terms of absolute stereochemistry, as (R) or (S). The present methods are meant to include all such possible isomers, including racemic mixtures, optically pure forms and intermediate mixtures. Optically active (R) and (S) isomers may be prepared using chiral synthons or chiral reagents, or resolved using conventional techniques. When the compounds described herein contain olefinic double bonds or other centers of geometric asymmetry, and unless specified otherwise, it is intended that the compounds include both E and Z geometric isomers. Likewise, all tautomeric forms and rotational isomers are also intended to be included.

When desired, the (R) and (S) isomers may be resolved by methods known to those skilled in the art, for example by formation of diastereoisomeric salts or complexes which may be separated, for example, by crystallization; via formation of diastereoisomeric derivatives which may be separated, for example, by crystallization, gas-liquid or liquid chromatography; selective reaction of one enantiomer with an enantiomer-specific reagent, for example enzymatic oxidation or reduction, followed by separation of the modified and unmodified enantiomers; or gas-liquid or liquid chromatography in a chiral environment, for example on a chiral support, such as silica with a bound chiral ligand or in the presence of a chiral solvent. It will be appreciated that where the desired enantiomer is converted into another chemical entity by one of the separation procedures described above, a further step may be required to liberate the desired enantiomeric form. Alternatively, specific enantiomer may be synthesized by asymmetric synthesis using optically active reagents, substrates, catalysts or solvents, or by converting one enantiomer to the other by asymmetric transformation.

PAH can be idiopathic (IPAH) or it can be associated with known causes (associated PAH or APAH). In some embodiments, mitotic kinesin inhibitors are used to treat idiopathic PAH. In other embodiments, mitotic kinesin inhibitors are used to treat associated PAH, including, for example, PAH associated with collagen vascular disease, congenital shunts between the systemic and pulmonary circulation, portal hypertension, HIV infection, drugs, toxins, glycogen storage disease, Gaucher's disease, hereditary haemorragic telangiectasia, haemoglobinopathies, myeloproliferative disorders, splenectomy, venoocclusive disease, or pulmonary capillary haemangiomatosis.

PAH occurs in approximately one in seven scleroderma (systemic sclerosis) patients and is a common cause of death for these patients. In some embodiments, mitotic kinesin inhibitors are used to treat PAH in subjects suffering from scleroderma. Acute PAH is also a frequent complication of allogenic bone marrow stem cell transplantation for malignant infantile osteoporosis, and late-onset PAH also occurs in association with graft-versus-host disease after allogenic stem cell transplantation. In some embodiments, mitotic kinesin inhibitors are used to treat PAH in subjects following allogenic bone marrow stem cell transplantation for malignant infantile osteoporosis and in subjects suffering from graft-versus-host disease after allogenic stem cell transplantation.

Familial PAH (FPAH) is caused or influenced by genetic factors. For example, studies have shown an association between polymorphisms of bone morphogenetic protein receptor type II (BMPR2) and familial PAH. In some embodiments, a mitotic kinesin inhibitor is used to treat familial PAH in a subject, for example, familial PAH associated with polymorphisms of BMPR2. Variations in other genes may also be associated with PAH. In some embodiments, a mitotic kinesin inhibitor is used to treat a subject suffering from familial PAH associated with variations in genes coding for (or regulating the expression or activity of) serotonin (5-HT), serotonin transporters (SERT), prostacyclin (PGI₂) receptors, PGI₂ synthase, voltage-dependent potassium channel (Kv) 1.5, nitric oxide, endothelin (ET)-1, ET-1 receptors A and/or B (ET_{A} and/or ET_{B}), or reactive oxygen species (e.g., NADPH oxidase).

In some embodiments, a subject suffering from PAH is screened for one or more genetic variations prior to being administered a mitotic kinesin inhibitor. For example, a subject may be screened for polymorphisms of BMPR2 and, based on the results of the genetic screen, a mitotic kinesin inhibitor may be administered to the subject.

In some embodiments, a mitotic kinesin inhibitor is administered to a subject suffering from PAH and who was previously treated with another PAH therapy. In some embodiments, the mitotic kinesin inhibitor and the other PAH therapy are administered sequentially. In some instances of sequential administration, the mitotic kinesin inhibitor is administered to the subject after the other PAH therapy has ended. The administration of the mitotic kinesin inhibitor may begin immediately following termination of the other PAH therapy, or there may be a time interval (e.g., one day, one week, one month, six months, one year, etc.) between the end of the other PAH therapy and the beginning of the mitotic kinesin inhibitor therapy. In other instances of sequential administration, the other PAH therapy is administered to the subject after the mitotic kinesin inhibitor therapy has ended. The administration of the other PAH therapy may begin immediately following termination of the administration of the mitotic kinesin inhibitor, or there may be a time interval (e.g., one day, one week, one month, six months, one year, etc.) between the end of the mitotic kinesin inhibitor therapy and the beginning of the other PAH therapy. In each instance, alternate administration may be repeated during a single treatment protocol. The determination of the order of administration and the number of repetitions of administration of each therapy during a treatment protocol is within the knowledge of the skilled physician after evaluation of the condition of the patient.

In other embodiments, the mitotic kinesin inhibitor is administered to the subject concurrently with the other PAH treatment, i.e., the mitotic kinesin inhibitor and the other PAH therapy are administered simultaneously, essentially simultaneously or within the same treatment protocol. In some instances of concurrent administration, administration of the mitotic kinesin inhibitor and the other PAH therapy begin and end at the same time (i.e., on the same day or within the same treatment protocol). In other instances of concurrent administration, only one of the mitotic kinesin inhibitor or the other PAH therapy is administered for a first period of time, followed by co-administration of the mitotic kinesin inhibitor and the other PAH therapy for a second period of time. For example, the subject may receive the other PAH therapy for a first period of time, then receive both the other PAH therapy and the mitotic kinesin inhibitor for a second period of time. Administration of either the mitotic kinesin inhibitor or the other PAH therapy may then continue for a third period of time. In another example, the subject may receive the mitotic kinesin inhibitor for a first period of time, then receive both the mitotic kinesin inhibitor and the other PAH therapy for a second period of time. Administration of either the mitotic kinesin inhibitor or the other PAH therapy may then continue for a third period of time. In other instances of concurrent administration, the mitotic kinesin inhibitor and the other PAH therapy are co-administered for a first period of time, followed by administration of only one of the mitotic kinesin inhibitor or the other PAH therapy for a second period of time. For example, the subject may receive both the mitotic kinesin inhibitor and the other PAH therapy for a first period of time, then receive the other PAH therapy for a second period of time. In another example, the subject may receive both the mitotic kinesin inhibitor and the other PAH therapy for a first period of time, then receive the mitotic kinesin inhibitor for a second period of time. In all instances, alternate administration may be repeated during a single treatment protocol. The determination of the order of administration and the number of repetitions of administration of each therapy during a treatment protocol is within the knowledge of the skilled physician after evaluation of the condition of the patient.

In some embodiments, the other PAH therapy is selected from one or more of prostanoids, endothelin receptor antagonists, phosphodiesterase inhibitors, prostacyclin receptor agonists (IP receptor agonists), anticoagulants, diuretics, calcium channel blockers, digoxin, oxygen therapy, nitric oxide therapy, tyrosine kinases, statins, 5-hydroxytryptamine (5-HT) receptor antagonists, phosphatidylinositol 3-kinase inhibitors, soluble guanylate cyclase activators, adrenomedullin, platelet-derived growth factor (PDGF) inhibitors, Rho-kinase inhibitors, mTOR inhibitors, lung transplantation, heart transplantation, and atrial septosomy.

In some embodiments, pulmonary arterial hypertension is treated in a subject by administering to the subject a therapeutically effective amount of a mitotic kinesin inhibitor and a prostanoid. Prostanoids include prostacyclin (epoprostenol, epoprostenol sodium) and prostacyclin analogs such as treprostinil sodium, iloprost, cisaprost, beraprost, and ciprostene sodium.

In some embodiments, pulmonary arterial hypertension is treated in a subject by administering to the subject a therapeutically effective amount of a mitotic kinesin inhibitor and an endothelin receptor antagonist. In some embodiments, the endothelin receptor antagonist is an agent that selectively or preferentially inhibits type A endothelin recpetor (ET_{A}). In some embodiments, the endothelin receptor antagonist is an agent that selectively or preferentially inhibits type B endothelin recpetor (ET_{B}). In some embodiments, the endothelin receptor antagonist is an agent that inhibits both ET_{A} and ET_{B}. In some embodiments, pulmonary arterial hypertension is treated in a subject by administering to the subject a therapeutically effective amount of a mitotic kinesin inhibitor, an agent that selectively or preferentially inhibits ET_{A}, and an agent that selectively or preferentially inhibits ET_{B}. Examples of endothelin receptor antagonists include bosentan, tazosentan, BQ123, ambrisentan, atrasentan, sitaxsentan, BQ788 and macitentan.

In some embodiments, pulmonary arterial hypertension is treated in a subject by administering to the subject a therapeutically effective amount of a mitotic kinesin inhibitor and a phosphodiesterase (PDE) inhibitor. PDE inhibitors include selective and non-selective inhibitors of any of the PDE isoforms, including, for example, PDE1 and PDE5 inhibitors. Examples of PDE inhibitors include sildenafil, tadalafil, vardenafil, udenafil avanafil, dipyridamole, and vinpocetine.

In some embodiments, pulmonary arterial hypertension is treated in a subject by administering to the subject a therapeutically effective amount of a mitotic kinesin inhibitor and a prostacyclin receptor agonist. An example of a prostacyclin receptor agonist is selexipag.

In some embodiments, pulmonary arterial hypertension is treated in a subject by administering to the subject a therapeutically effective amount of a mitotic kinesin inhibitor and an anticoagulant. Anticoagulants include, for example, coumarins (e.g., warfarin, acenocoumarol, phenprocoumon, phenindione), heparin, low molecular weight heparin, and direct thrombin inhibitors (e.g., argatroban, lepirudin, bivalirudin, dabigatran).

In some embodiments, pulmonary arterial hypertension is treated in a subject by administering to the subject a therapeutically effective amount of a mitotic kinesin inhibitor and a diuretic. Examples of diuretics include thiazide-like diuretics (e.g., chlorothiazide, chlorthalidone, hydrochlorothiazide, indapamide, metolazone), loop diuretics (e.g., bumetanide, furosemide, torsemide) and potassium-sparing diuretics (e.g., amiloride hydrochloride, spironolactone, triamterene).

In some embodiments, pulmonary arterial hypertension is treated in a subject by administering to the subject a therapeutically effective amount of a mitotic kinesin inhibitor and a calcuim channel blocker. Examples of calcium channel blockers include amlodipine, diltiazem, felodipine, isradipine, nicardipine, nifedipine, nisoldipine and verapamil.

In some embodiments, pulmonary arterial hypertension is treated in a subject by administering to the subject a therapeutically effective amount of a mitotic kinesin inhibitor and a tyrosine kinase. Tyrosine kinases include, for example, axitinib, bosutinib, cediranib, crizotinib, dasatinib, erlotinib, gefitinib, imatinib, lapatinib, lestaurtinib, neratinib, nilotinib, semaxanib, sorafenib, sunitinib, toceranib, vandetanib, and vatalanib.

In some embodiments, pulmonary arterial hypertension is treated in a subject by administering to the subject a therapeutically effective amount of a mitotic kinesin inhibitor and a statin. Examples of statins include atorvastatin, cerivastatin, fluvastatin, lovastatin, mevastatin, pitavastatin, pravastatin, rosuvastatin, simvastatin, simvastatin/ezetimibe, lovastatin/niacin, atorvastatin/amlodipine and simvastatin/niacin.

In some embodiments, pulmonary arterial hypertension is treated in a subject by administering to the subject a therapeutically effective amount of a mitotic kinesin inhibitor and a 5-HT receptor antagonist. 5-HT receptor antagonists include selective and non-selective antagonists of any of the 5-HT receptor isoforms, including, for example, 5-HT_{1B}, 5-HT_{2A} and/or 5-HT_{2C} receptor antagonists. Examples of 5-HT receptor antagonists include citalopram, clozapine, fluoxetine, ketanserin and paroxetine.

In some embodiments, pulmonary arterial hypertension is treated in a subject by administering to the subject a therapeutically effective amount of a mitotic kinesin inhibitor and a PDGF inhibitor. Examples of PDGF inhibitors include imatinib, sorafenib, sunitinib, lefluonamide, midostaurin, semaxanib and vatalanib.

In some embodiments, pulmonary arterial hypertension is treated in a subject by administering to the subject a therapeutically effective amount of a mitotic kinesin inhibitor and a soluble guanylate cyclase inhibitor. An example of a soluble guanylate cyclase activator is riociguat.

In some embodiments, pulmonary arterial hypertension is treated in a subject by administering to the subject a therapeutically effective amount of a mitotic kinesin inhibitor and a Rho-kinase inhibitor. An example of a Rho-kinase inhibitor is fasudil (fasudil hydrochloride).

In some embodiments, pulmonary arterial hypertension is treated in a subject by administering to the subject a therapeutically effective amount of a mitotic kinesin inhibitor and an mTOR inhibitor. Examples of mTOR inhibitors include rapamycin, temsirolimus and resveratrol.

In some embodiments, pulmonary arterial hypertension is treated in a subject by administering to the subject a therapeutically effective amount of *N*-(3-aminopropyl)-*N-*[(1*R*)-1-(3-benzyl-7-chloro-4-oxo-3,4-dihydroquinazolin-2-yl)-2-methylpropyl]-4-methylbenzamide monomethanesulfonate (ispinesib mesylate). In some embodiments, pulmonary arterial hypertension is treated in a subject by administering to the subject a therapeutically effective amount of ispinesib mesylate and a second therapy. In some embodiments, pulmonary arterial hypertension is treated in a subject by administering to the subject a therapeutically effective amount of ispinesib mesylate and an endothelin receptor antagonist. In some embodiments, pulmonary arterial hypertension is treated in a subject by administering to the subject a therapeutically effective amount of ispinesib mesylate and a type A endothelin receptor (ET_{A}) antagonist. In some embodiments, pulmonary arterial hypertension is treated in a subject by administering to the subject a therapeutically effective amount of ispinesib mesylate and a type B endothelin receptor (ET_{B}) antagonist. In some embodiments, pulmonary arterial hypertension is treated in a subject by administering to the subject a therapeutically effective amount of ispinesib mesylate and an antagonist of both ET_{A} and ET_{B}. In some embodiments, pulmonary arterial hypertension is treated in a subject by administering to the subject a therapeutically effective amount of ispinesib mesylate and bosentan. In some embodiments, pulmonary arterial hypertension is treated in a subject by administering to the subject a therapeutically effective amount of ispinesib mesylate and ambrisentan.

In some embodiments, pulmonary arterial hypertension is treated in a subject by administering to the subject a therapeutically effective amount of ispinesib mesylate and a prostanoid. In some embodiments, pulmonary arterial hypertension is treated in a subject by administering to the subject a therapeutically effective amount of ispinesib mesylate and treprostinil sodium. In some embodiments, pulmonary arterial hypertension is treated in a subject by administering to the subject a therapeutically effective amount of ispinesib mesylate and iloprost. In some embodiments, pulmonary arterial hypertension is treated in a subject by administering to the subject a therapeutically effective amount of ispinesib mesylate and epoprostenol (e.g., epoprostenol sodium).

In some embodiments, pulmonary arterial hypertension is treated in a subject by administering to the subject a therapeutically effective amount of *N*-(3-aminopropyl)-*N*-[(*R*)-(1-3-benzyl-7-chloro-4-oxo-4H-chromen-2-yl)-2-methyl-propyl]-4-methyl-benzamide hydrochloride hydrate. In some embodiments, pulmonary arterial hypertension is treated in a subject by administering to the subject a therapeutically effective amount of *N-*(3-aminopropyl)-*N*-[(*R*)-(1-3-benzyl-7-chloro-4-oxo-4H-chromen-2-yl)-2-methyl-propyl]-4-methyl-benzamide hydrochloride hydrate and a second therapy. In some embodiments, pulmonary arterial hypertension is treated in a subject by administering to the subject a therapeutically effective amount of *N*-(3-aminopropyl)-*N*-[(*R*)-(1-3-benzyl-7-chloro-4-oxo-4H-chromen-2-yl)-2-methyl-propyl]-4-methyl-benzamide hydrochloride hydrate and an endothelin receptor antagonist. In some embodiments, pulmonary arterial hypertension is treated in a subject by administering to the subject a therapeutically effective amount of *N-*(3-aminopropyl)-*N*-[(*R*)-(1-3-benzyl-7-chloro-4-oxo-4H-chromen-2-yl)-2-methyl-propyl]-4-methyl-benzamide hydrochloride hydrate and an ET_{A} antagonist. In some embodiments, pulmonary arterial hypertension is treated in a subject by administering to the subject a therapeutically effective amount of *N*-(3-aminopropyl)-*N*-[(*R*)-(1-3-benzyl-7-chloro-4-oxo-4H-chromen-2-yl)-2-methyl-propyl]-4-methyl-benzamide hydrochloride hydrate and an ET_{B} antagonist. In some embodiments, pulmonary arterial hypertension is treated in a subject by administering to the subject a therapeutically effective amount of *N*-(3-aminopropyl)-*N*-[(*R*)-(1-3-benzyl-7-chloro-4-oxo-4H-chromen-2-yl)-2-methyl-propyl]-4-methyl-benzamide hydrochloride hydrate and an antagonist of both ET_{A} and ET_{B}. In some embodiments, pulmonary arterial hypertension is treated in a subject by administering to the subject a therapeutically effective amount of *N*-(3-aminopropyl)-*N*-[(*R*)-(1-3-benzyl-7-chloro-4-oxo-4H-chromen-2-yl)-2-methyl-propyl]-4-methyl-benzamide hydrochloride hydrate and bosentan. In some embodiments, pulmonary arterial hypertension is treated in a subject by administering to the subject a therapeutically effective amount of *N*-(3-aminopropyl)-*N*-[(*R*)-(1-3-benzyl-7-chloro-4-oxo-4H-chromen-2-yl)-2-methyl-propyl]-4-methyl-benzamide hydrochloride hydrate and ambrisentan.

In some embodiments, pulmonary arterial hypertension is treated in a subject by administering to the subject a therapeutically effective amount of *N*-(3-aminopropyl)-*N*-[(*R*)-(1-3-benzyl-7-chloro-4-oxo-4H-chromen-2-yl)-2-methyl-propyl]-4-methyl-benzamide hydrochloride hydrate and a prostanoid. In some embodiments, pulmonary arterial hypertension is treated in a subject by administering to the subject a therapeutically effective amount of *N*-(3-aminopropyl)-*N*-[(*R*)-(1-3-benzyl-7-chloro-4-oxo-4H-chromen-2-yl)-2-methyl-propyl]-4-methyl-benzamide hydrochloride hydrate and treprostinil sodium. In some embodiments, pulmonary arterial hypertension is treated in a subject by administering to the subject a therapeutically effective amount of *N*-(3-aminopropyl)-*N*-[(*R*)-(1-3-benzyl-7-chloro-4-oxo-4H-chromen-2-yl)-2-methyl-propyl]-4-methyl-benzamide hydrochloride hydrate and iloprost. In some embodiments, pulmonary arterial hypertension is treated in a subject by administering to the subject a therapeutically effective amount of *N-*(3-aminopropyl)-*N*-[(*R*)-(1-3-benzyl-7-chloro-4-oxo-4H-chromen-2-yl)-2-methyl-propyl]-4-methyl-benzamide hydrochloride hydrate and epoprostenol (e.g., epoprostenol sodium).

In some cases not part of the present invention, idiopathic pulmonary fibrosis is treated in a subject by administering to the subject a therapeutically effective amount of a mitotic kinesin inhibitor. IPF is a type of idiopathic interstitial pneumonia, which also includes non-specific interstitial pneumonia (NSIP), desquamative interstitial pneumonia (DIP) and acute interstitial pneumonia (AIP). Examples of known causes of interstitial lung disease include sarcoidosis, hypersensitivity pneumonitis, pulmonary Langerhans cell histiocytosis, asbestosis and collagen vascular diseases such as scleroderma and rheumatoid arthritis. IPF may be the result of an autoimmune disorder, the effects of an infection (e.g., a viral infection), or other injuries to the lung. In some instances, IPF occurs with pulmonary hypertension. In some cases, IPF with secondary pulmonary arterial hypertension (PAH) is treated in a subject by administering to the subject a therapeutically effective amount of a mitotic kinesin inhibitor.

In some cases, a mitotic kinesin inhibitor is administered to a subject suffering from IPF and who was previously treated with another IPF therapy. In some cases, the mitotic kinesin inhibitor and the other IPF therapy are administered sequentially. In some instances of sequential administration, the mitotic kinesin inhibitor is administered to the subject after the other IPF therapy has ended. The administration of the mitotic kinesin inhibitor may begin immediately following termination of the other IPF therapy, or there may be a time interval (e.g., one day, one week, one month, six months, one year, etc.) between the end of the other IPF therapy and the beginning of the mitotic kinesin inhibitor therapy. In other instances of sequential administration, the other IPF therapy is administered to the subject after the mitotic kinesin inhibitor therapy has ended. The administration of the other IPF therapy may begin immediately following termination of the administration of the mitotic kinesin inhibitor, or there may be a time interval (e.g., one day, one week, one month, six months, one year, etc.) between the end of the mitotic kinesin inhibitor therapy and the beginning of the other IPF therapy. In each instance, alternate administration may be repeated during a single treatment protocol. The determination of the order of administration and the number of repetitions of administration of each therapy during a treatment protocol is within the knowledge of the skilled physician after evaluation of the condition of the patient.

In other cases, the mitotic kinesin inhibitor is administered to the subject concurrently with the other IPF treatment, i.e., the mitotic kinesin inhibitor and the other IPF therapy are administered simultaneously, essentially simultaneously or within the same treatment protocol. In some instances of concurrent administration, administration of the mitotic kinesin inhibitor and the other IPF therapy begin and end at the same time (i.e., on the same day or within the same treatment protocol). In other instances of concurrent administration, only one of the mitotic kinesin inhibitor or the other IPF therapy is administered for a first period of time, followed by co-administration of the mitotic kinesin inhibitor and the other IPF therapy for a second period of time. For example, the subject may receive the other IPF therapy for a first period of time, then receive both the other IPF therapy and the mitotic kinesin inhibitor for a second period of time. Administration of either the mitotic kinesin inhibitor or the other IPF therapy may then continue for a third period of time. In another example, the subject may receive the mitotic kinesin inhibitor for a first period of time, then receive both the mitotic kinesin inhibitor and the other IPF therapy for a second period of time. Administration of either the mitotic kinesin inhibitor or the other IPF therapy may then continue for a third period of time. In other instances of concurrent administration, the mitotic kinesin inhibitor and the other IPF therapy are co-administered for a first period of time, followed by administration of only one of the mitotic kinesin inhibitor or the other IPF therapy for a second period of time. For example, the subject may receive both the mitotic kinesin inhibitor and the other IPF therapy for a first period of time, then receive the other IPF therapy for a second period of time. In another example, the subject may receive both the mitotic kinesin inhibitor and the other IPF therapy for a first period of time, then receive the mitotic kinesin inhibitor for a second period of time. In all instances, alternate administration may be repeated during a single treatment protocol. The determination of the order of administration and the number of repetitions of administration of each therapy during a treatment protocol is within the knowledge of the skilled physician after evaluation of the condition of the patient.

In some cases, the other IPF therapy is selected from immune suppressive agents, such as corticosteroids (e.g., prednisone). The corticosteroid may be administered daily, for example, by an oral route. Other immunosuppressants, such as cyclophosphamide (Cytoxan), azathioprine, mycophenolate (e.g., mycophenolate mofetil), methotrexate, penicillamine, and cyclosporine, may be administered, either alone or in combination with prednisone or other corticosteroids. The antioxidant N-acetylcysteine may be administered along with any of these therapies, for example, in combination with prednisone and azathioprine, mycophenolate or cyclophosphamide. Other IPF treatments include, for example, chlorambucil, vincristine sulfate, colchicine, interferon gamma-1b, pirfenidone, bosentan, and combinations thereof.

In some cases, the other IPF treatment is oxygen therapy, either alone or in combination with one or more IPF medications described herein. In some cases, the other IPF therapy is transplantation of one or both lungs.

In some cases not part of the present invention, idiopathic pulmonary fibrosis is treated in a subject by administering to the subject a therapeutically effective amount of *N*-(3-aminopropyl)-*N*-[(1*R*)-1-(3-benzyl-7-chloro-4-oxo-3,4-dihydroquinazolin-2-yl)-2-methylpropyl]-4-methylbenzamide monomethanesulfonate (ispinesib mesylate). In some cases, idiopathic pulmonary fibrosis is treated in a subject by administering to the subject a therapeutically effective amount of ispinesib mesylate and a second therapy. In some cases, ideopathic pulmonary fibrosis (IPF) with secondary pulmonary arterial hypertension (PAH) is treated in a subject by administering to the subject a therapeutically effective amount of ispinesib mesylate. In some cases, ideopathic pulmonary fibrosis (IPF) with secondary pulmonary arterial hypertension (PAH) is treated in a subject by administering to the subject a therapeutically effective amount of ispinesib mesylate and a second therapy.

In some cases, idiopathic pulmonary fibrosis is treated in a subject by administering to the subject a therapeutically effective amount of *N*-(3-aminopropyl)-*N*-[(*R*)-(1-3-benzyl-7-chloro-4-oxo-4H-chromen-2-yl)-2-methyl-propyl]-4-methyl-benzamide hydrochloride hydrate. In some cases, idiopathic pulmonary fibrosis is treated in a subject by administering to the subject a therapeutically effective amount of *N*-(3-aminopropyl)-*N*-[(*R*)-(1-3-benzyl-7-chloro-4-oxo-4H-chromen-2-yl)-2-methyl-propyl]-4-methyl-benzamide hydrochloride hydrate and a second therapy. In some cases, ideopathic pulmonary fibrosis (IPF) with secondary pulmonary arterial hypertension (PAH) is treated in a subject by administering to the subject a therapeutically effective amount of *N*-(3-aminopropyl)-*N*-[(*R*)-(1-3-benzyl-7-chloro-4-oxo-4H-chromen-2-yl)-2-methyl-propyl]-4-methyl-benzamide hydrochloride hydrate. In some cases, ideopathic pulmonary fibrosis (IPF) with secondary pulmonary arterial hypertension (PAH) is treated in a subject by administering to the subject a therapeutically effective amount of *N*-(3-aminopropyl)-*N*-[(*R*)-(1-3-benzyl-7-chloro-4-oxo-4H-chromen-2-yl)-2-methyl-propyl]-4-methyl-benzamide hydrochloride hydrate and a second therapy.

In some cases not part of the present invention, lymphangioleiomyomatosis (LAM) is treated in a subject by administering to the subject a therapeutically effective amount of a mitotic kinesin inhibitor. LAM is a rare lung disease that results in a proliferation of disorderly smooth muscle growth (leiomyoma) throughout the lungs, in the bronchioles, alveolar septa, perivascular spaces, and lymphatics, resulting in the obstruction of small airways (leading to pulmonary cyst formation and pneumothorax) and lymphatics (leading to chylous pleural effusion). LAM occurs in a sporadic form, which mainly affects females usually of childbearing age. LAM also occurs in patients who have tuberous sclerosis (TSC), often referred to as TSC-LAM. In some cases, TSC-LAM is treated in a subject by administering to the subject a therapeutically effective amount of a mitotic kinesin inhibitor. TSC is a genetic disease caused by a defect in one or more of two genes, TSC1 and TSC2. Patients who have LAM may also have abnormal TSC1 and/or TSC2 genes. In some cases, LAM or TSC-LAM is treated in a subject who has abnormal TSC1 and/or TSC2 genes by administering to the subject a therapeutically effective amount of a mitotic kinesin inhibitor.

In some cases, a subject suffering from LAM or TSC-LAM is screened for one or more genetic variations prior to being administered a mitotic kinesin inhibitor. For example, a subject may be screened for polymorphisms of TSC1 and/or TSC2 and, based on the results of the genetic screen, a mitotic kinesin inhibitor may be administered to the subject.

About 6 out of 10 women who have LAM develop pneumothorax, or collapsed lung. In this condition, air leaks out of a lung and into the space between the lung and the chest wall (the pleural space). LAM can also be associated with kidney growths (angiomyolipomas) and/or lung cysts. Currently, treatments for LAM are aimed at easing symptoms and preventing complications; no treatment is available to stop the growth of the cysts and cell clusters that occur in LAM.

In some cases, a mitotic kinesin inhibitor is administered to a subject suffering from LAM or TSC-LAM and who was previously treated with another LAM or TSC-LAM therapy. In some cases, the mitotic kinesin inhibitor and the other LAM or TSC-LAM therapy are administered sequentially. In some instances of sequential administration, the mitotic kinesin inhibitor is administered to the subject after the other LAM or TSC-LAM therapy has ended. The administration of the mitotic kinesin inhibitor may begin immediately following termination of the other LAM or TSC-LAM therapy, or there may be a time interval (e.g., one day, one week, one month, six months, one year, etc.) between the end of the other LAM or TSC-LAM therapy and the beginning of the mitotic kinesin inhibitor therapy. In other instances of sequential administration, the other LAM or TSC-LAM therapy is administered to the subject after the mitotic kinesin inhibitor therapy has ended. The administration of the other LAM or TSC-LAM therapy may begin immediately following termination of the administration of the mitotic kinesin inhibitor, or there may be a time interval (e.g., one day, one week, one month, six months, one year, etc.) between the end of the mitotic kinesin inhibitor therapy and the beginning of the other LAM or TSC-LAM therapy. In each instance, alternate administration may be repeated during a single treatment protocol. The determination of the order of administration and the number of repetitions of administration of each therapy during a treatment protocol is within the knowledge of the skilled physician after evaluation of the condition of the patient.

In other cases, the mitotic kinesin inhibitor is administered to the subject concurrently with the other LAM or TSC-LAM treatment, i.e., the mitotic kinesin inhibitor and the other LAM or TSC-LAM therapy are administered simultaneously, essentially simultaneously or within the same treatment protocol. In some instances of concurrent administration, administration of the mitotic kinesin inhibitor and the other LAM or TSC-LAM therapy begin and end at the same time (i.e., on the same day or within the same treatment protocol). In other instances of concurrent administration, only one of the mitotic kinesin inhibitor or the other LAM or TSC-LAM therapy is administered for a first period of time, followed by co-administration of the mitotic kinesin inhibitor and the other LAM or TSC-LAM therapy for a second period of time. For example, the subject may receive the other LAM or TSC-LAM therapy for a first period of time, then receive both the other LAM or TSC-LAM therapy and the mitotic kinesin inhibitor for a second period of time. Administration of either the mitotic kinesin inhibitor or the other LAM or TSC-LAM therapy may then continue for a third period of time. In another example, the subject may receive the mitotic kinesin inhibitor for a first period of time, then receive both the mitotic kinesin inhibitor and the other LAM or TSC-LAM therapy for a second period of time. Administration of either the mitotic kinesin inhibitor or the other LAM or TSC-LAM therapy may then continue for a third period of time. In other instances of concurrent administration, the mitotic kinesin inhibitor and the other LAM or TSC-LAM therapy are co-administered for a first period of time, followed by administration of only one of the mitotic kinesin inhibitor or the other LAM or TSC-LAM therapy for a second period of time. For example, the subject may receive both the mitotic kinesin inhibitor and the other LAM or TSC-LAM therapy for a first period of time, then receive the other LAM or TSC-LAM therapy for a second period of time. In another example, the subject may receive both the mitotic kinesin inhibitor and the other LAM or TSC-LAM therapy for a first period of time, then receive the mitotic kinesin inhibitor for a second period of time. In all instances, alternate administration may be repeated during a single treatment protocol. The determination of the order of administration and the number of repetitions of administration of each therapy during a treatment protocol is within the knowledge of the skilled physician after evaluation of the condition of the patient.

In some cases, the other LAM or TSC-LAM therapy is selected from one or more of medicines to improve air flow in the lungs and reduce wheezing (e.g., bronchodilators); medicines to prevent bone loss (osteoporosis); oxygen therapy; procedures to remove fluid from the chest or abdomen and stop it from building up again (e.g., thoracentesis, paracentesis); oophorectomy; procedures to shrink angiomyolipomas (AMLs); lung transplant; hormone therapy (e.g., progesterone, androgen); tamoxifen; gonadotropinreleasing hormone (GnRH) agonists; and rapamycin.

In some cases, LAM or TSC-LAM is treated in a subject by administering to the subject a therapeutically effective amount of *N*-(3-aminopropyl)-*N*-[(1R)-1-(3-benzyl-7-chloro-4-oxo-3,4-dihydroquinazolin-2-yl)-2-methylpropyl]-4-methylbenzamide monomethanesulfonate (ispinesib mesylate). In some cases, LAM or TSC-LAM is treated in a subject by administering to the subject a therapeutically effective amount of ispinesib mesylate and a second therapy.

In some cases, LAM or TSC-LAM is treated in a subject by administering to the subject a therapeutically effective amount of *N*-(3-aminopropyl)-*N*-[(*R*)-(1-3-benzyl-7-chloro-4-oxo-4H-chromen-2-yl)-2-methyl-propyl]-4-methyl-benzamide hydrochloride hydrate. In some cases, LAM or TSC-LAM is treated in a subject by administering to the subject a therapeutically effective amount of *N*-(3-aminopropyl)-*N*-[(*R*)-(1-3-benzyl-7-chloro-4-oxo-4H-chromen-2-yl)-2-methyl-propyl]-4-methyl-benzamide hydrochloride hydrate and a second therapy.

The mitotic kinesin inhibitor is administered at a therapeutically effective dosage, e.g., a dosage sufficient to provide treat or otherwise ameliorate the symptoms of PAH and/or IPF and/or LAM. While human dosage levels may not yet be optimized for the mitotic kinesin inhibitors described herein, generally, a daily dose ranges from about 0.05 to 100 mg/kg of body weight, from about 0.10 to 10.0 mg/kg of body weight, or from about 0.15 to 1.0 mg/kg of body weight. The amount of the mitotic kinesin inhibitor administered is dependent on the individual subject, the severity of the affliction, the manner and schedule of administration and the judgment of the prescribing physician.

Combination therapies have been used in the treatment of diseases; however, care must be taken when combining drugs since certain drugs can interact in a harmful manner. The combination of two agents that treat the same condition may result in an additive effect, meaning that the degree of the effect is the sum of the effect of each drug alone. Applicants disclose herein that the combination of a mitotic kinesin inhibitor and a second therapy (e.g., a second PAH therapy, a second IPF therapy, or a second LAM therapy) can result in a supra additive or synergistic effect. As used herein a "synergistic effect" means that the therapeutic effect observed with the combination therapy is greater than the sum of the individual therapeutic effects of each compound. One advantage of using a synergistic combination therapy is that less of each compound may be required to achieve a therapeutic effect, potentially resulting in fewer side effects from treatment. In some cases, side effects are not seen at the lower doses used. Also, in some cases, the side effect profile of one drug can mitigate or average out the side effect profile of the other drug. For example, one of the drugs may result in increased blood pressure and the other drug results in lowered blood pressure so that the combination therapy does not effect blood pressure. Another potential advantage of combination therapy is that, since less compound is required, the cost of therapy can be reduced.

In one embodiment, the amount of the mitotic kinesin inhibitor alone and/or the amount of the second therapy (e.g., a second PAH therapy, a second IPF therapy, or a second LAM therapy) alone are therapeutically sub-effective in treating the subject. As used herein, "therapeutically sub-effective" means that the mitotic kinesin inhibitor and/or the second therapy are provided in amounts that do not have a statistically significant effect on the measured parameter. For example, the amount of mitotic kinesin inhibitor alone and/or the amount of a second PAH therapy alone can be therapeutically ineffective in treating PAH in a subject. Similarly, the amount of mitotic kinesin inhibitor alone and/or the amount of a second IPF therapy alone can be therapeutically ineffective in treating IPF in a subject; or the amount of mitotic kinesin inhibitor alone and/or the amount of a second LAM therapy alone can be therapeutically ineffective in treating LAM in a subject. However, the combination of a therapeutically sub-effective amount of the mitotic kinesin inhibitor and/or a therapeutically sub-effective amount of the second therapy results in the desired therapeutic effect.

In some embodiments, pulmonary arterial hypertension (PAH) is treated in a subject by administering to the subject a therapeutically sub-effective amount of mitotic kinesin inhibitor and a therapeutically effective amount of a second PAH therapy. In some embodiments, PAH is treated in a subject by administering to the subject a therapeutically effective amount of mitotic kinesin inhibitor and a therapeutically sub-effective amount of a second PAH therapy. In some embodiments, PAH is treated in a subject by administering to the subject a therapeutically sub-effective amount of mitotic kinesin inhibitor and a therapeutically sub-effective amount of a second PAH therapy.

In some cases not part of the present invention, idiopathic pulmonary fibrosis (IPF) is treated in a subject by administering to the subject a therapeutically sub-effective amount of mitotic kinesin inhibitor and a therapeutically effective amount of a second IPF therapy. In some cases, IPF is treated in a subject by administering to the subject a therapeutically effective amount of mitotic kinesin inhibitor and a therapeutically sub-effective amount of a second IPF therapy. In some cases, IPF is treated in a subject by administering to the subject a therapeutically sub-effective amount of mitotic kinesin inhibitor and a therapeutically sub-effective amount of a second IPF therapy.

In some cases not part of the present invention, lymphangioleiomyomatosis (LAM) is treated in a subject by administering to the subject a therapeutically sub-effective amount of mitotic kinesin inhibitor and a therapeutically effective amount of a second LAM therapy. In some cases, LAM is treated in a subject by administering to the subject a therapeutically effective amount of mitotic kinesin inhibitor and a therapeutically sub-effective amount of a second PAH therapy. In some cases, LAM is treated in a subject by administering to the subject a therapeutically sub-effective amount of mitotic kinesin inhibitor and a therapeutically sub-effective amount of a second LAM therapy.

Administration of the mitotic kinesin inhibitor can be via any of the accepted modes of administration for therapeutic agents including, but not limited to, orally, sublingually, subcutaneously, intravenously, intranasally, topically, transdermally, intraperitoneally, intramuscularly, intrapulmonarily, vaginally, rectally, or intraocularly. In some embodiments, the mitotic kinesin inhibitor is administered orally. In other embodiments, the mitotic kinesin inhibitor is administered intravenously. In still other embodiments, the mitotic kinesin inhibitor is administered intrapulmonarily by inhalation or spraying of a dry powder, suspension, solution or aerosol comprising the mitotic kinesin inhibitor.

Pharmaceutically acceptable compositions include solid, semi-solid, liquid and aerosol dosage forms, such as, e.g., tablets, capsules, powders, liquids, suspensions, suppositories, aerosols or the like. The mitotic kinesin inhibitor can also be administered in sustained or controlled release dosage forms, including depot injections, osmotic pumps, pills, transdermal (including electrotransport) patches, and the like, for prolonged and/or timed, pulsed administration at a predetermined rate. In certain embodiments, the compositions are provided in unit dosage forms suitable for single administration of a precise dose.

The mitotic kinesin inhibitor can be administered either alone or in combination with a conventional pharmaceutical carrier, excipient or the like (e.g., mannitol, lactose, starch, magnesium stearate, sodium saccharine, talcum, cellulose, sodium crosscarmellose, glucose, gelatin, sucrose, magnesium carbonate, and the like). If desired, the pharmaceutical composition can also contain minor amounts of nontoxic auxiliary substances such as wetting agents, emulsifying agents, solubilizing agents, pH buffering agents and the like (e.g., sodium acetate, sodium citrate, cyclodextrine derivatives, sorbitan monolaurate, triethanolamine acetate, triethanolamine oleate, and the like). Generally, depending on the intended mode of administration, the pharmaceutical composition will contain about 0.005% to 95% or, in certain embodiments, about 0.5% to 50% by weight of a mitotic kinesin inhibitor. Actual methods of preparing such dosage forms are known, or will be apparent, to those skilled in this art; for example, see Remington's Pharmaceutical Sciences, Mack Publishing Company, Easton, Pennsylvania.

When the mitotic kinesin inhibitor is administered in combination with one or more other therapeutic agents or procedures, the doses of one or both agents will in some instances be lower than the corresponding dose for single-agent therapy. In general, the mitotic kinesin inhibitor and the other therapeutic agent(s) do not have to be administered in the same pharmaceutical composition, and may, because of different physical and chemical characteristics, be administered by different routes. For example, one agent can be administered orally, while the other is administered intravenously. Alternatively, each agent may be administered by the same route. The determination of the mode of administration and the advisability of administration, in the same pharmaceutical composition (if possible) is within the knowledge of the skilled clinician. The initial administration can be made according to established protocols known in the art, and then, based upon the observed effects, the dosage, modes of administration and times of administration can be modified by the skilled clinician.

In certain embodiments, the compositions will take the form of a pill or tablet and thus the composition may contain, along with the active ingredient, a diluent such as lactose, sucrose, dicalcium phosphate, or the like; a lubricant such as magnesium stearate or the like; and a binder such as starch, gum acacia, polyvinylpyrrolidine, gelatin, cellulose, cellulose derivatives or the like. In certain embodiments of a solid dosage form, a powder, marume, solution or suspension (e.g., in propylene carbonate, vegetable oils or triglycerides) is encapsulated in a gelatin capsule.

Liquid pharmaceutically administrable compositions can, for example, be prepared by dissolving, dispersing, etc. the mitotic kinesin inhibitor and optional pharmaceutical adjuvants in a carrier (e.g., water, saline, aqueous dextrose, glycerol, glycols, ethanol or the like) to form a solution or suspension. Injectables can be prepared in conventional forms, either as liquid solutions or suspensions, as emulsions, or in solid forms suitable for dissolution or suspension in liquid prior to injection. The percentage of the mitotic kinesin inhibitor contained in such parenteral compositions is dependent on the specific nature of the compound, as well as the activity of the mitotic kinesin inhibitor and the needs of the subject. In some embodiments, percentages of active ingredient of 0.01% to 10% in solution are employable, and may be higher if the composition is a solid which will be subsequently diluted.

Pharmaceutical compositions of the mitotic kinesin inhibitor may also be administered to the respiratory tract as an aerosol or solution for a nebulizer, or as a microfine powder for insufflation, alone or in combination with an inert carrier such as lactose. In some embodiments, the particles of the pharmaceutical composition have diameters of less than 50 microns, in certain embodiments, less than 10 microns.

The following examples serve to more fully describe the manner of using the above-described invention.

### Example 1: Human pulmonary arterial smooth muscle cell (HPASMC) proliferation assay

Primary cultures of human pulmonary arterial smooth muscle cells were derived from distal blood vessels taken from lungs harvested from patients with idiopathic pulmonary arterial hypertension (IPAH) who had been treated with iloprost or a combination of epoprostenol and bosentan for at least 1.4 years. Cells were cultured for 24 hours in 6-well plates in a solution of human smooth muscle basal medium (SMBM; TCS Cellworks, UK) with 9% fetal bovine serum (FBS; Invitrogen, US) and penicillin-streptomycin (PenStrep; 50 units/mL; Invitrogen, US). The incubating solution was then changed to PenStrep in SMBM and the cells were allowed to incubate for another 48 hours. Test compounds were administered to the test wells as DMSO solutions with FBS (FBS and DMSO or SMBM were administered to the control wells). After 96 hours of incubation, the cells were trypsinized (0.05% trypsin/EDTA; Invitrogen, US) and 20 uL of cell suspension was added to 20 uL of Accustain solution T and Accustain solution N (Labtech, UK). The cells were counted and their viability assessed using an ADAM Automated Cell Counter (Digital Bio, Korea). Results were expressed in cell number per mL and percent growth compared to FBS-induced growth alone.

### Example 2: Effect of iloprost in IPAH HPASMC proliferation assay

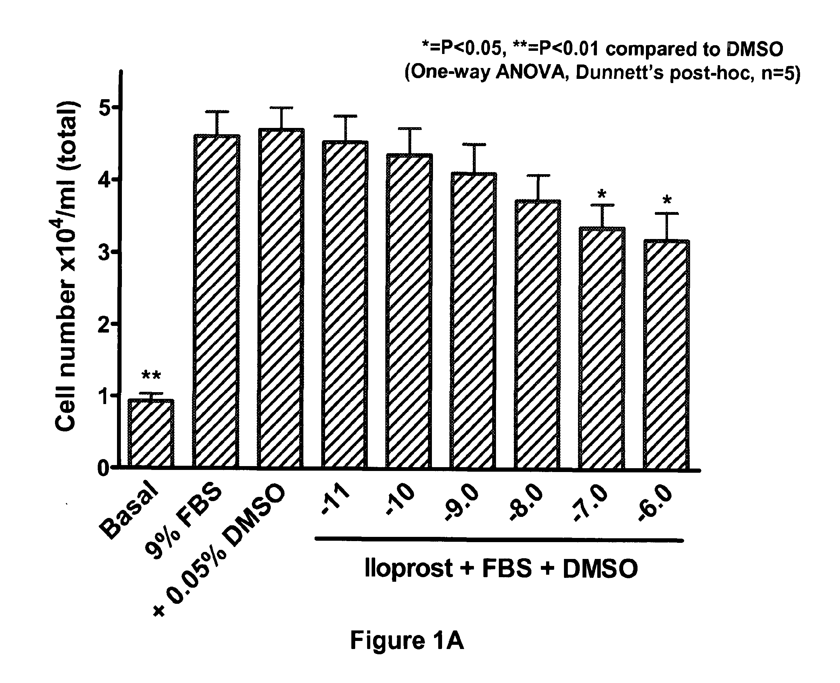

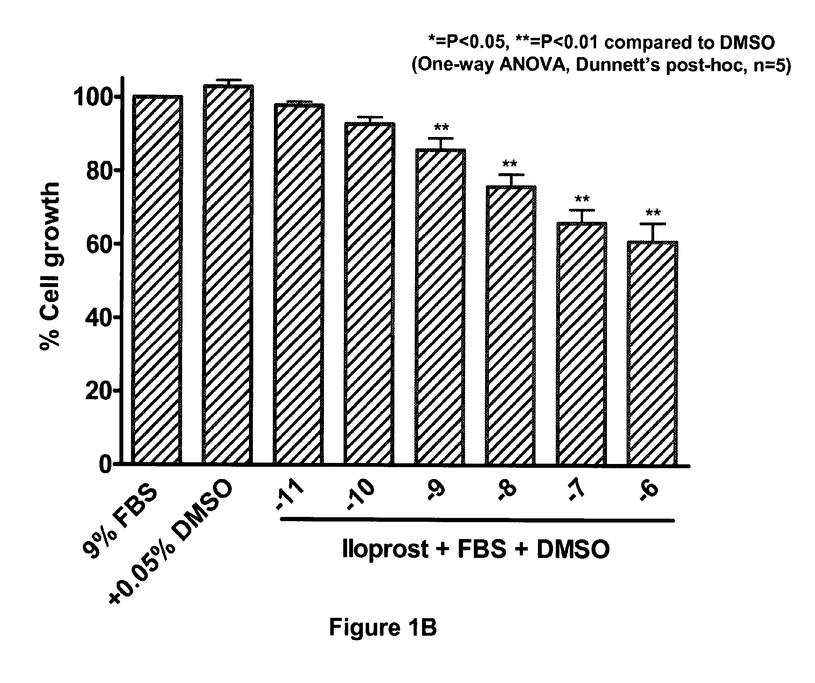

Iloprost is a synthetic prostacyclin analog used in the treatment of pulmonary arterial hypertension. Iloprost was tested under the conditions described in Example 1 at concentrations of 10 pM, 100 pM, 1 nM, 10 nM, 100 nM, and 1 uM. The experiment was repeated five times and the results were averaged. The results of these experiments, illustrated in Figures 1A and 1B, indicate that iloprost inhibited proliferation of IPAH HPASMC cells in a dose dependent manner, with approximately 40% inhibition at 1uM when normalized to FBS-induced growth.

### Example 3: Effect of treprostinil sodium in IPAH HPASMC proliferation assay

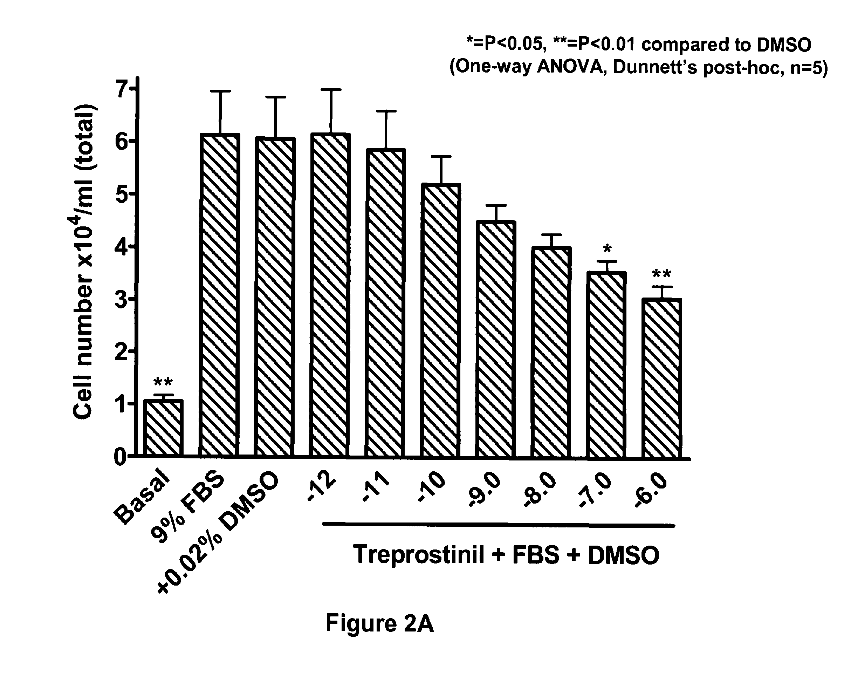

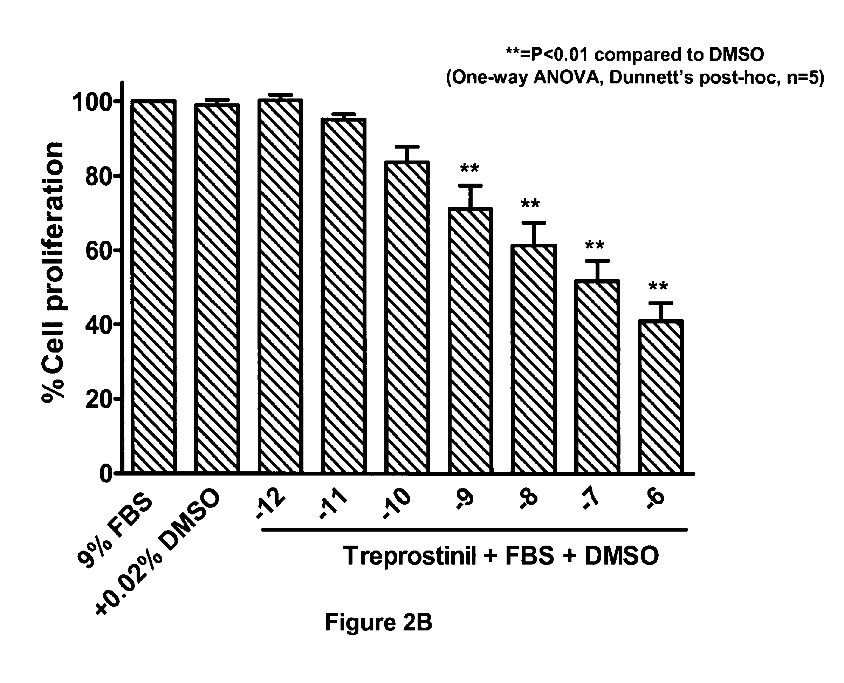

Treprostinil sodium is a synthetic prostacyclin analog used in the treatment of pulmonary arterial hypertension. Treprostinil sodium was tested under the conditions described in Example 1 at concentrations of 1 pM, 10 pM, 100 pM, 1 nM, 10 nM, 100 nM, and 1 uM. The results of these experiments, illustrated in Figures 2A and 2B, indicate that treprostinil sodium inhibited proliferation of IPAH HPASMC cells in a dose dependent manner, with approximately 60% inhibition at 1uM when normalized to FBS-induced growth.

### Example 4: Effect of bosentan in IPAH HPASMC proliferation assay

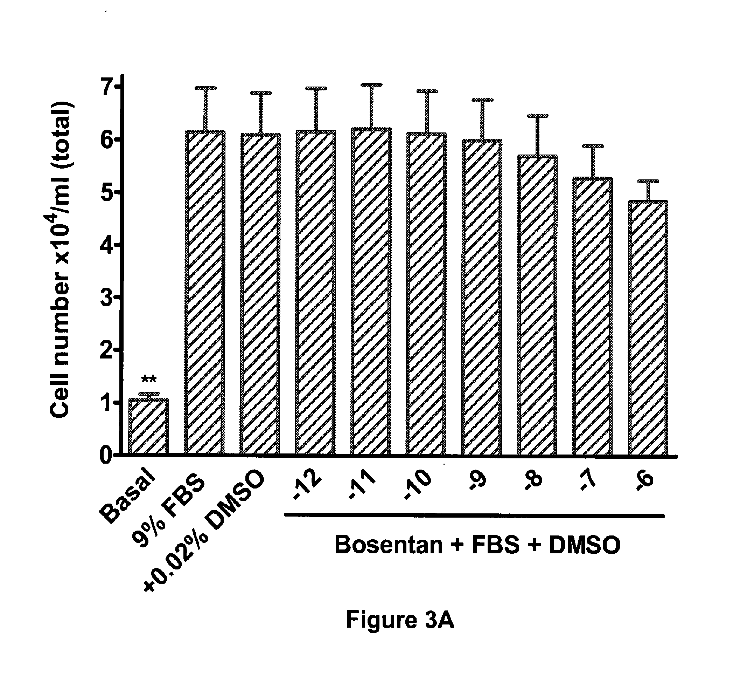

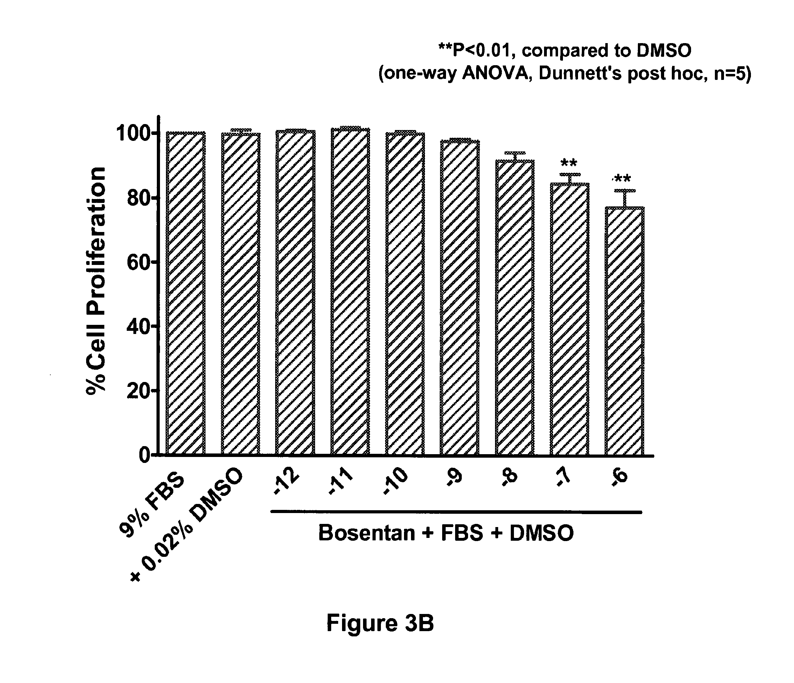

Bosentan is a dual endothelin receptor (ET_{A} and ET_{B}) antagonist used in the treatment of pulmonary arterial hypertension. Bosentan was tested under the conditions described in Example 1 at concentrations of 1 pM, 10 pM, 100 pM, 1 nM, 10 nM, 100 nM, and 1 uM. The experiment was repeated five times and the results were averaged. As shown in Figures 3A and 3B, bosentan inhibited proliferation of IPAH HPASMC cells at higher concentrations, with approximately 20% inhibition at 1uM when normalized to FBS-induced growth.

### Example 5: Effect of ambrisentan in IPAH HPASMC proliferation assay

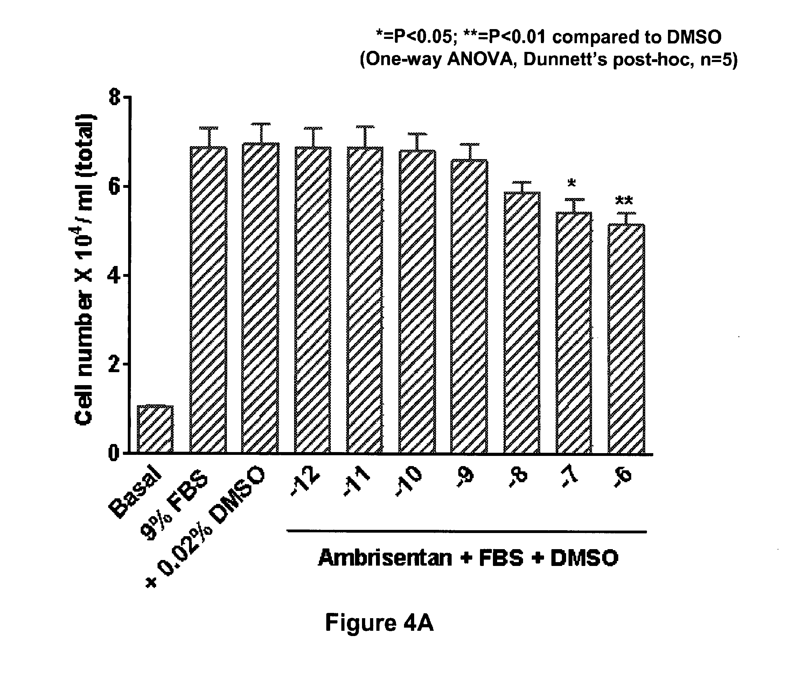

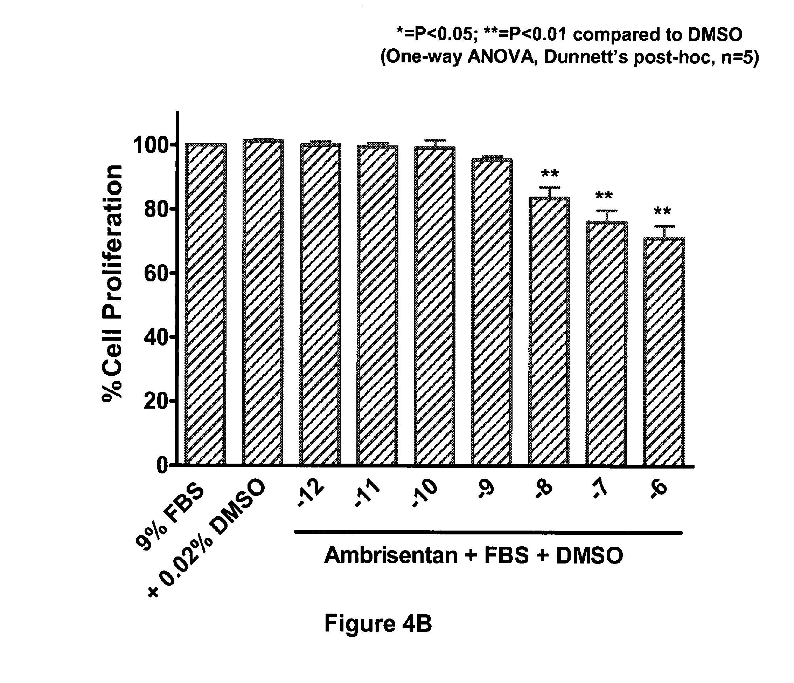

Ambrisentan is a type A endothelin receptor (ET_{A}) antagonist used in the treatment of pulmonary arterial hypertension. Ambrisentan was tested under the conditions described in Example 1 at concentrations of 1 pM, 10 pM, 100 pM, 1 nM, 10 nM, 100 nM, and 1 uM. The experiment was repeated five times and the results were averaged. The results of these experiments, summarized in Figures 4A and 4B, indicate that ambrisentan inhibited proliferation of IPAH cells at higher concentrations, with approximately 25% inhibition at 1uM when normalized to FBS-induced growth.

### Example 6: Effect of BQ788 in IPAH HPASMC proliferation assay

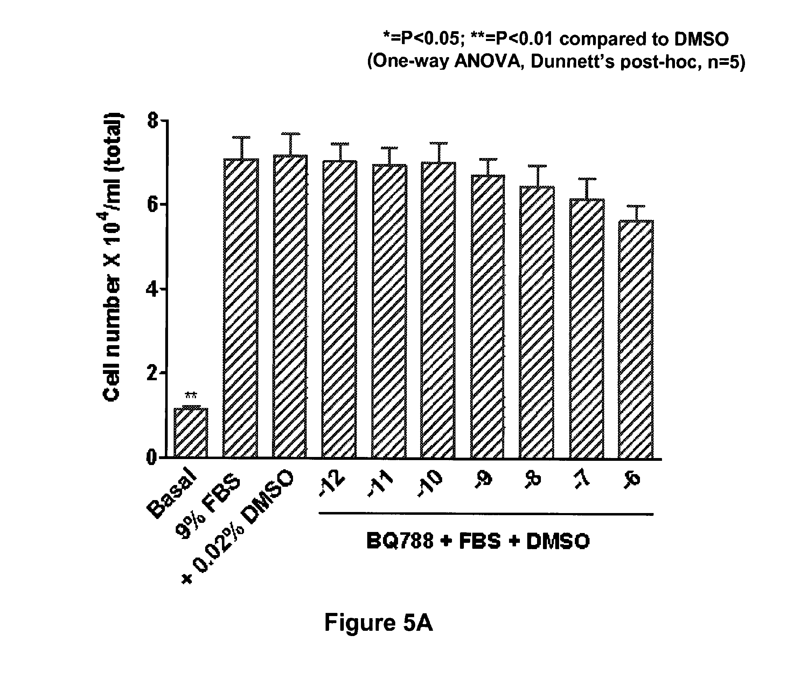

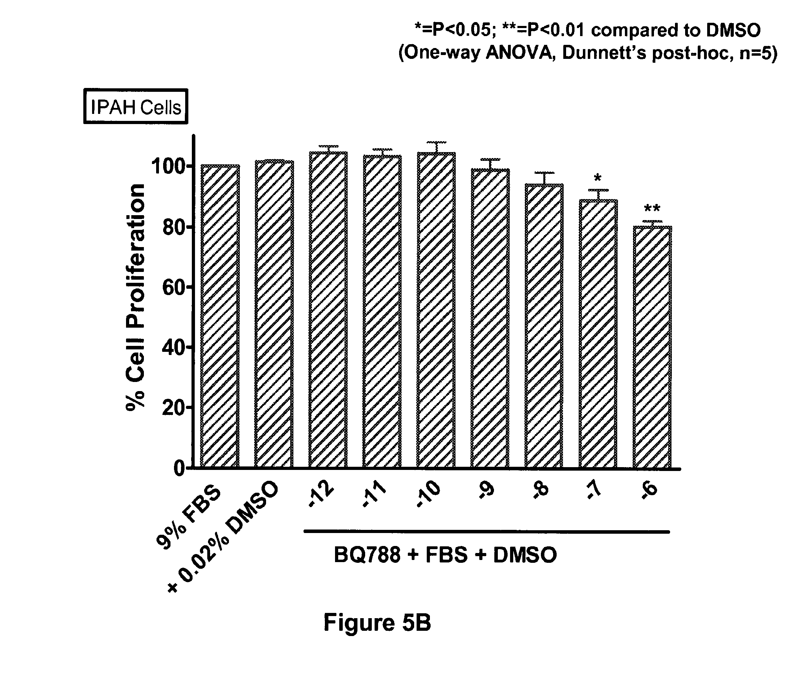

BQ788 is an type B endothelin receptor (ET_{B}) antagonist. BQ788 was tested under the conditions described in Example 1 at concentrations of 1 pM, 10 pM, 100 pM, 1 nM, 10 nM, 100 nM, and 1 uM. The experiment was repeated five times and the results were averaged. The results of these experiments, summarized in Figures 5A and 5B, indicate that BQ788 inhibited proliferation of IPAH cells at higher concentrations, with approximately 20% inhibition at 1uM when normalized to FBS-induced growth.

### Example 7: Effect of CENP-E inhibitor in IPAH HPASMC proliferation assay

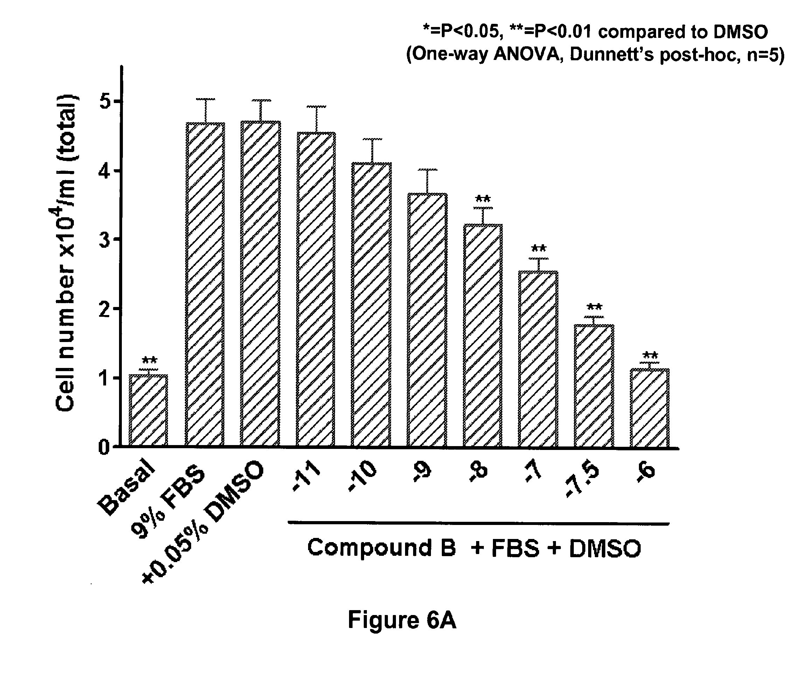

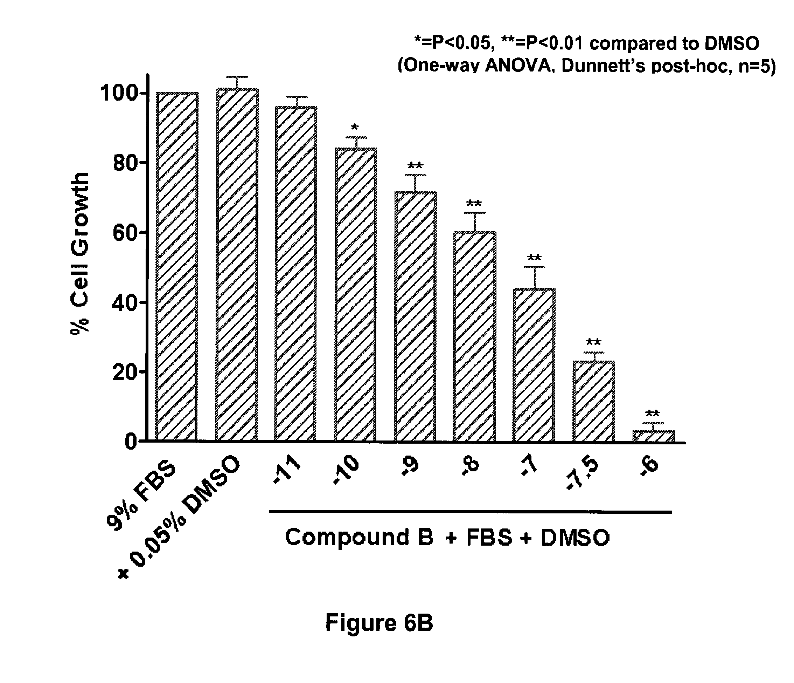

3-Chloro-*N*-{(1S)-2-[(*N*,*N*-dimethylglycyl)amino]-1-[(4-{8-[(1S)-1-hydroxyethyl] imidazo[1,2-*a*]pyridin-2-yl}phenyl)methyl]ethyl}-4-[(1-methylethyl)oxy]benzamide ("Compound B") is a CENP-E inhibitor (see, e.g., U.S. Patent Nos. 7,504,413 and 7,618,981). Compound B was tested under the conditions described in Example 1 at concentrations of 10 pM, 100 pM, 1 nM, 10 nM, 100 nM, and 1 uM. The experiment was repeated five times and the results were averaged. The results of these experiments, illustrated in Figures 6A and 6B, indicate that Compound B inhibited proliferation of IPAH cells in a dose dependent manner, with approximately 95% inhibition at 1uM when normalized to FBS-induced growth.

### Example 8: Effect of KSP inhibitor in IPAH HPASMC proliferation assay

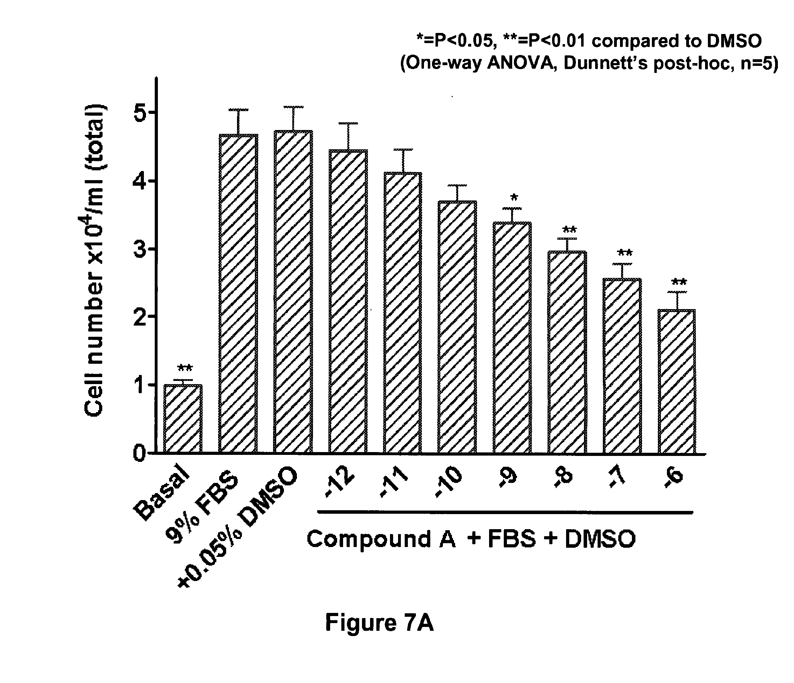

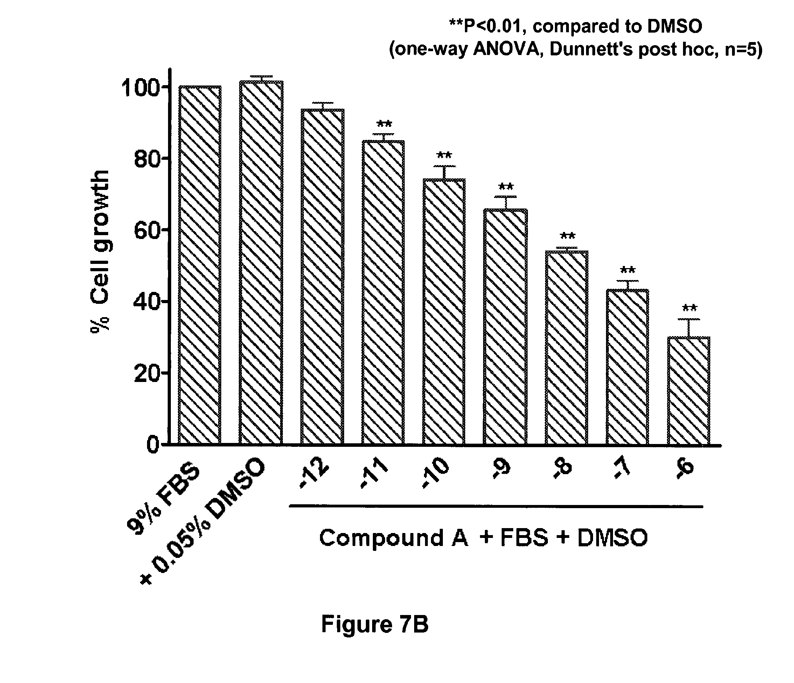

*N*-(3-Aminopropyl)-*N*-[(*R*)-(1-3-benzyl-7-chloro-4-oxo-4H-chromen-2-yl)-2-methylpropyl]-4-methyl-benzamide ("Compound A") is a KSP inhibitor (see, e.g., U.S. Patent Nos. 6,924,376 and 7,629,477). Compound A was tested under the conditions described in Example 1 at concentrations of 1 pM, 10 pM, 100 pM, 1 nM, 10 nM, 100 nM, and 1 uM. The experiment was repeated five times and the results were averaged. The results of these experiments, illustrated in Figures 7A and 7B, indicate that Compound A inhibited proliferation of IPAH cells in a dose dependent manner, with approximately 70% inhibition at 1uM when normalized to FBS-induced growth.

### Example 9: Effect of ispinesib mesylate in IPAH HPASMC proliferation assay

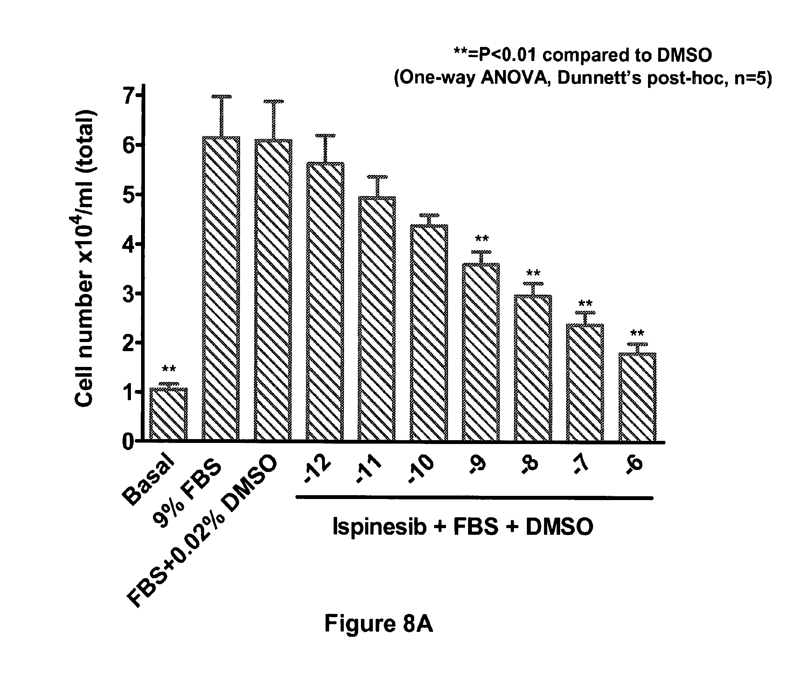

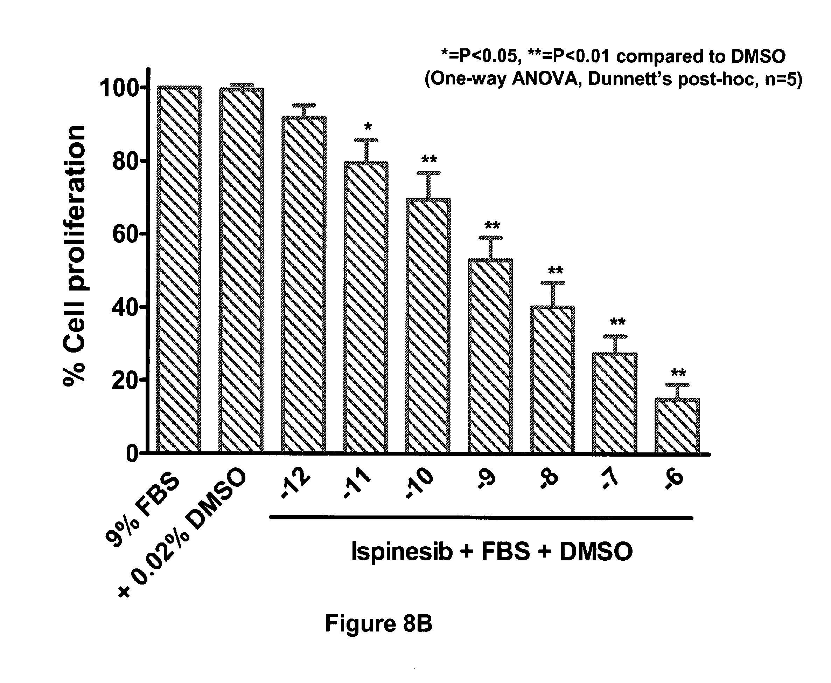

*N*-(3-aminopropyl)-*N*-[(1*R*)-1-(3-benzyl-7-chloro-4-oxo-3,4-dihydroquinazolin-2-yl)-2-methylpropyl]-4-methylbenzamide monomethanesulfonate (ispinesib mesylate) is a KSP inhibitor (see, e.g., U.S. Patent Nos. 6,545,004, 7,671,200, and 7,763,628). Ispinesib mesylate was tested under the conditions described in Example 1 at concentrations of 1 pM, 10 pM, 100 pM, 1 nM, 10 nM, 100 nM, and 1 uM. The experiment was repeated five times and the results were averaged. The results of these experiments, illustrated in Figures 8A and 8B, indicate that ispinesib mesylate inhibited proliferation of IPAH cells in a dose dependent manner, with approximately 80% inhibition at 1uM when normalized to FBS-induced growth.

### Example 10: Effect of combination of ispinesib mesylate and treprostinil sodium in IPAH HPASMC proliferation assay

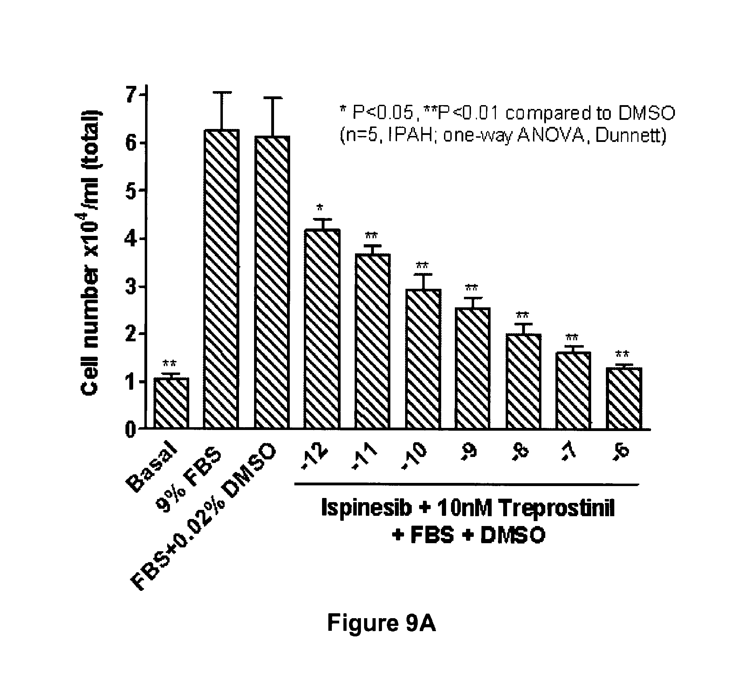

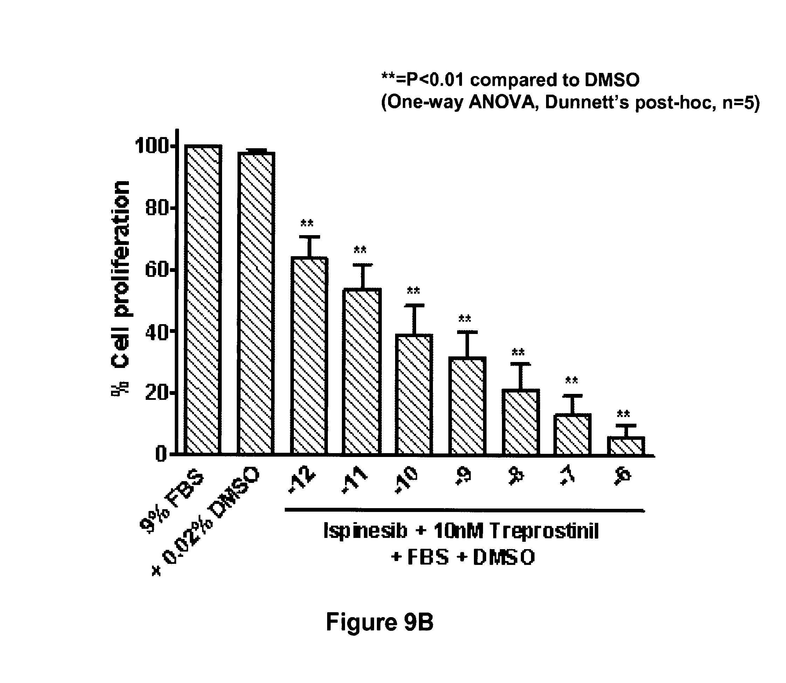

Combinations of treprostinil sodium (10 nM) and ispinesib mesylate at concentrations of 1 pM, 10 pM, 100 pM, 1 nM, 10 nM, 100 nM, and 1 uM were tested under the conditions described in Example 1. The experiment was repeated five times and the results were averaged. The results of these experiments, illustrated in Figures 9A and 9B, indicate that combined treatment with ispinesib mesylate and treprostinil sodium enhanced the antiproliferative effects of either agent alone, with approximately 90% inhibition at 1uM ispinesib mesylate and 10nM treprostinil sodium when normalized to FBS-induced growth.

### Example 11: Effect of combination of ispinesib mesylate and bosentan in IPAH HPASMC proliferation assay

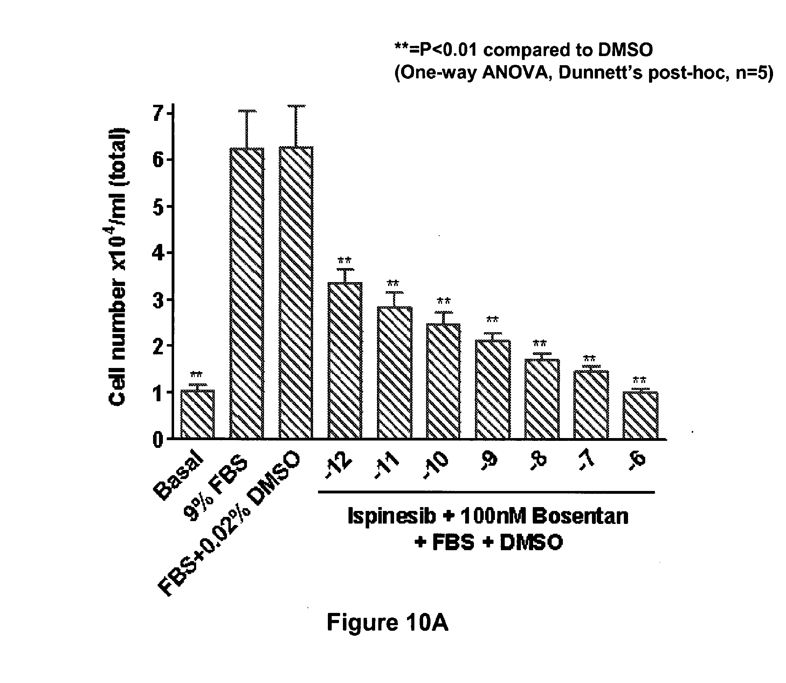

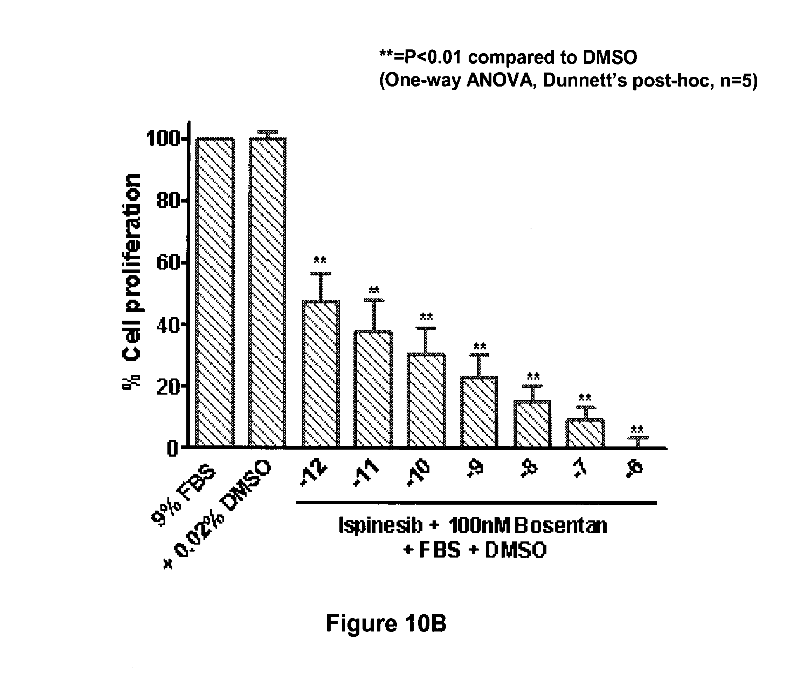

Combinations of bosentan (100 nM) and ispinesib mesylate at concentrations of 1 pM, 10 pM, 100 pM, 1 nM, 10 nM, 100 nM, and 1 uM were tested under the conditions described in Example 1. The experiment was repeated five times and the results were averaged. The results of these experiments, illustrated in Figures 10A and 10B, indicate that combined treatment with ispinesib mesylate and bosentan enhanced the antiproliferative effects of either agent alone, with approximately 99% inhibition at 1uM ispinesib mesylate and 100nM bosentan when normalized to FBS-induced growth.

### Example 12: Effect of combination of ispinesib mesylate and ambrisentan in IPAH HPASMC proliferation assay

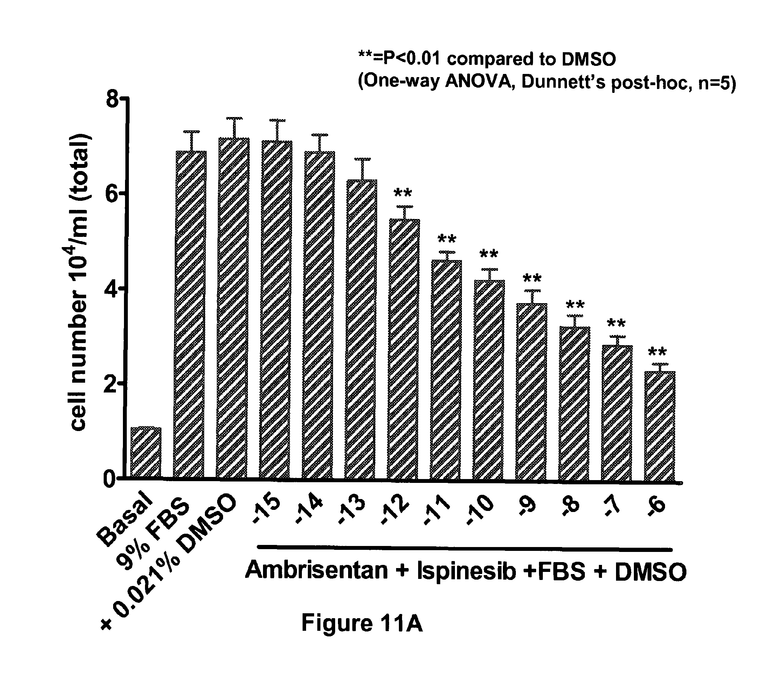

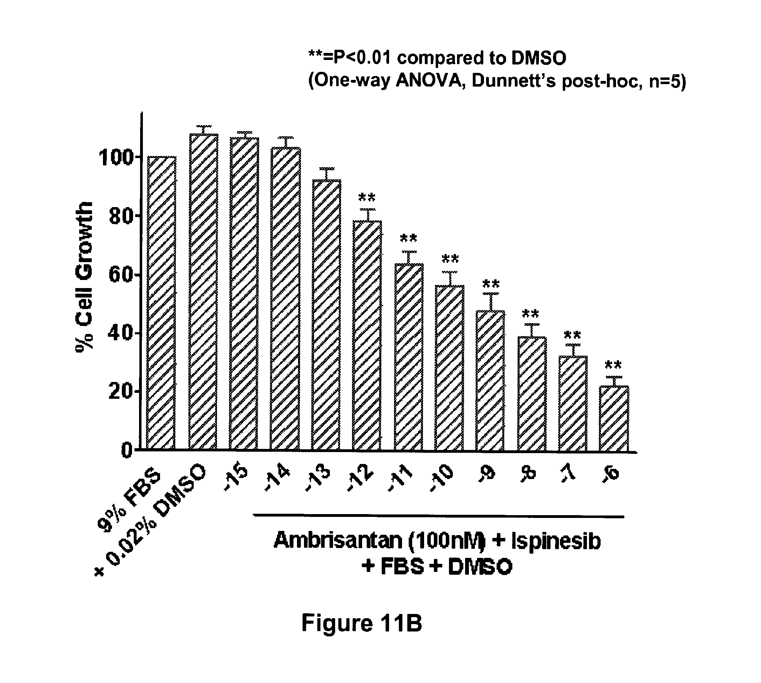

Combinations of ambrisentan (100 nM) and ispinesib mesylate at concentrations of 0.001 pM, 0.01 pM, 0.1 pM, 1 pM, 10 pM, 100 pM, 1 nM, 10 nM, 100 nM, and 1 uM were tested under the conditions described in Example 1. The experiment was repeated five times and the results were averaged. The results of these experiments, illustrated in Figures 11A and 11B, indicate that combined treatment with ispinesib mesylate and ambrisentan inhibited IPAH HPASMC proliferation by approximately 80% (1uM ispinesib mesylate and 100nM ambrisentan) when normalized to FBS-induced growth.

### Example 13: Effect of combination of ispinesib mesylate and BQ788 in IPAH HPASMC proliferation assay

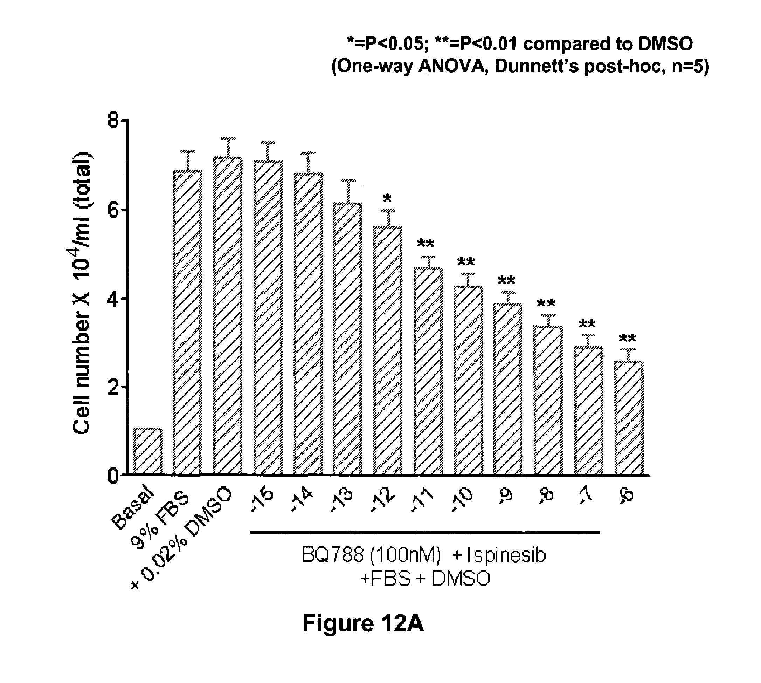

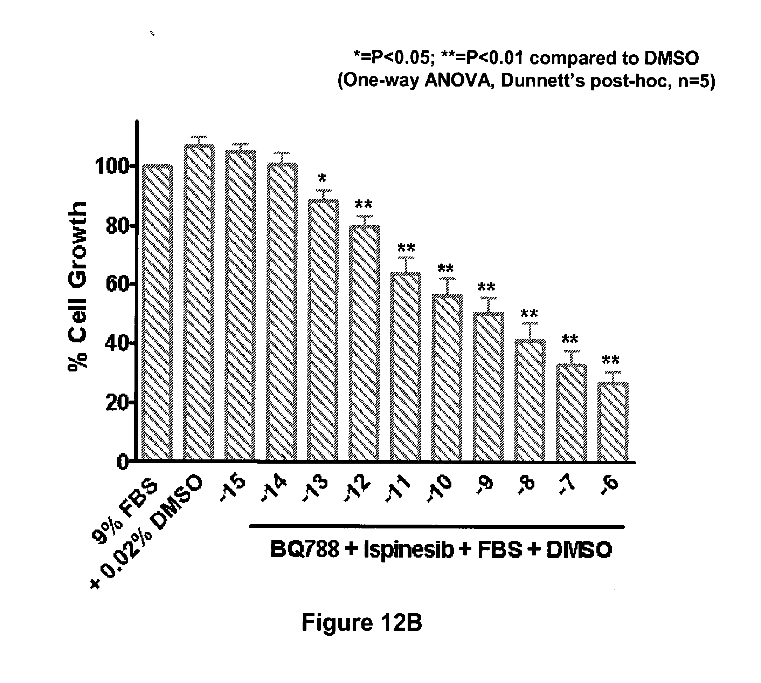

Combinations of BQ788 (100 nM) and ispinesib mesylate at concentrations of 0.001 pM, 0.01 pM, 0,1 pM, 1 pM, 10 pM, 100 pM, 1 nM, 10 nM, 100 nM, and 1 uM were tested under the conditions described in Example 1. The experiment was repeated five times and the results were averaged. The results of these experiments, illustrated in Figures 12A and 12B, indicate that combined treatment with ispinesib mesylate and BQ788 inhibited IPAH HPASMC proliferation by approximately 75% (1uM ispinesib mesylate and 100nM BQ788) when normalized to FBS-induced growth.

### Example 14: Effect of combination of ispinesib mesylate, ambrisentan and BQ788 in IPAH HPASMC proliferation assay

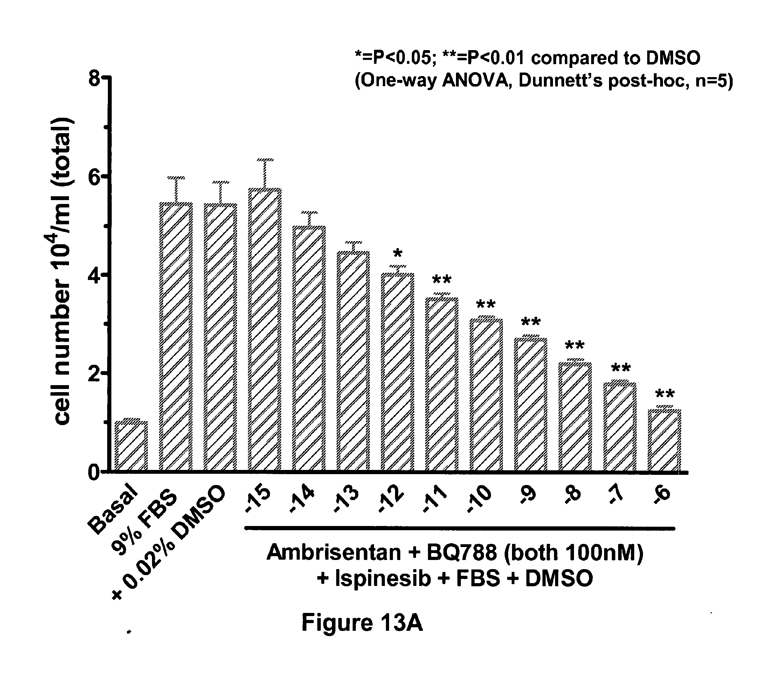

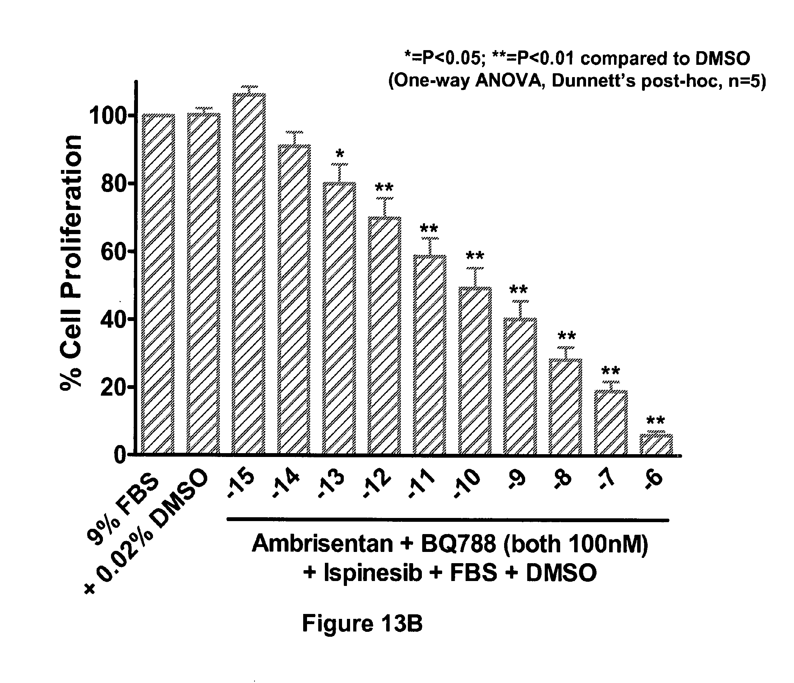

Combinations of ambrisentan (100 nM), BQ788 (100 nM) and ispinesib mesylate at concentrations of 0.001 pM, 0.01 pM, 0,1 pM, 1 pM, 10 pM, 100 pM, 1 nM, 10 nM, 100 nM, and 1 uM were tested under the conditions described in Example 1. The experiment was repeated five times and the results were averaged. The results of these experiments, illustrated in Figures 13A and 13B, indicate that combined treatment with ispinesib mesylate, ambrisentan and BQ788 inhibited IPAH HPASMC proliferation by approximately 95% (1uM ispinesib mesylate, 100 nM ambrisentan and 100nM BQ788) when normalized to FBS-induced growth.

### Example 15: Effects of treprostinil sodium in fibroblast proliferation assay

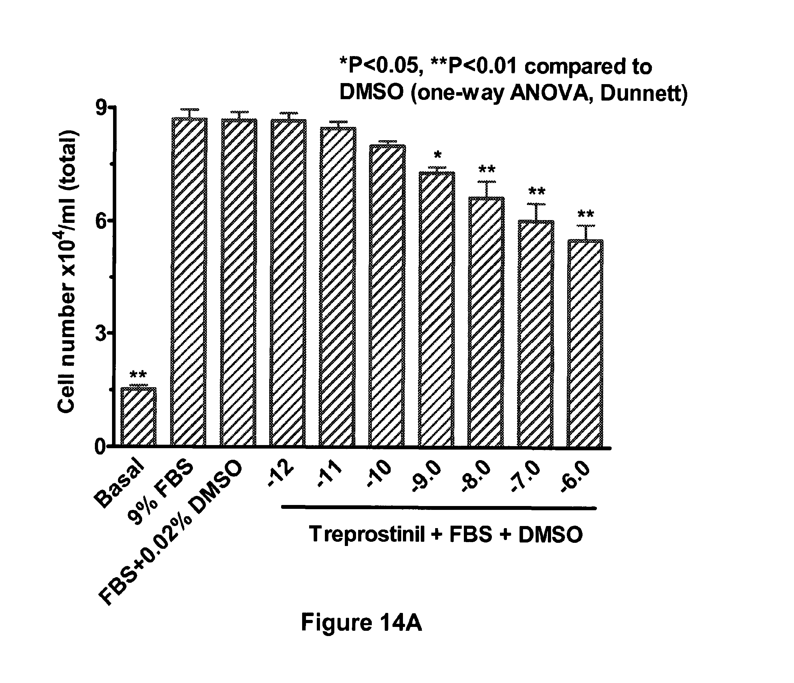

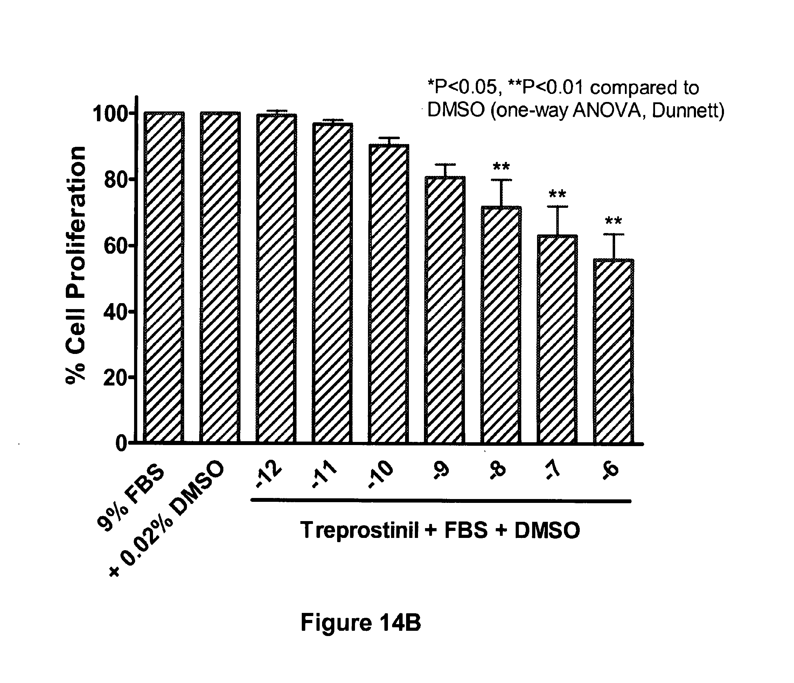

Using the procedure described in Example 1, primary cultures of human lung fibroblasts were derived from lungs harvested from patients with ideopathic pulmonary fibrosis (IPF) with secondary pulmonary arterial hypertension (PAH) who had previously been treated with epoprostenol, bosentan, and/or steriods. Treprostinil sodium was tested in these cells under the conditions described in Example 1 at concentrations of 1 pM, 10 pM, 100 pM, 1 nM, 10 nM, 100 nM, and 1 uM. The experiment was repeated four times and the results were averaged. The results of these experiments, illustrated in Figures 14A and 14B, indicate that treprostinil sodium inhibited proliferation of fibroblasts at higher concentrations, with approximately 45% inhibition at 1uM when normalized to FBS-induced growth.

### Example 16: Effects of ispinesib mesylate in fibroblast proliferation assay

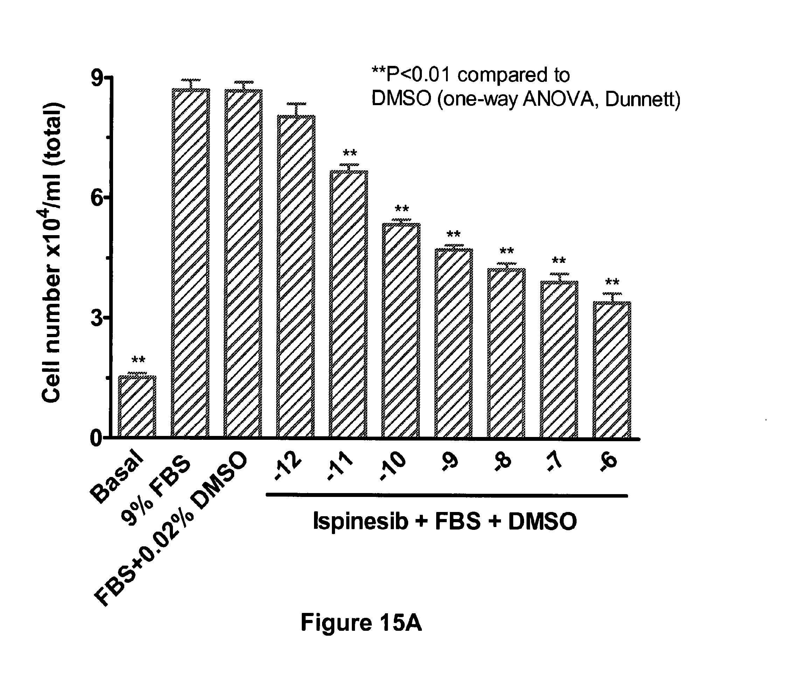

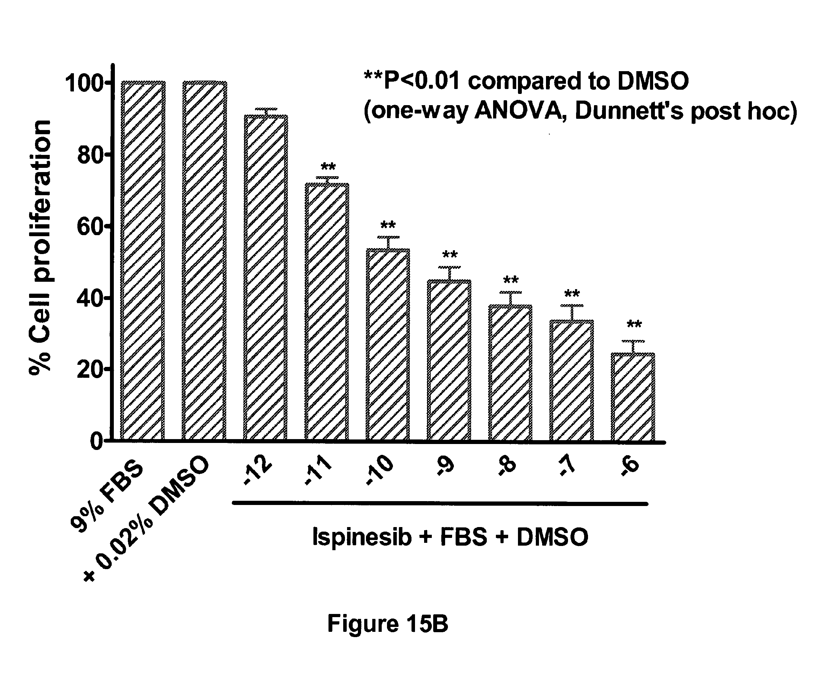

Ispinesib mesylate was tested under the conditions described in Example 15 at concentrations of 1 pM, 10 pM, 100 pM, 1 nM, 10 nM, 100 nM, and 1 uM. The experiment was repeated four times and the results were averaged. The results of these experiments, illustrated in Figures 15A and 15B, indicate that ispinesib mesylate inhibited proliferation of fibroblasts in a dose dependent manner, with approximately 75% inhibition at 1uM when normalized to FBS-induced growth. When comparing these results against those in Exampe 15, it can been seen that ispinesib mesylate is significantly more potent than treprostinil sodium at inhibiting the proliferation of lung fibroblasts derived from IPF patients with secondary PAH.

### Example 17: Effects of treprostinil sodium in normal HPASMC proliferation assay

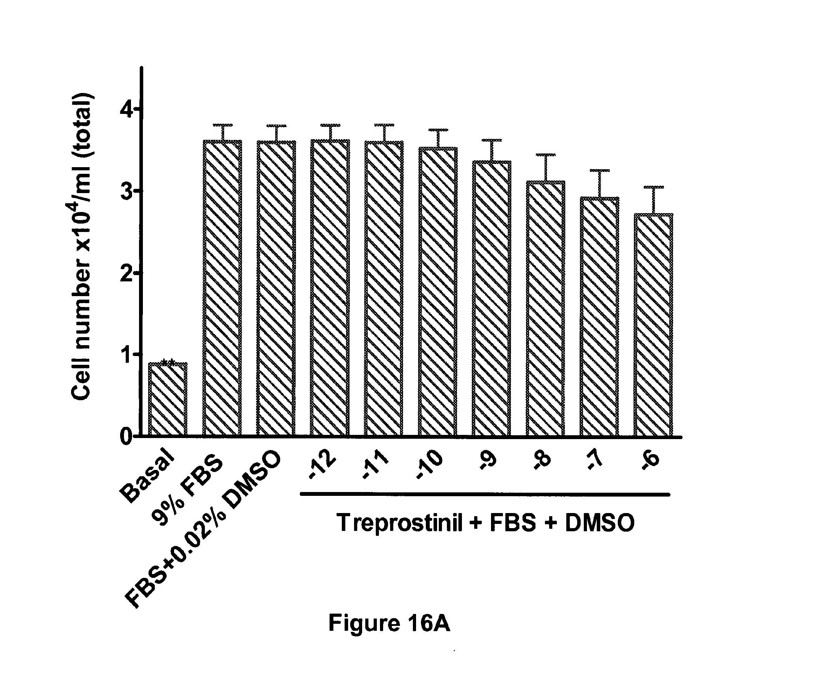

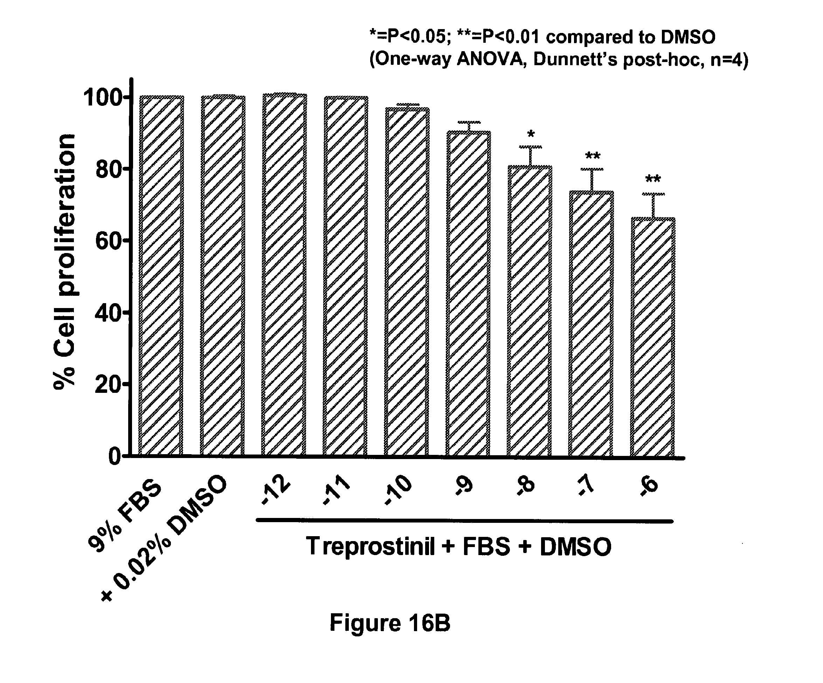

As a control, primary cultures of human pulmonary arterial smooth muscle cells were derived from distal blood vessels taken from lungs harvested from healthy patients according to procedure described in Example 1. Treprostinil sodium was tested in these cells under the conditions described in Example 1 at concentrations of 1 pM, 10 pM, 100 pM, 1 nM, 10 nM, 100 nM, and 1 uM. The experiment was repeated four times and the results were averaged. The results of these experiments, illustrated in Figures 16A and 16B, indicate that treprostinil sodium inhibited normal HPASMC proliferation at higher concentrations, with approximately 35% inhibition at 1uM when normalized to FBS-induced growth.

### Example 18: Effects of ispinesib mesylate in normal HPASMC proliferation assay

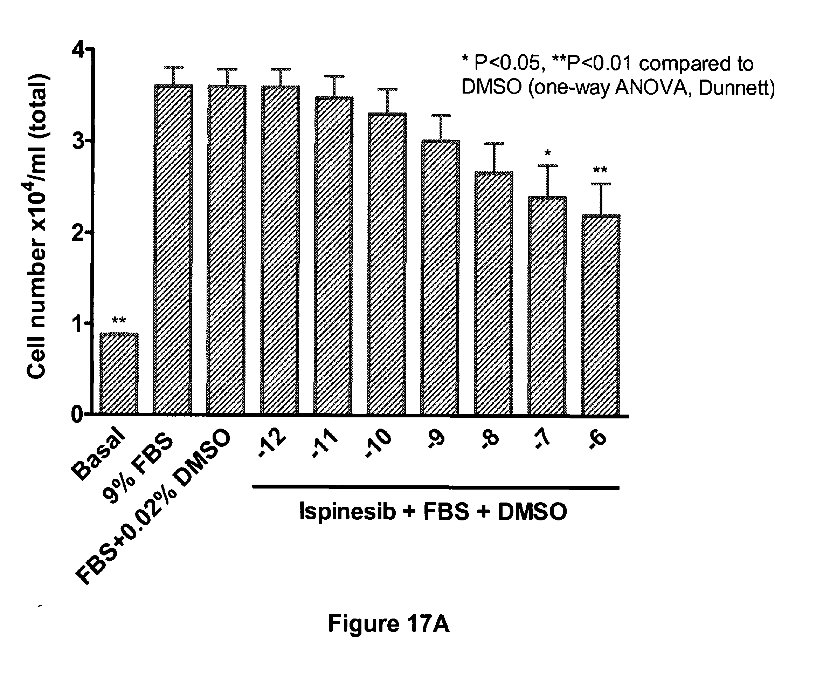

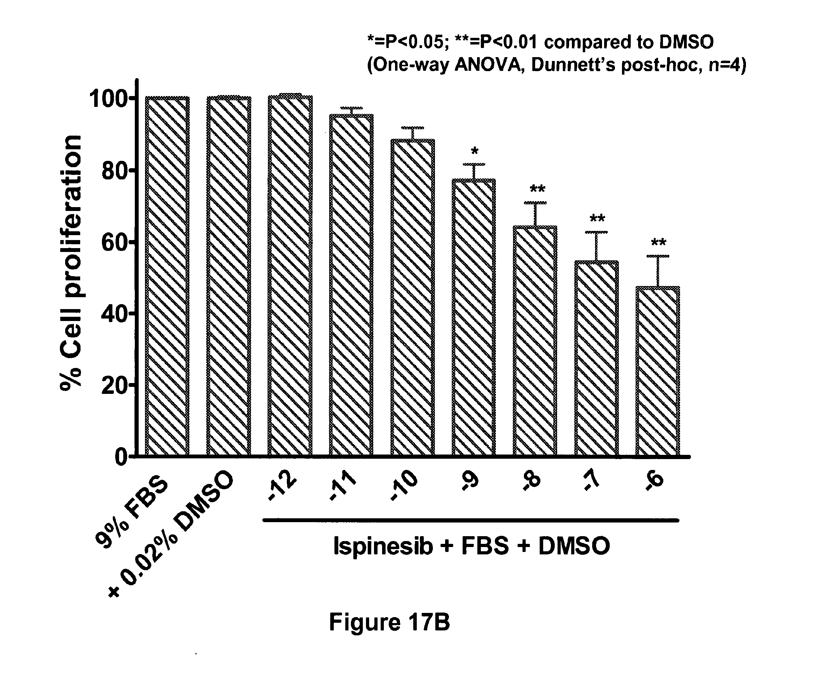

Ispinesib mesylate was tested under the conditions described in Example 17 at concentrations of 1 pM, 10 pM, 100 pM, 1 nM, 10 nM, 100 nM, and 1 uM. The experiment was repeated four times and the results were averaged. The results of these experiments, illustrated in Figures 17A and 17B, indicate that ispinesib mesylate inhibited proliferation of fibroblasts in a dose dependent manner, with approximately 55% inhibition at 1uM when normalized to FBS-induced growth. When comparing these results against those in Exampe 17, it can been seen that ispinesib mesylate is slightly more potent than treprostinil sodium at inhibiting the proliferation of normal HPASMCs.

When comparing these results against those in Examples 9 and 16, it can be seen that ispinesib mesylate is significantly more potent at inhibiting proliferation of IP AH HPASMCs and fibroblasts than normal HPASMCs.

### Example 19: Chronic hypoxia-induced pulmonary arterial hypertension animal model

Male Sprague-Dawley rats (175 - 250 g) are placed in the hypoxia chamber at FIO₂ of 10% for two weeks, during which time some rats receive test compounds once every four days i.p. At the end of the two weeks, animals are anesthetized with a ketamine cocktail and the right ventricular systolic pressure, systemic blood pressure (systolic, diastolic and mean), and heart rate are determined via right jugular vein catheterization. Body weight is measured prior to placing rats in the hypoxia chamber and once weekly at the time of cage changing. Animals that survive to the scheduled necropsy are weighed prior to euthanasia. Thereafter, the animals are euthanized and tissues are collected for histology. The heart is removed and dissected into appropriate sections for the determination of the right ventricular hypertrophy index (right ventricular free wall weight/left ventricular free wall + septum weight). The lung is processed and paraffin embedded for microtomy to obtain 5 µm transverse and frontal sections. Serially sectioned slides are stained with (i) hematoxylin and eosin, and (ii) Verhoeff's Elastic stain plus Van Gieson. Cellular proliferation is identified using an antibody directed toward the nuclear protein, Ki67. Medial wall thickness is determined in slides immunostained for alpha-smooth muscle actin. The cellular makeup of vascular lesions is characterized using antibodies directed toward alpha-smooth muscle actin or Von Willebrand factor to identify smooth muscle and endothelial cells, respectively. Histological characterization is performed by a veterinary pathologist.

## Claims

1. A mitotic kinesin inhibitor for use in a method of treating pulmonary arterial hypertension in a subject, comprising administering to the subject a therapeutically effective amount of the mitotic kinesin inhibitor;
wherein the mitotic kinesin inhibitor is an inhibitor of kinesin spindle protein (KSP) or wherein the mitotic kinesin inhibitor is an inhibitor of centromere-associated protein E (CENP-E).

2. The mitotic kinesin inhibitor of claim 1, wherein the pulmonary arterial hypertension is idiopathic pulmonary arterial hypertension.

3. The mitotic kinesin inhibitor of claim 1, wherein the pulmonary arterial hypertension is associated pulmonary arterial hypertension.

4. The mitotic kinesin inhibitor of claim 1, wherein the pulmonary arterial hypertension is familial pulmonary arterial hypertension.

5. The mitotic kinesin inhibitor of any one of claims 1-4, wherein the mitotic kinesin inhibitor is an inhibitor of kinesin spindle protein, which is selected from ispinesib mesylate; *N*-(3-aminopropyl)-*N*-[(*R*)-(1-3-benzyl-7-chloro-4-oxo-4H-chromen-2-yl)-2-methyl-propyl]-4-methyl-benzamide hydrochloride hydrate; (S)-2-(3-aminopropyl)-5-(2,5-difluorophenyl)-*N*-methoxy-*N*-methyl-2-phenyl-1,3,4-thiadiazole-3(2H)-carboxamide, or a pharmaceutically acceptable salt thereof; 3-(5-(2,5-difluorophenyl)-3-(5-methyl-1,3,4-thiadiazol-2-yl)-2-phenyl-2,3-dihydro-1,3,4-thiadiazol-2-yl)propan-1-amine, or a pharmaceutically acceptable salt thereof; ARRY-520, or a pharmaceutically acceptable salt thereof; (2S)-4-(2,5-difluorophenyl)-N-[(3R,4S)-3-fluoro-1-methylpiperidin-4-yl]-2-(hydroxymethyl)-N-methyl-2-phenyl-2,5-dihydro-1H-pyrrole-1-carboxamide, or a pharmaceutically acceptable salt thereof; MK-0731, or a pharmaceutically acceptable salt thereof; 1-[2-(dimethylamino)ethyl]-3-{[(2R,4aS,5R,10bS)-5-phenyl-9-(trifluoromethyl)-3,4,4a,5,6,10b-hexahydro-2H-pyrano[3,2-c]quinolin-2-yl]methyl}urea, or a pharmaceutically acceptable salt thereof; EMD 534085, or a pharmaceutically acceptable salt thereof; (R)-N-(3-aminopropyl)-N-[1-(5-benzyl-3-methyl-4-oxo-4,5-dihydroisothiazolo[5,4-d]pyrimidin-6-yl)-2-methylpropyl]-4-methylbenzamide, or a pharmaceutically acceptable salt thereof; AZD4877, or a pharmaceutically acceptable salt thereof; litronesib, or a pharmaceutically acceptable salt thereof; N-(3-amino-propyl)-3-chloro-N-[(R)-1-(7-chloro-4-oxo-3-phenylamino-3,4-dihydro-quinazolin-2-yl)-but-3-ynyl]-2-fluoro-benzamide, or a pharmaceutically acceptable salt thereof; ARQ 621, or a pharmaceutically acceptable salt thereof; 4SC-205, or a pharmaceutically acceptable salt thereof; CRx-026, or a pharmaceutically acceptable salt thereof; and SCH 2047069, or a pharmaceutically acceptable salt thereof

6. The mitotic kinesin inhibitor of claim 5, wherein the inhibitor of kinesin spindle protein is ispinesib mesylate.

7. The mitotic kinesin inhibitor of any one of claims 1-4, wherein the mitotic kinesin inhibitor is an CENP-E inhibitor, which is selected from N-(1-{4-[2-(1-acetylamino-ethyl)-1-ethyl-1H-imidazol-4-yl]-benzyl}-3-hydroxy-propyl)-3-chloro-4-(2,2,2-trifluoro-1-methyl-ethoxy)-benzamide, N-(2-(2-dimethylamino-acetylamino)-1-{4-[8-(1-hydroxy-ethyl)-imidazo[1,2-a]pyridin-2-yl]-benzyl}-ethyl)-3-chloro-4-isopropoxy-benzamide, N-(1-{4-[2-(1-methyl-1-hydroxy-ethyl)-1-ethyl-1H-imidazol-4-yl]-benzyl}-3-hydroxy-propyl)-3-chloro-4-(2,2,2-trifluoro-1-methyl-ethoxy)-benzamide, N-(2-(2-dimethylamino-acetylamino)-1-{4-[8-methyl-imidazo[1,2-a]pyridin-2-yl]-benzyl}-ethyl)-3-chloro-4-isopropoxy-benzamide, N-(1-{4-[2-(1-hydroxy-1-methyl-ethyl)-1-methyl-1H-imidazol-4-yl]-benzyl} -3-hydroxy-propyl)-3-chloro-4-(2,2,2-trifluoro-1-methyl-ethoxy)-benzamide, N-(2-(2-amino-2-methyl-propionylamino)-1-{4-[8-methyl-imidazo[1,2-a]pyridin-2-yl]-benzyl}-ethyl)-3-chloro-4-isopropoxy-benzamide, and N-{1-[4-(8-(1-hydroxy-ethyl)-imidazo[1,2-a]pyridin-2-yl)-benzyl]-3-hydroxy-propyl}-3-chloro-4-isopropoxy-benzamide, or a pharmaceutically acceptable salt thereof.

8. The mitotic kinesin inhibitor of claim 7, wherein the CENP-E inhibitor is N-(2-(2-dimethylamino-acetylamino)-1-{4-[8-(1-hydroxy-ethyl)-imidazo[1,2-a]pyridin-2-yl]-benzyl}-ethyl)-3-chloro-4-isopropoxy-benzamide, or a pharmaceutically acceptable salt thereof.

9. The mitotic kinesin inhibitor of any one of claims 1-8, wherein the mitotic kinesin inhibitor is administered orally, intravenously, subcutaneously, intranasally, transdermally, intraperitoneally, intramuscularly, or intrapulmonarily.

10. The mitotic kinesin inhibitor of any one of claims 1-9, further comprising administering to the subject a second therapy.

11. The mitotic kinesin inhibitor of claim 10, wherein the second therapy is selected from prostanoids, endothelin receptor antagonists, phosphodiesterase inhibitors, prostacyclin receptor agonists, anticoagulants, diuretics, calcium channel blockers, digoxin, oxygen therapy, nitric oxide therapy, tyrosine kinases, statins, 5-HT receptor antagonists, phosphatidylinositol 3-kinase inhibitors, soluble guanylate cyclase activators, adrenomedullin, platelet-derived growth factor inhibitors, Rho-kinase inhibitors, lung transplantation, heart transplantation, and atrial septosomy.

12. The mitotic kinesin inhibitor of claim 11, wherein the second therapy is an endothelin receptor antagonist.

13. The mitotic kinesin inhibitor of claim 12, wherein the endothelin receptor antagonist is a type A endothelin receptor antagonist.

14. The mitotic kinesin inhibitor of claim 12, wherein the endothelin receptor antagonist is a dual type A/type B endothelin receptor antagonist.

15. The mitotic kinesin inhibitor of claim 12, wherein the endothelin receptor antagonist is bosentan.

16. The mitotic kinesin inhibitor of claim 12, wherein the endothelin receptor antagonist is ambresentan.

17. The mitotic kinesin inhibitor of claim 10, wherein the second therapy is a prostanoid.

18. The mitotic kinesin inhibitor of claim 17, wherein the prostanoid is treprostinil sodium.

19. The mitotic kinesin inhibitor of claim 17, wherein the prostanoid is iloprost.

20. The mitotic kinesin inhibitor of any one of claims 10-19, wherein the mitotic kinesin inhibitor and the second therapy are administered simultaneously to the subject.

21. The mitotic kinesin inhibitor of any one of claims 10-19, wherein the mitotic kinesin inhibitor and the second therapy are administered sequentially to the subject.

## Patentansprüche

1. Inhibitor von mitotischem Kinesin zur Verwendung in einem Verfahren zur Behandlung von pulmonalarterieller Hypertonie in einem Individuum, wobei dem Individuum eine therapeutisch wirksame Menge des Inhibitors von mitotischem Kinesin verabreicht wird; wobei es sich bei dem Inhibitor von mitotischem Kinesin um einen Inhibitor des Kinesin-Spindel-Proteins (KSP) handelt oder wobei es sich bei dem Inhibitor von mitotischem Kinesin um einen Inhibitor des mit dem Centromer assoziierten Proteins E (CENP-E) handelt.

2. Inhibitor von mitotischem Kinesin nach Anspruch 1, wobei es sich bei der pulmonal-arteriellen Hypertonie um idiopathische pulmonal-arterielle Hypertonie handelt.

3. Inhibitor von mitotischem Kinesin nach Anspruch 1, wobei es sich bei der pulmonal-arteriellen Hypertonie um assoziierte pulmonal-arterielle Hypertonie handelt.

4. Inhibitor von mitotischem Kinesin nach Anspruch 1, wobei es sich bei der pulmonal-arteriellen Hypertonie um familiäre pulmonal-arterielle Hypertonie handelt.

5. Inhibitor von mitotischem Kinesin nach einem der Ansprüche 1-4, wobei es sich bei dem Inhibitor von mitotischem Kinesin um einen Inhibitor des Kinesin-Spindel-Proteins handelt, der aus der Reihe Ispinesibmesylat; N-(3-Aminopropyl)-N-[(R)-(1-3-benzyl-7-chlor-4-oxo-4H-chromen-2-yl)-2-methylpropyl]-4-methylbenzamid-hydrochlorid-Hydrat, (S)-2-(3-Aminopropyl)-5-(2,5-difluorphenyl)-N-methoxy-N-methyl-2-phenyl-1,3,4-thiadiazol-3(2H)carboxamid oder einem pharmazeutisch unbedenklichen Salz davon, 3-(5-(2,5-Difluorphenyl)-3-(5-methyl-1,3,4-thiadiazol-2-yl)-2-phenyl-2,3-dihydro-1,3,4-thiadiazol-2-yl)propan-1-amin oder einem pharmazeutisch unbedenklichen Salz davon, ARRY-520 oder einem pharmazeutisch unbedenklichen Salz davon, (2S)-4-(2,5-Difluorphenyl)-N-[(3R,4S)-3-fluor-1-methylpiperidin-4-yl]-2-(hydroxymethyl)-N-methyl-2-phenyl-2,5-dihydro-1H-pyrrol-1-carboxamid oder einem pharmazeutisch unbedenklichen Salz davon, MK-0731 oder einem pharmazeutisch unbedenklichen Salz davon, 1-[2-(Dimethylamino)ethyl]-3-{[(2R,4aS,5R,10bS)-5-phenyl-9-(trifluormethyl)-3,4,4a,5,6,10b-hexahydro-2H-pyrano[3,2-c]chinolin-2-yl]methyl}Harnstoff oder einem pharmazeutisch unbedenklichen Salz davon, EMD 534085 oder einem pharmazeutisch unbedenklichen Salz davon, (R)-N-(3-Aminopropyl)-N-[1-(5-benzyl-3-methyl-4-oxo-4,5-dihydroisothiazolo[5,4-d]pyrimidin-6-yl)-2-methylpropyl]-4-methylbenzamid oder einem pharmazeutisch unbedenklichen Salz davon, AZD4877 oder einem pharmazeutisch unbedenklichen Salz davon, Litronesib oder einem pharmazeutisch unbedenklichen Salz davon, N-(3-Aminopropyl)-3-chlor-N-[(R)-1-(7-chlor-4-oxo-3-phenylamino-3,4-dihydrochinazolin-2-yl)-but-3-inyl]-2-fluorbenzamid oder einem pharmazeutisch unbedenklichen Salz davon, ARQ 621 oder einem pharmazeutisch unbedenklichen Salz davon, 4SC-205 oder einem pharmazeutisch unbedenklichen Salz davon, CRx-026 oder einem pharmazeutisch unbedenklichen Salz davon und SCH 2047069 oder einem pharmazeutisch unbedenklichen Salz davon ausgewählt ist.

6. Inhibitor von mitotischem Kinesin nach Anspruch 5, wobei es sich bei dem Inhibitor des Kinesin-Spindel-Proteins um Ispinesib-mesylat handelt.

7. Inhibitor von mitotischem Kinesin nach einem der Ansprüche 1-4, wobei es sich bei dem Inhibitor von mitotischem Kinesin um einen CENP-E-Inhibitor handelt, der aus N-(1-{4-[2-(1-Acetylaminoethyl)-1-ethyl-1H-imidazol-4-yl]-benzyl}-3-hydroxypropyl)-3-chlor-4-(2,2,2-trifluor-1-methylethoxy)benzamid, N-(2-(2-Dimethylaminoacetylamino)-1-{4-[8-(1-hydroxyethyl)-imidazo[1,2-a]pyridin-2-yl]benzyl}ethyl)-3-chlor-4-isopropoxybenzamid, N-(1-{4-[2-(1-Methyl-1-hydroxy-ethyl)-1-ethyl-1H-imidazol-4-yl]benzyl}-3-hydroxy-propyl)-3-chlor-4-(2,2,2-trifluor-1-methylethoxy)-benzamid, N-(2-(2-Dimethylaminoacetylamino)-1-{4-[8-methylimidazo[1,2-a]pyridin-2-yl]benzyl}ethyl)-3-chlor-4-isopropoxybenzamid, N-(1-{4-[2-(1-Hydroxy-1-methyl-ethyl)-1-methyl-1H-imidazol-4-yl]benzyl}-3-hydroxypropyl)-3-chlor-4-(2,2,2-trifluor-1-methylethoxy)benzamid, N-(2-(2-Amino-2-methyl-propion-ylamino)-1-{4-[8-methylimidazo[1,2-a]pyridin-2-yl]benzyl}ethyl)-3-chlor-4-isopropoxybenzamid und N-{1-[4-(8-(1-Hydroxyethyl)imidazo[1,2-a]pyridin-2-yl)benzyl]-3-hydroxypropyl}-3-chlor-4-isopropoxy-benzamid oder einem pharmazeutisch unbedenklichen Salz davon ausgewählt ist.

8. Inhibitor von mitotischem Kinesin nach Anspruch 7, wobei es sich bei dem CENP-E-Inhibitor um N-(2-(2-Dimethylaminoacetylamino)-1-{4-[8-(1-hydroxyethyl)-imidazo[1,2-a]pyridin-2-yl]benzyl}ethyl)-3-chlor-4-isopropoxybenzamid oder einem pharmazeutisch unbedenklichen Salz davon handelt.

9. Inhibitor von mitotischem Kinesin nach einem der Ansprüche 1-8, wobei der Inhibitor von mitotischem Kinesin oral, intravenös, subkutan, intranasal, transdermal, intraperitoneal, intramuskulär oder intrapulmonal verabreicht wird.

10. Inhibitor von mitotischem Kinesin nach einem der Ansprüche 1-9, der weiterhin die Verabreichung einer zweiten Therapie an das Individuum umfasst.

11. Inhibitor von mitotischem Kinesin nach Anspruch 10, wobei die zweite Therapie aus Prostanoiden, Endothelinrezeptorantagonisten, Phosphodiesterase-inhibitoren, Prostacyclinrezeptoragonisten, Antikoagulantien, Diuretika, Calciumkanalblockern, Digoxin, Sauerstofftherapie, Stickstoffmonoxidtherapie, Tyrosinkinasen, Statinen, 5-HAT-Rezeptorantagonisten, Phosphatidylinositol-3-Kinase-Inhibitoren, Aktivatoren der löslichen Guanylatcyclase, Adrenomedullin, Inhibitoren des PDGF (Platelet-Derived-Growth-Factor), Rho-Kinase-Inhibitoren, Lungentransplantation, Herztransplantation und Atrioseptostomie ausgewählt ist.

12. Inhibitor von mitotischem Kinesin nach Anspruch 11, wobei es sich bei der zweiten Therapie um einen Endothelinrezeptorantagonisten handelt.

13. Inhibitor von mitotischem Kinesin nach Anspruch 12, wobei es sich bei dem Endothelinrezeptorantagonisten um einen Typ-A-Endothelinrezeptorantagonisten handelt.

14. Inhibitor von mitotischem Kinesin nach Anspruch 12, wobei es sich bei dem Endothelinrezeptorantagonisten um einen dualen Endothelin-Typ-A/Typ-B-Rezeptorantagonisten handelt.

15. Inhibitor von mitotischem Kinesin nach Anspruch 12, wobei es sich bei dem Endothelinrezeptorantagonisten um Bosentan handelt.

16. Inhibitor von mitotischem Kinesin nach Anspruch 12, wobei es sich bei dem Endothelinrezeptorantagonisten um Ambresentan handelt.

17. Inhibitor von mitotischem Kinesin nach Anspruch 10, wobei es sich bei der zweiten Therapie um ein Prostanoid handelt.

18. Inhibitor von mitotischem Kinesin nach Anspruch 17, wobei es sich bei dem Prostanoid um Treprostinil-Natrium handelt.

19. Inhibitor von mitotischem Kinesin nach Anspruch 17, wobei es sich bei dem Prostanoid um Iloprost handelt.

20. Inhibitor von mitotischem Kinesin nach einem der Ansprüche 10-19, wobei der Inhibitor von mitotischem Kinesin und die zweite Therapie dem Individuum gleichzeitig verabreicht werden.

21. Inhibitor von mitotischem Kinesin nach einem der Ansprüche 10-19, wobei der Inhibitor von mitotischem Kinesin und die zweite Therapie dem Individuum der Reihe nach verabreicht werden.

## Revendications

1. Inhibiteur de kinésine mitotique, pour une utilisation dans une méthode de traitement d'une hypertension artérielle pulmonaire chez un sujet, comprenant l'administration au sujet d'une quantité thérapeutiquement efficace de l'inhibiteur de kinésine mitotique ;
**caractérisé en ce que** l'inhibiteur de kinésine mitotique est un inhibiteur de la protéine du fuseau kinésine (KSP) ou **caractérisé en ce que** l'inhibiteur de kinésine mitotique est un inhibiteur de la protéine E associée au centromère (CENP-E).

2. Inhibiteur de kinésine mitotique selon la revendication 1, **caractérisé en ce que** l'hypertension artérielle pulmonaire est l'hypertension artérielle pulmonaire idiopathique.

3. Inhibiteur de kinésine mitotique selon la revendication 1, **caractérisé en ce que** l'hypertension artérielle pulmonaire est l'hypertension artérielle pulmonaire associée.

4. Inhibiteur de kinésine mitotique selon la revendication 1, **caractérisé en ce que** l'hypertension artérielle pulmonaire est l'hypertension artérielle pulmonaire familiale.

5. Inhibiteur de kinésine mitotique selon l'une quelconque des revendications 1-4, **caractérisé en ce que** l'inhibiteur de kinésine mitotique est un inhibiteur de la protéine du fuseau kinésine, qui est choisi parmi le mésylate d'ispinésib ; le chlorhydrate de N-(3-aminopropyl)-N-[(R)-(1-3-benzyl-7-chloro-4-oxo-4H-chromén-2-yl)-2-méthylpropyl]-4-méthylbenzamide hydraté ; le (S)-2-(3-aminopropyl)-5-(2,5-difluorophényl)-N-méthoxy-N-méthyl-2-phényl-1,3,4-thiadiazole-3(2H)-carboxamide, ou un sel pharmaceutiquement acceptable de celui-ci ; la 3-(5-(2,5-difluorophényl)-3-(5-méthyl-1,3,4-thiadiazol-2-yl)-2-phényl-2,3-dihydro-1,3,4-thiadiazol-2-yl)propan-1-amine, ou un sel pharmaceutiquement acceptable de celle-ci ; ARRY-520, ou un sel pharmaceutiquement acceptable de celui-ci ; le (2S)-4-(2,5-difluorophényl)-N-[(3R,4S)-3-fluoro-1-méthylpipéridin-4-yl]-2-(hydroxyméthyl)-N-méthyl-2-phényl-2,5-dihydro-1H-pyrrole-1-carboxamide, ou un sel pharmaceutiquement acceptable de celui-ci ; MK-0731, ou un sel pharmaceutiquement acceptable de celui-ci ; la 1-[2-(diméthylamino)éthyl]-3-{[(2R,4aS,5R,10bS)-5-phényl-9-(trifluorométhyl)-3,4,4a,5,6,10b-hexahydro-2H-pyrano[3,2-c]quinoléin-2-yl]méthyl}urée, ou un sel pharmaceutiquement acceptable de celle-ci ; EMD 534085, ou un sel pharmaceutiquement acceptable de celui-ci ; le (R)-N-(3-aminopropyl)-N-[1-(5-benzyl-3-méthyl-4-oxo-4,5-dihydroisothiazolo[5,4-d]pyrimidin-6-yl)-2-méthylpropyl]-4-méthylbenzamide, ou un sel pharmaceutiquement acceptable de celui-ci ; AZD4877, ou un sel pharmaceutiquement acceptable de celui-ci ; le litronésib, ou un sel pharmaceutiquement acceptable de celui-ci ; le N-(3-aminopropyl)-3-chloro-N-[(R)-1-(7-chloro-4-oxo-3-phénylamino-3,4-dihydroquinazolin-2-yl)-but-3-ynyl]-2-fluorobenzamide, ou un sel pharmaceutiquement acceptable de celui-ci ; ARQ 621, ou un sel pharmaceutiquement acceptable de celui-ci ; 4SC-205, ou un sel pharmaceutiquement acceptable de celui-ci ; CRx-026, ou un sel pharmaceutiquement acceptable de celui-ci ; et SCH 2047069, ou un sel pharmaceutiquement acceptable de celui-ci.

6. Inhibiteur de kinésine mitotique selon la revendication 5, **caractérisé en ce que** l'inhibiteur de la protéine du fuseau kinésine est le mésylate d'ispinésib.

7. Inhibiteur de kinésine mitotique selon l'une quelconque des revendications 1-4, **caractérisé en ce que** l'inhibiteur de kinésine mitotique est un inhibiteur de CENP-E, qui est choisi parmi le N-(1-{4-[2-(1-acétylaminoéthyl)-1-éthyl-1H-imidazol-4-yl]-benzyl}-3-hydroxypropyl)-3-chloro-4-(2,2,2-trifluoro-1-méthyléthoxy)benzamide, le N-(2-(2-diméthylamino-acétylamino)-1-{4-[8-(1-hydroxyéthyl)-imidazo[1,2-a]-pyridin-2-yl]benzyl}éthyl)-3-chloro-4-isopropoxy-benzamide, le N-(1-{4-[2-(1-méthyl-1-hydroxyéthyl)-1-éthyl-1H-imidazol-4-yl]benzyl}-3-hydroxypropyl)-3-chloro-4-(2,2,2-trifluoro-1-méthyléthoxy)benzamide, le N-(2-(2-diméthylaminoacétylamino)-1-{4-[8-méthyl-imidazo[1,2-a]pyridin-2-yl]benzyl}éthyl)-3-chloro-4-isopropoxybenzamide, le N-(1-{4-[2-(1-hydroxy-1-méthyléthyl)-1-méthyl-1H-imidazol-4-yl]benzyl}-3-hydroxypropyl)-3-chloro-4-(2,2,2-trifluoro-1-méthyléthoxy)benzamide, le N-(2-(2-amino-2-méthyl-propionylamino)-1-{4-[8-méthylimidazo[1,2-a]pyridin-2-yl]benzyl}éthyl)-3-chloro-4-isopropoxybenzamide, et le N-{1-[4-(8-(1-hydroxyéthyl)imidazo[1,2-a]pyridin-2-yl)benzyl]-3-hydroxypropyl}-3-chloro-4-isopropoxy-benzamide, ou un sel pharmaceutiquement acceptable de ceux-ci.

8. Inhibiteur de kinésine mitotique selon la revendication 7, **caractérisé en ce que** l'inhibiteur de CENP-E est le N-(2-(2-diméthylaminoacétylamino)-1-{4-[8-(1-hydroxyéthyl)-imidazo[1,2-a]pyridin-2-yl]benzyl}-éthyl)-3-chloro-4-isopropoxybenzamide, ou un sel pharmaceutiquement acceptable de celui-ci.

9. Inhibiteur de kinésine mitotique selon l'une quelconque des revendications 1-8, **caractérisé en ce que** l'inhibiteur de kinésine mitotique est administré par voie orale, intraveineuse, sous-cutanée, intranasale, transdermique, intrapéritonéale, intramusculaire ou intrapulmonaire.

10. Inhibiteur de kinésine mitotique selon l'une quelconque des revendications 1-9, comprenant en outre l'administration au sujet d'une deuxième thérapie.

11. Inhibiteur de kinésine mitotique selon la revendication 10, **caractérisé en ce que** la deuxième thérapie est choisie parmi les prostanoïdes, les antagonistes du récepteur de l'endothéline, les inhibiteurs de phosphodiestérase, les agonistes du récepteur de la prostacycline, les anticoagulants, les diurétiques, les inhibiteurs du canal calcique, la digoxine, l'oxygénothérapie, la thérapie à l'oxyde nitrique, les tyrosine kinases, les statines, les antagonistes du récepteur de 5-HT, les inhibiteurs de phosphatidylinositol 3-kinase, les activateurs de guanylate cyclase soluble, l'adrénomédulline, les inhibiteurs du facteur de croissance dérivé des plaquettes, les inhibiteurs de Rho-kinase, la greffe de poumon, la greffe de coeur, et la septostomie atriale.

12. Inhibiteur de kinésine mitotique selon la revendication 11, **caractérisé en ce que** la deuxième thérapie est un antagoniste du récepteur de l'endothéline.

13. Inhibiteur de kinésine mitotique selon la revendication 12, **caractérisé en ce que** l'antagoniste du récepteur de l'endothéline est un antagoniste du récepteur de l'endothéline de type A.

14. Inhibiteur de kinésine mitotique selon la revendication 12, **caractérisé en ce que** l'antagoniste du récepteur de l'endothéline est un antagoniste du récepteur de l'endothéline double de type A/type B.

15. Inhibiteur de kinésine mitotique selon la revendication 12, **caractérisé en ce que** l'antagoniste du récepteur de l'endothéline est le bosentan.

16. Inhibiteur de kinésine mitotique selon la revendication 12, **caractérisé en ce que** l'antagoniste du récepteur de l'endothéline est l'ambresentan.

17. Inhibiteur de kinésine mitotique selon la revendication 10, **caractérisé en ce que** la deuxième thérapie est un prostanoïde.

18. Inhibiteur de kinésine mitotique selon la revendication 17, **caractérisé en ce que** le prostanoïde est le tréprostinil de sodium.

19. Inhibiteur de kinésine mitotique selon la revendication 17, **caractérisé en ce que** le prostanoïde est l'iloprost.

20. Inhibiteur de kinésine mitotique selon l'une quelconque des revendications 10-19, **caractérisé en ce que** l'inhibiteur de kinésine mitotique et la deuxième thérapie sont administrés simultanément au sujet.

21. Inhibiteur de kinésine mitotique selon l'une quelconque des revendications 10-19, **caractérisé en ce que** l'inhibiteur de kinésine mitotique et la deuxième thérapie sont administrés séquentiellement au sujet.
